(19) Europäisches Patentamt
European Patent Office
Office européen des brevets

(11) **EP 4 363 051 B1**

(12) **EUROPÄISCHE PATENTSCHRIFT**

(45) Veröffentlichungstag und Bekanntmachung des Hinweises auf die Patenterteilung:
**29.07.2026   Patentblatt 2026/31**

(21) Anmeldenummer: **22733003.2**

(22) Anmeldetag: **08.06.2022**

(51) Internationale Patentklassifikation (IPC):
**A61N 5/06** *(2006.01)*     **A61F 7/00** *(2006.01)*
**A47C 7/74** *(2006.01)*

(52) Gemeinsame Patentklassifikation (CPC):
**A61N 5/0625;** A61F 2007/0024; A61F 2007/0088;
A61N 2005/0642; A61N 2005/0659

(86) Internationale Anmeldenummer:
**PCT/EP2022/065471**

(87) Internationale Veröffentlichungsnummer:
**WO 2022/258649 (15.12.2022 Gazette 2022/50)**

(54) **VORRICHTUNG MIT INFRAROTHEIZMODULEN ZUM ZUFÜHREN VON WÄRME**

DEVICE WITH INFRARED HEATING MODULES FOR SUPPLYING HEAT

DISPOSITIF AVEC DES MODULES DE CHAUFFAGE À INFRAROUGE POUR APPORTER DE LA CHALEUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **08.06.2021   EP 21178318**
**13.07.2021   EP 21185394**
**26.08.2021   EP 21193188**

(43) Veröffentlichungstag der Anmeldung:
**08.05.2024   Patentblatt 2024/19**

(73) Patentinhaber: **Mattheiss, Gerd**
**7203 Trimmis (CH)**

(72) Erfinder:
• **PECHER, Otto**
**85653 Aying b. München (DE)**

• **ZEIGER, Thomas**
**6134 Vomp (AT)**
• **MATTHEISS, Gerd**
**7203 Trimmis (CH)**

(74) Vertreter: **Fuchs Patentanwälte Partnerschaft mbB**
**Tower 185**
**Friedrich-Ebert-Anlage 35-37**
**60327 Frankfurt am Main (DE)**

(56) Entgegenhaltungen:
**CA-A1- 3 057 840       DE-A1- 102013 017 586**
**US-A1- 2007 179 571     US-A1- 2012 122 636**
**US-A1- 2015 105 844**

## Beschreibung

Die vorliegende Erfindung betrifft eine Vorrichtung zum Zuführen von Wärme an eine Hautpartie eines Lebewesens. Die vorliegende Erfindung betrifft außerdem ein Infrarotheizmodul zum Zuführen von Wärme an eine Hautpartie eines Lebewesens. Die vorliegende Erfindung betrifft ferner eine erfindungsgemäße Vorrichtung, die ein erfindungsgemäßes Infrarotheizmodul aufweist. Die vorliegende Erfindung betrifft auch eine Einheit zum Zuführen von Wärme an eine Hautpartie eines Lebewesens, eine Anordnung, die mehrere solcher Einheiten aufweist, sowie eine Kabine, eine Wärmestaudecke und eine Tragevorrichtung, die jeweils eine solche Einheit oder Anordnung aufweisen. Die Erfindung betrifft auch eine Vorrichtung mit mehreren solcher Einheiten.

### Stand der Technik

CA 3 057 840 A1 betrifft einstellbare Beleuchter, Verfahren zur photodynamischen Behandlung und Diagnose. US 2015/105844 A1 betrifft Stimulation mittels thermischer Reize. US 2007/179571 A1 betrifft eine lichtemittierende Vorrichtung und ein Verfahren zum Bereitstellen von Photobehandlung an das Gehirn. US 2012/122636 A1 betrifft ein Infrarotwärme-Übungspad.

Aus dem Stand der Technik sind Infrarotstrahler für den Einsatz bei Wärmeanwendungen bekannt. Allerdings ist es bei bekannten Strahlern häufig kompliziert, oder teils gar nicht möglich, die abgegebene Wärme zu kontrollieren und/oder zuverlässig einzustellen. Dadurch führen Wärmeanwendungen nicht immer zu optimalen Ergebnissen.

Es ist daher eine Aufgabe der vorliegenden Erfindung, Mittel anzugeben, mit denen die Nachteile des Stands der Technik überwunden werden können und die insbesondere eine zuverlässige, effiziente und sichere Wärmeanwendung ermöglichen.

### Beschreibung der Erfindung

Die Erfindung ist durch die anhängenden Ansprüche definiert. Gemäß einem ersten Aspekt ist eine Vorrichtung zum Zuführen von Wärme an eine Hautpartie eines Lebewesens offenbart, die drei oder mehr als drei Infrarotheizmodule aufweist, wobei vorzugsweise die Vorrichtung zumindest ein Abstützelement zum zumindest teilweisen Abstützen der Vorrichtung an dem Lebewesen aufweist.

Dem liegt damit die überraschende Erkenntnis zugrunde, dass der Hautpartie die Wärmestrahlung besonders kontrolliert bereitgestellt werden kann, indem die Vorrichtung definiert oder definierbar relativ zu der Hautpartie anordenbar ist.

Dadurch ist es besonders zuverlässig möglich, einen definierten oder definierbaren Abstand zwischen dem einen, aber vor allem den mehreren, Infrarotheizmodulen und der Hautpartie vorzugeben. Dies wiederum ermöglicht es, eine abgestimmte Verteilung der von den Infrarotheizmodulen abgegebenen Infrarotstrahlung und/oder Wärme zwischen der Vorrichtung und der Hautpartie zu ermöglichen und damit einen zuverlässigen Einsatz der Vorrichtung zu fördern.

Außerdem wird der Einsatz der Vorrichtung durch den Anwender aufgrund des Abstützelements vereinfacht, da mittels des Abstützelements die Vorrichtung leichter in eine richtige Position gebracht und dort gehalten werden kann. Dadurch wird nicht zuletzt auch die Sicherheit beim Einsatz einer solchen Vorrichtung erhöht.

Das Lebewesen kann vorliegend beispielsweise ein Mensch oder ein Tier sein.

Die Hautpartie kann die Hautpartie einer Körperregion des Lebewesens sein. Beispielsweise kann es eine Hautpartie des Rückens oder des Beins, insbesondere des Knies, des Lebewesens sein.

Die Vorrichtung kann beispielsweise drei, vier, fünf, sechs, sieben, acht, neun, zehn oder mehr als zehn Infrarotheizmodule aufweisen. Durch das Bereitstellen mehrerer Infrarotheizmodule und die mittels des Abstützelements realisierbare, definierte relative Anordnung der Vorrichtung und des Lebewesens zueinander, kann eine optimale und gleichmäßige Bestrahlung des Lebewesens erreicht werden.

Alternativ oder ergänzend kann vorgesehen sein, dass die Vorrichtung wenigstens ein Gehäuse aufweist, und wobei vorzugsweise innerhalb eines von dem Gehäuse zumindest bereichsweise eingeschlossenen Volumens die Infrarotheizmodule angeordnet sind.

Das Gehäuse kann beispielsweise einstückig oder mehrteilig ausgeführt sein. Neben den darin angeordneten Infrarotheizmodulen ist auch weitere Elektronik innerhalb des von dem Gehäuse eingeschlossenen Volumens anordenbar. Damit sind gerade die elektrischen Komponenten der Vorrichtung sicher untergebracht.

Alternativ oder ergänzend kann vorgesehen sein, dass (i) das zumindest eine Abstützelement an einer Seitenpartie und/oder an einer Frontpartie des Gehäuses direkt oder indirekt angeordnet ist, (ii) mehrere gleichartige Abstützelemente an der Vorrichtung, insbesondere ihrer Seiten-und/oder Frontpartie, angeordnet sind und/oder (iii) das zumindest eine Abstützelement zum zumindest teilweisen Abstützen der Vorrichtung an einem Rückenbereich des Lebewesens geeignet ist.

Indem das Abstützelement an einer Seitenpartie des Gehäuses angeordnet ist, beispielsweise also seitlich von dem übrigen Teil der Vorrichtung herausragt, kann der mittlere Teil der Vorrichtung kompakter ausgestaltet werden. Ist hingegen das Abstützelement an einer Frontpartie des Gehäuses angeordnet, kann die Vorrichtung robuster ausgeführt werden und beispielsweise auch einen Teil des Rückens des Lebewesens komplett umschließen.

Das Abstützelement ist vorzugsweise dann direkt an der Partie des Gehäuses angeordnet, wenn kein weiteres Element zwischen Abstützelement und Gehäuse vorgesehen ist und ist anderenfalls indirekt an der Partie des Gehäuses angeordnet.

Es kann freilich vorzugsweise auch ganz allgemein vorgesehen sein, dass das zumindest eine Abstützelement an dem Gehäuse direkt oder indirekt angeordnet ist.

Alternativ oder ergänzend kann vorgesehen sein, dass das Abstützelement eine spezifische Oberfläche bereitstellt, die vorzugsweise zum Abstützen der Vorrichtung an dem Lebewesen mit einer Kontaktoberfläche, insbesondere eines Rückenbereichs, des Lebewesens in Kontakt bringbar ist, wobei der Verlauf und/oder die Gestalt der spezifischen Oberfläche zumindest abschnittsweise und/oder bereichsweise an den Verlauf und/oder die Gestalt der Kontaktoberfläche anpassbar ist.

Indem das Abstützelement also in dem Oberflächenbereich (nämlich derjenige der spezifischen Oberfläche), in dem es beim Einsatz der Vorrichtung an dem Lebewesen aufkommt, gewissermaßen an die Kontur des Lebewesens anschmiegbar ist, wird ein besonders komfortabler und sicherer Einsatz der Vorrichtung ermöglicht.

Alternativ oder ergänzend kann vorgesehen sein, dass das Abstützelement mit spezifischer Oberfläche mehrere, vorzugsweise gegeneinander bewegbare, insbesondere in ihrer Position und/oder Lage veränderbare, und/oder, insbesondere durch Komprimierung, in ihrer Form und/oder Gestalt reversibel veränderbare, Einzelelemente aufweist und jedes Einzelelement mit einem Oberflächenbereich einen Teil der spezifischen Oberfläche des Abstützelements bereitstellt.

Die Einzelelemente können damit jeweils einen individuellen Oberflächenbereich bereitstellen und alle Einzelelemente zusammen bilden dann die spezifische Oberfläche des Abstützelements.

Durch die Einzelelemente ist es besonders einfach möglich, den Verlauf der spezifischen Oberfläche bereichsweise/abschnittsweise an den Verlauf der Oberfläche des Lebewesens anzupassen. Denn die spezifische Oberfläche wird sozusagen durch mehrere kleinere Oberflächen zusammengestellt. Dadurch kann der lokale Verlauf der Oberfläche des Lebewesens (jedenfalls bereichsweise bei der Kontaktoberfläche) besser nachempfunden werden und somit der Sitz der Vorrichtung an dem Lebewesen verbessert werden.

Indem nämlich die einzelnen Einzelelemente gegeneinander bewegbar sind, kann der Verlauf und/oder die Gestalt der spezifischen Oberfläche anpassbar sein. Auch kann vorzugsweise der Oberflächenbereich in seiner Form und/oder Gestalt reversibel veränderbar sein.

Es ist bevorzugt, dass die spezifische Oberfläche vollständig durch die Oberflächenbereiche der Einzelelemente bereitgestellt wird. Dabei ist es möglich, dass die spezifische Oberfläche nicht zusammenhängend ist. Beispielsweise kann in dem Bereich zwischen den einzelnen Einzelelementen eine Unterbrechung der spezifischen Oberfläche auftreten.

Alternativ oder ergänzend kann vorgesehen sein, dass das Abstützelement mit spezifischer Oberfläche ein elastisches Grundelement, insbesondere bestehend aus oder aufweisend Silikon, aufweist, auf dem die Einzelelemente angeordnet sind, und insbesondere stoffschlüssig mit dem elastischen Grundelement verbunden sind.

Indem das elastische Grundelement vorgesehen ist, kann besonders einfach erreicht werden, dass die Einzelelemente gegeneinander bewegbar sind. Denn das Abstützelement ist in diesem Fall besonders gut dafür einsetzbar, dass durch eine äußere auf das Abstützelement einwirkende Belastung (durch das Lebewesen) die Einzelelemente abhängig von dem lokal bestehendem Belastungsdruck in das elastische Grundelement hineingedrückt werden, etwa indem das elastische Grundelement lokal komprimiert wird, und dadurch die Position und/oder Lage des Einzelelements verändert wird.

Das elastische Grundelement kann einstückig ausgebildet sein. Alternativ kann es auch mehrteilig ausgebildet sein. Dann können beispielsweise unterschiedliche Einzelelemente auf verschiedenen Teilen des elastischen Grundelements angeordnet sein.

Alternativ oder ergänzend kann vorgesehen sein, dass (i) die Einzelelemente, vorzugsweise nebeneinander und/oder flächig, auf dem elastischen Grundelement angeordnet sind, (ii) das elastische Grundelement schichtförmig ausgebildet ist, (iii) das elastische Grundelement, insbesondere unmittelbar, zwischen einerseits den Einzelelementen und andererseits dem Gehäuse angeordnet ist, vorzugsweise das elastische Grundelement direkt auf dem Gehäuse angeordnet ist, (iv) das elastische Grundelement quaderförmig ist und/oder (v) das elastische Grundelement eine Dicke von wenigstens 0,50 cm und/oder von maximal 10 cm, vorzugsweise maximal 5 cm, aufweist.

Einen besonders einfachen aber dennoch wirkungsvollen Aufbau kann mit dem schichtförmig ausgebildeten oder quaderförmigen elastischen Grundelement erreicht werden. Gleichermaßen ist ein einfacher und kostengünstiger Herstellungsprozess möglich, indem das elastische Grundelement direkt auf dem Gehäuse aufgebracht ist. Optional kann auch beispielsweise ein Haftvermittler zwischen dem elastischem Grundelement und dem Gehäuse vorgesehen sein.

Alternativ oder ergänzend kann vorgesehen sein, dass die Einzelelemente selbst nicht-kompressibel sind und/oder als Material Kunststoffe, Kunstleder, Leder und/oder sonstige Naturmaterialien aufweisen oder daraus bestehen.

Vor allem im Zusammenspiel mit einem elastischen Grundelement müssen die Einzelelemente nicht kompressibel sein, um eine Oberfläche des Abstützelements mit anpassbarem Verlauf und/oder anpassbarer Gestalt bereitzustellen. Dadurch kann der Vorteil einer anpassbaren Oberfläche auch für Einzelelementen erreicht werden, die mit kostengünstigen Verfahren, wie Spritzguss, hergestellt oder herstellbar sind.

Indem die Einzelelemente aus einem kompressiblen Material sind, können der Verlauf und/oder die Gestalt der spezifischen Oberfläche optimal an den Verlauf der Kontaktoberfläche angeglichen werden, wodurch ein besonders guter Halt der Vorrichtung an dem Lebewesen ermöglicht werden kann.

Alternativ oder ergänzend kann vorgesehen sein, dass das Abstützelement mit spezifischer Oberfläche 2 oder mehr als 2, vorzugsweise 5 oder mehr als 5, vorzugsweise 10 oder mehr als 10, und/oder 20 oder weniger als 20, vorzugsweise 15 oder weniger als 15, vorzugsweise 10 oder weniger als 10, vorzugsweise 7 oder weniger als 7, vorzugsweise 5 oder weniger als 5, vorzugsweise 3 oder weniger als 3, Einzelelemente aufweist.

Alternativ oder ergänzend kann vorgesehen sein, dass die Vorrichtung, insbesondere an der Seitenpartie und/oder an der Frontpartie, zumindest ein, vorzugsweise pyramidenstumpfförmige oder parallelepipedförmige, Podestelement aufweist, vorzugsweise mehrere solcher Podestelemente aufweist, und wobei vorzugsweise die Podestelemente von dem Gehäuse ausgebildet werden.

Ein solches Podestelement ermöglicht es besonders einfach, bestimmte Elemente von übrigen Teilen der Vorrichtung beabstandet und vor allem exponiert zu positionieren oder positionieren zu können.

Alternativ oder ergänzend kann vorgesehen sein, dass die Vorrichtung zumindest einen Infrarot-Temperatursensor aufweist, der insbesondere dazu eingerichtet ist, eine lokale Hauttemperatur des Lebewesens zu messen, wobei vorzugsweise der Sensor (i) an dem Gehäuse angeordnet ist, (ii) derart an der Vorrichtung, insbesondere an dem Gehäuse, angeordnet ist, dass der Sensor beim Einsatz der Vorrichtung zwischen den Infrarotheizmodulen und der Hautpartie des Lebewesens angeordnet ist, (iii) außerhalb des von dem Gehäuse zumindest bereichsweise eingeschlossenen Volumens angeordnet ist, (iv) im Bereich eines Podestelements angeordnet ist, (v) an einem Podestelement angeordnet ist, insbesondere an einer Seitenfläche des Podestelements, und/oder (vi) ganz oder teilweise innerhalb eines Podestelements angeordnet ist, und wobei vorzugsweise das Podestelement, insbesondere einer seiner Seitenflächen, zumindest bereichsweise einen für die Infrarot-Temperaturmessung durchlässigen Wandbereiche aufweist.

Mittels eines solchen Sensors kann die jeweils bestehende Hauttemperatur in einem von dem Sensor erfassten Hautbereich gemessen werden. Anhand des Temperaturwertes kann wiederum die von den Infrarotheizmodulen abgegebene Wärmestrahlung geregelt werden, so dass eine Überhitzung der Hauptpartie vermieden werden kann. Dadurch ist der Einsatz der Vorrichtung sicherer.

Indem der Sensor zwischen den Infrarotheizmodulen und der Hautpartie angeordnet ist, kann der Sensor einen ausreichenden Abstand von den Infrarotheizmodulen aufweisen, so dass die Messung nicht oder nicht erheblich von den Infrarotheizmodulen und der von diesen ausgestrahlten Infrarotstrahlung gestört wird.

Wenn davon gesprochen wird, dass der Sensor "zwischen" den Infrarotheizmodulen und der Hautpartie angeordnet ist, dann kann dies vorzugsweise auch den Fall mit einschließen, dass der Sensor lateral, also nach außen hin, versetzt angeordnet ist.

Durch die exponierte Anordnung des Sensors an oder in einem Podestelement kann erreicht werden, dass andere Teile der Vorrichtung die freie Sicht des Sensors auf die Hautpartie nicht beeinträchtigen oder gefährden.

Indem der Sensor an einem Podestelement angeordnet ist, lässt sich der Blickwinkel des Sensors und damit der mittels des Sensors überwachte Hautbereich besonders zuverlässig einstellen. Insbesondere das Vorsehen des Sensors an einer Seitenfläche eines Podestelements ermöglicht durch eine entsprechend geneigte Seitenfläche einen besonders vorteilhaften Blickwinkel auf die Hautpartie des Lebewesens. Indem der Sensor innerhalb eines Podestelements angeordnet ist, kann ein besonders kompaktes Design erreicht werden. Gerade in Verbindung mit einem für die Infrarot-Messung durchlässigen Wandbereich kann der Sensor komplett in der Vorrichtung verborgen werden, wodurch der Sensor vor externen Einflüssen wie Berührung oder Schweiß zuverlässig geschützt sein kann.

Der Wandbereich kann für die Infrarot-Temperaturmessung durchlässig sein, wenn beispielsweise der Wandbereich für Infrarotstrahlung im Bereich zwischen 0,78 $\mu$m und 15 $\mu$m einen Transmissionsgrad von 30 % oder mehr, vorzugsweise von 50 % oder mehr, bevorzugt von 80 % oder mehr, aufweist.

Alternativ oder ergänzend kann vorgesehen sein, dass das Abstützelement mit spezifischer Oberfläche, vorzugsweise alle Abstützelemente mit spezifischer Oberfläche, jeweils auf einem Podestelement angeordnet ist und/oder auf Silikon gelagert angeordnet ist.

Indem das Abstützelement auf einem Podestelement angeordnet ist, kann die Distanz zwischen dem Lebewesen und Teilen der Vorrichtung (beispielsweise den Infrarotheizmodulen) besonders einfach angepasst werden, ohne die Abstützelemente selbst ändern zu müssen. Mit anderen Worten, es kann mit nur einem Typ von Abstützelement verschiedene Abstände erreicht werden. Damit kann beispielsweise auch für Modelle der Vorrichtung mit unterschiedlichen Abmessungen und daher mit unterschiedlich notwendigen Abständen zu der Hautpartie, der Wärme zugeführt werden soll, die gleichen Abstützelemente eingesetzt werden. Die unterschiedlichen Abstände können über die Abmessungen der jeweiligen Podestelemente eingestellt werden. Dadurch sind die Produktionskosten der Vorrichtung geringer. Ein gerade bei großen Stückzahlen nicht zu unterschätzender wirtschaftlicher Faktor.

Indem das Abstützelement auf Silikon gelagert angeordnet ist, kann auf das Abstützelement ausgeübter Druck, beispielsweise bei Beanspruchung durch das Lebewesen, zumindest zum Teil abgefangen werden, ohne das Abstützelement zu beschädigen. Dadurch hält das Abstützelement auch höheren Belastungen besser Stand.

Alternativ oder ergänzend kann vorgesehen sein, dass das Abstützelement mit spezifischer Oberfläche eine Rückenlehne ausbildet.

Vorzugsweise sind mehrere Abstützelemente von der Vorrichtung aufgewiesen und mehr als eines, insbesondere alle, dieser Abstützelemente bilden die Rückenlehne aus.

Die Rückenlehne ermöglicht ein sicheres Anordnen der Vorrichtung an dem Lebewesen.

Alternativ oder ergänzend kann vorgesehen sein, dass das Abstützelement mit spezifischer Oberfläche zerstörungsfrei lösbar und/oder austauschbar an der Vorrichtung, insbesondere an dem Podestelement, angeordnet ist.

Dadurch sind beschädigte Abstützelemente leicht ersetzbar, was die Vorrichtung günstig im laufenden Unterhalt macht. Auch können dadurch unterschiedliche Abstützelemente angeboten werden (zum Beispiel hinsichtlich Größe, Steifigkeit, Material, etc.) aus denen abhängig vom jeweiligen Anwendungsszenario und/oder Benutzer ausgewählt werden kann. So lässt sich die Vorrichtung besonders einfach individuell konfigurieren und sicher einsetzen.

Alternativ oder ergänzend kann vorgesehen sein, dass der Oberflächenbereich zumindest eines, vorzugsweise mehrere, der Einzelelemente eines jeden Abstützelements mit spezifischer Oberfläche sechseckförmig ist.

Insbesondere eine wabenförmige Struktur ist dabei besonders vorteilhaft, da benachbarte Einzelelemente einen überlappenden Übergangsbereich bereitstellen können, wodurch die spezifische Oberfläche besonders zuverlässig in ihrem Verlauf anpassbar ist.

Alternativ oder ergänzend kann vorgesehen sein, dass die Vorrichtung einen Berührschutz aufweist, wodurch ein unbeabsichtigtes Berühren der Infrarotheizmodule verhinderbar ist, wobei der Berührschutz vorzugsweise in einer von dem Gehäuse ausgebildeten Öffnung angeordnet ist und/oder diese zumindest teilweise ausfüllt.

Durch den Berührschutz kann der Einsatz der Vorrichtung sicherer gestaltet sein, da ein versehentliches Berühren der Infrarotheizmodule verhindert oder zumindest erschwert ist.

Alternativ oder ergänzend kann vorgesehen sein, dass der Berührschutz gitterförmig ist, insbesondere mehrere zueinander beabstandete Gitterstäbe und/oder Saiten aufweist oder daraus besteht.

Ein derart gestalteter Berührschutz ermöglicht einen besonders effektiven Berührschutz, bei einem nur geringen Materialeinsatz. Dementsprechend kann dadurch auch das Gewicht der Vorrichtung reduziert sein, was wiederum auch das Handhaben und der Vorrichtung einfacher und sicherer gestaltet.

Alternativ oder ergänzend kann vorgesehen sein, dass die Vorrichtung eine Beleuchtung aufweist, wobei vorzugsweise die Beleuchtung zumindest einen seitlichen Innenteil des Gehäuses beleuchtet oder an diesem angeordnet ist.

Die Beleuchtung kann die Wartung der Vorrichtung besonders einfach und sicher gestalten. Aber auch der Einsatz der Vorrichtung lässt sich durch die Beleuchtung sicherer gestalten, indem beispielsweise die Beleuchtung dazu eingerichtet ist, einem Benutzer einen Zustand der Vorrichtung zu signalisieren. Beispielsweise kann durch Farbe, Rhythmus und/oder Frequenz der Beleuchtung ein bestimmter Zustand signalisiert werden.

Alternativ oder ergänzend kann vorgesehen sein, dass die Vorrichtung eine Kopfstütze und ein Haltemittel aufweist, wobei die Kopfstütze mittels des Haltemittels beabstandet zu dem Gehäuse der Vorrichtung befestigt oder befestigbar ist.

Die Kopfstütze ermöglicht einen angenehmen und sicheren Einsatz der Vorrichtung.

Alternativ oder ergänzend kann vorgesehen sein, dass die Kopfstütze eine Plastikschale und ein daran angeordnetes flexibles Netzgewebe aufweist.

Die Plastikschale einer derartigen Kopfstütze ist günstig beispielsweise mittels Spritzgusses herstellbar. Durch das Netzgewebe kann eine komfortable Abstützung des Kopfes des Benutzers der Vorrichtung erreicht werden. Das flexible Netzgewebe überspannt beispielsweise einen Öffnungsbereich der Plastikschale. Dadurch lässt sich das Netzgewebe durch Belastung durch den Kopf in den Öffnungsbereich hinein dehnen und passt sich damit der Kopfform des Benutzers an und vermittelt so eine zuverlässige Abstützung.

Alternativ oder ergänzend kann vorgesehen sein, dass das Haltemittel von dem Gehäuse lösbar und/oder in dem Gehäuse versenkbar ist und/oder federnde Eigenschaften hat.

Alternativ oder ergänzend kann vorgesehen sein, dass die Kopfstütze als Gehäuseabdeckung in einem beim Einsatz der Vorrichtung unteren und/oder oberen Bereich an dem Gehäuse lösbar befestigbar ist.

Dadurch lässt sich die Vorrichtung bei Nichtgebrauch kompakt aufbewahren.

Alternativ oder ergänzend kann vorgesehen sein, dass die Vorrichtung einen Aroma-Diffusor aufweist, insbesondere im beim Einsatz der Vorrichtung oberen Bereich der Vorrichtung.

Alternativ oder ergänzend kann vorgesehen sein, dass die Infrarotheizmodule jeweils ein lasergeschnittenes Heizelement oder ein Flächenheizelement aus Kunstglimmer aufweisen und/oder eine Abdeckung aus, insbesondere, eloxiertem, Aluminium aufweisen.

Dadurch kann gerade der Rückenbereich eines Lebewesens besonders gut mit Wärme versorgt werden.

Alternativ oder ergänzend kann vorgesehen sein, dass die Infrarotheizmodule beim Einsatz der Vorrichtung derart relativ zu und/oder an dem Lebewesen angeordnet sind, dass für eine definierte oder definierbare spezifische Transversalebene des Lebewesens jedes der Infrarotheizmodule eine Schnittfläche mit der spezifischen Transversalebene hat, wobei vorzugsweise jedes Infrarotheizmodul auf einer individuellen Betriebstemperatur betreibbar ist, und/oder beim Einsatz der Vorrichtung die Betriebstemperaturen der Infrarotheizmodule derart zumindest teilweise unterschiedlich eingestellt oder einstellbar sind, dass die Intensität der, insbesondere beim Einsatz der Vorrichtung, von der Vorrichtung bereitgestellten Wärmestrahlung zum Zuführen an die Hautpartie zumindest entlang einer ersten und einer zweiten jeweils parallel zur spezifischen Transversalebene verlaufenden Richtung innerhalb zumindest eines betrachteten Bereichs außerhalb der Vorrichtung homogen ist.

Dieser Ausgestaltung liegt die überraschende Erkenntnis zugrunde, dass der Hautpartie die Wärmestrahlung besonders kontrolliert bereitgestellt werden kann, indem die Abstrahlcharakteristik der einzelnen Infrarotheizmodule berücksichtigt wird. Dadurch kann in einem bestimmten Bereich außerhalb des Strahlers ein auf die jeweilige Anwendung abgestimmtes Wärmefeld bereitgestellt werden. Wenn sich bei der Wärmebehandlung die Hautpartie innerhalb dieses Bereichs befindet, kann damit eine zuverlässige Wärmebehandlung durchgeführt werden. Außerdem kann so besonders einfach das Risiko einer lokalen Überhitzung der Hautpartie - sogenannte Hotspots - in Folge eines zu hohen Wärmestrahlungsbeitrags mehrerer Infrarotheizmodule zuverlässig verringert werden (ggf. in Kombination mit weiteren Maßnahmen, insbesondere die Steuerung betreffend). Gleichzeitig können ganz entsprechend auch Bereiche mit zu geringer Wärmestrahlungszufuhr vermieden werden, wodurch eine zuverlässige und gleichmäßige Wärmeanwendung ermöglicht wird.

Die Erfinder haben insoweit erkannt, dass die unterschiedlichen Abstrahlcharakteristiken besonders einfach aber dennoch wirkungsvoll dadurch berücksichtigt werden können, indem die Infrarotheizmodule mit unterschiedlicher Temperatur betrieben werden. Dadurch ist es überraschend einfach möglich, innerhalb des betrachteten Bereichs, der außerhalb der Vorrichtung liegt, eine homogene Intensität der Wärmestrahlung, die sich aus überlagerten Beiträgen auch mehrerer Infrarotheizmodule ergeben kann, bereitzustellen. Da mit unterschiedlichen Betriebstemperaturen regelmäßig auch eine veränderliche Abstrahlcharakteristik einhergeht, kann durch eine gezielt einstellbare oder eingestellte Betriebstemperatur daher eine homogene Intensität der Wärmestrahlung in dem betrachteten Bereich erreicht werden.

In dem betrachteten Bereich wird gerade ein Teil derjenigen von der Vorrichtung bereitgestellten Wärmestrahlung betrachtet, die zum Zuführen an die Hautpartie verwendbar ist. Dadurch adressiert die vorgeschlagene Vorrichtung auch tatsächlich gerade die Wärmestrahlung (indem diese Wärmestrahlung eine homogene Intensität aufweist), auf die es maßgeblich bei einer Wärmebehandlung ankommt.

Der betrachtete Bereich erstreckt sich dabei mitunter gemäß dem Verlauf der ersten und zweiten Richtungen und verläuft daher insbesondere innerhalb oder parallel zu der spezifischen Transversalebene.

Eine homogene Intensität der Wärmestrahlung in dem betrachteten Bereich erlaubt damit eine zuverlässige Behandlung der Hautpartie mit Wärme, da zum Beispiel durch die Betriebstemperaturen die Wärmezufuhr innerhalb bestimmter Grenzen gehalten werden kann.

Gelegentlich wird in dieser Anmeldung der Einfachheit halber auch von einem "homogenen Wärmefeld" gesprochen, das innerhalb des betrachteten Bereichs besteht. Damit ist dann die besagte homogene Intensität der Wärmestrahlung gemeint, sofern sich aus dem jeweiligen Zusammenhang nichts anderes ergibt.

Das homogene Wärmefeld ermöglicht dabei nicht nur eine, vor allem hinsichtlich der zulässigen Grenzwerte, besonders kontrollierte Wärmebehandlung. Zusätzlich erlaubt ein Bereich mit einem homogenen Wärmefeld auch eine Toleranz gegenüber einer relativen Bewegung von Vorrichtung und Hautpartie. Denn auch bei einer versehentlichen relativen Verschiebung oder einer ungenauen Positionierung der Vorrichtung relativ zur Hautpartie ist eine zuverlässige Wärmebehandlung durchführbar. Dadurch ist der Einsatz der Vorrichtung anwenderfreundlich und robust.

Durch die unterschiedlichen Betriebstemperaturen ist also eine derartige Überlagerung der Wärmestrahlungs-Beiträge der einzelnen Infrarotheizmodule erreicht oder erreichbar, so dass sich eine homogene Intensität der Wärmestrahlung in dem betrachteten Bereich einstellt.

Unter der Betriebstemperatur eines Infrarotheizmoduls wird dabei vorzugsweise diejenige Temperatur verstanden, mit der das Infrarotheizmodul beim Einsatz der Vorrichtung Wärmestrahlung, insbesondere in einer Richtung hin zur Hautpartie, abstrahlt.

Durch die Wahl der einzelnen Betriebstemperaturen ist die Intensität der von den einzelnen Infrarotheizmodulen innerhalb des betrachteten Bereichs bereitgestellten Wärmestrahlung anpassbar, insbesondere verringerbar oder vergrößerbar.

Beispielsweise kann durch ein Anpassen der Betriebstemperatur eines Infrarotheizmoduls die Wellenlänge des Strahlungsmaximums der von dem Infrarotheizmodul abgestrahlten Wärmestrahlung im Spektrum verschoben werden und damit auch der Anteil der im Infrarotbereich, vorzugsweise im nahen Infrarotbereich, abgegebenen Wärmestrahlung angepasst, insbesondere erhöht oder verringert, werden.

Indem die einzelnen Infrarotheizmodule auf ganz oder teilweise unterschiedlichen Betriebstemperaturen betrieben werden, lässt sich somit innerhalb des betrachteten Bereichs die beschriebene homogene Intensität der Wärmestrahlung, insbesondere bezogen auf die zu bestrahlende Hautpartie, einstellen. Beispielsweise kann auch der absolute Intensitätswert innerhalb des betrachteten Bereichs einstellbar sein, etwa indem die Betriebstemperaturen gemeinsam abgesenkt und/oder erhöht werden.

Die Intensität der Wärmestrahlung in dem betrachteten Bereich ist dabei vorzugsweise dann homogen, wenn der Streuungsparameter

$$\delta = 2 \frac{Imax - Imin}{Imax + Imin},$$

worin $I_{min}$ der Minimalwert und $I_{max}$ der Maximalwert der Intensität der Wärmestrahlung innerhalb des betrachteten Bereichs ist, einen Wert von 1,9 oder kleiner, vorzugsweise von 1,7 oder kleiner, vorzugsweise von 1,5 oder kleiner, vorzugsweise von 1,3 oder kleiner, vorzugsweise von 1,0 oder kleiner, vorzugsweise von 0,7 oder kleiner, vorzugsweise von 0,5 oder kleiner, vorzugsweise von 0,3 oder kleiner, vorzugsweise von 0,1 oder kleiner, aufweist.

Mit anderen Worten liegt eine homogene Intensität der Wärmestrahlung dann vor, wenn innerhalb des betrachteten Bereichs, insbesondere entlang der ersten und zweiten Richtung, die Schwankung der Intensität der Wärmestrahlung entsprechend begrenzt ist.

Vorzugsweise ist beim Einsatz der Vorrichtung diese relativ zu und/oder an dem Lebewesen abgeordnet. Alternativ oder ergänzend sind beim Einsatz der Vorrichtung alle oder einige der Infrarotheizmodule mit einer Spannung beaufschlagt. Dadurch strahlen diese Wärmestrahlung gemäß der dann jeweils bestehenden Betriebstemperatur ab.

Die Betriebstemperatur eines einzelnen Infrarotheizmodules liegt vorzugsweise jeweils zwischen 100 °C und 400 °C, vorzugsweise zwischen 100 °C und 300 °C, vorzugsweise zwischen 150 °C und 300 °C, vorzugsweise zwischen 150 °C und 250 °C oder zwischen 200 °C und 300 °C.

Eine Transversalebene des Lebewesens steht vorzugsweise senkrecht auf der Längsachse des Lebewesens. Im Fall eines aufrechtstehenden Menschen teilt die Transversalebene den menschlichen Körper in einen unteren und einen oberen Anteil. Es gibt beliebig viele parallel zueinander verlaufende Transversalebenen. Eine davon kann als die spezifische Transversalebene definierbar sowie ausgewählt und damit definiert sein.

Es versteht sich von selbst, dass die spezifische Transversalebene nicht Teil der Vorrichtung ist, sondern Mittel zum Zweck, um die Anordnung der Infrarotheizmodule sowie die Richtungen, entlang derer innerhalb des betrachteten Bereichs ein homogenes Wärmefeld besteht, zu definieren.

In einer Ausführungsform ist die Maximal-Intensität der Wärmestrahlung in dem betrachteten Bereich 1000 W/m² oder geringer, vorzugsweise 800 W/m², vorzugsweise 600 W/m² oder geringer, vorzugsweise 500 W/m² oder geringer, vorzugsweise 350 W/m² oder geringer, vorzugsweise 300 W/m² oder geringer, vorzugsweise 200 W/m² oder geringer, vorzugsweise 150 W/m² oder geringer, vorzugsweise 100 W/cm² oder geringer. Optional ist die Minimal-Intensität der Wärmestrahlung in dem betrachteten Bereich 50 W/m² oder größer, vorzugsweise 100 W/m² oder größer, vorzugsweise 200 W/m² oder größer, vorzugsweise 400 W/m² oder größer, vorzugsweise 500 W/m² oder größer, vorzugsweise 700 W/m² oder größer.

In einer Ausführungsform ist die Maximal-Intensität der Wärmestrahlung in dem betrachteten Bereich 200 mW/cm² oder geringer, vorzugsweise 170 mW/cm², vorzugsweise 150 mW/cm² oder geringer, vorzugsweise 130 mW/cm² oder geringer, vorzugsweise 100 mW/cm² oder geringer, vorzugsweise 70 mW/cm² oder geringer, vorzugsweise 50 mW/cm² oder geringer. Optional ist die Minimal-Intensität der Wärmestrahlung in dem betrachteten Bereich 20 mW/cm² oder größer, vorzugsweise 30 mW/cm² oder größer, vorzugsweise 50 mW/cm² oder größer, vorzugsweise 70 mW/cm² oder größer, vorzugsweise 100 mW/cm² oder größer, vorzugsweise 130 mW/cm² oder größer, vorzugsweise 150 mW/cm² oder größer.

Die Intensität der Wärmestrahlung an unterschiedlichen Punkten in dem betrachteten Bereich, insbesondere deren Minimal- und Maximalwert in dem betrachteten Bereich, kann beispielsweise mit dem Messgerät Ophir Vega, Messsensor Ophir 12A-V1, und/oder auf folgende Weise ermittelt werden: Der Messsensor wird in einem Abstand von 1 cm bis 30 cm (bevorzugt sind 4 cm) in einem definierten Raster/Matrix vor der abstrahlenden Vorrichtung, insbesondere vor dem jeweiligen abstrahlenden Element, positioniert. Optional kann die abstrahlende Vorrichtung / Element auch mit einer konstanten Geschwindigkeit in dem definierten Abstand abgefahren werden. Der Messsensor kann die Daten kontinuierlich aufzeichnen und darstellen.

Alternativ oder ergänzend kann vorgesehen sein, dass sich die Infrarotheizmodule entlang einer zur spezifischen Transversalebene senkrecht verlaufenden Richtung streifenförmig erstrecken.

Solche Infrarotheizmodule sind zuverlässig im Betrieb und kostengünstig in der Herstellung.

Alternativ oder ergänzend kann vorgesehen sein, dass beim Einsatz der Vorrichtung die erste Richtung parallel zu einer definierten oder definierbaren Sagittalebene des Lebewesens ist und/oder die erste Richtung eine Abstandsrichtung von der Vorrichtung zu der Hautpartie ist, und sich vorzugsweise der betrachtete Bereich 1 cm oder mehr und/oder 30 cm oder weniger entlang der ersten Richtung erstreckt.

Indem sich der betrachtete Bereich in Abstandsrichtung erstreckt, schwankt die bereitgestellte Intensität der Wärmestrahlung in Abstandsrichtung nicht oder nur innerhalb tolerierbarer Grenzen. Dadurch ist die Vorrichtung robust im Einsatz und die Wärmebehandlung kann zuverlässig durchgeführt werden. Vor allem kann die Vorrichtung dadurch gleichermaßen gut und zuverlässig bei unterschiedlichen Anatomien des Körpers, insbesondere des Rückens eines Menschen, eingesetzt werden. Eine homogene Strahlungsintensität ist damit beispielsweise über einen weiten Bereich eines Hohlkreuzes bereitstellbar.

Die Sagittalebene steht dabei senkrecht auf der spezifischen Transversalebene. Im Fall eines aufrechtstehenden Menschen erstreckt sich eine Sagittalebene von oben nach unten und hinten nach vorne. Vorzugsweise wird vorliegend als Sagittalebene diejenige Ebene betrachtet, die den Menschen mittig in eine linke und eine rechte Hälfte teilt.

Vorzugsweise erstreckt sich der betrachtete Bereich 2 cm oder mehr, vorzugsweise 3 cm oder mehr, vorzugsweise 5 cm oder mehr, vorzugsweise 7 cm oder mehr, vorzugsweise 10 cm oder mehr, vorzugsweise 15 cm oder mehr, vorzugsweise 18 cm oder mehr, vorzugsweise 20 cm oder mehr, vorzugsweise 25 cm oder mehr, entlang der ersten Richtung.

Vorzugsweise erstreckt sich der betrachtete Bereich 25 cm oder weniger, vorzugsweise 20 cm oder weniger, vorzugsweise 15 cm oder weniger, vorzugsweise 10 cm oder weniger, vorzugsweise 7 cm oder weniger, vorzugsweise 5 cm oder weniger, entlang der ersten Richtung.

Beispielsweise kann sich der Betrachtete Bereich zwischen 5 cm und 20 cm entlang der ersten Richtung erstrecken.

Alternativ oder ergänzend kann vorgesehen sein, dass beim Einsatz der Vorrichtung die zweite Richtung eine Umfangsrichtung des die Hautpartie aufweisenden Körperteils oder Körperbereichs des Lebewesens ist und/oder sich der betrachtete Bereich 10 cm oder mehr und/oder 100 cm oder weniger entlang der zweiten Richtung erstreckt.

Indem sich der betrachtete Bereich in Umfangsrichtung erstreckt, kann die Wärmebehandlung zuverlässig durchgeführt werden. Denn die bereitgestellte Intensität der Wärmestrahlung schwankt in Umfangsrichtung nicht oder nur innerhalb tolerierbarer Grenzen. Dadurch ist die Vorrichtung robust im Einsatz.

Die Umfangsrichtung kann sich dabei also entlang der Hautpartie erstrecken.

Die Umfangsrichtung kann dabei auch gekrümmt verlaufen. Dies kann gerade bei einer Hautpartie, die zum Rückenbereich oder dem Kniebereich gehört der Fall sein.

Vorzugsweise erstreckt sich der betrachtete Bereich 15 cm oder mehr, vorzugsweise 20 cm oder mehr, vorzugsweise 25 cm oder mehr, vorzugsweise 30 cm oder mehr, vorzugsweise 40 cm oder mehr, vorzugsweise 50 cm oder mehr, vorzugsweise 60 cm oder mehr, vorzugsweise 70 cm oder mehr, vorzugsweise 70 cm oder mehr, entlang der zweiten Richtung.

Vorzugsweise erstreckt sich der betrachtete Bereich 90 cm oder weniger, vorzugsweise 80 cm oder weniger, vorzugsweise 70 cm oder weniger, vorzugsweise 60 cm oder weniger, vorzugsweise 50 cm oder weniger, vorzugsweise 40 cm oder weniger, vorzugsweise 30 cm oder weniger, entlang der zweiten Richtung.

Beispielsweise kann sich der betrachtete Bereich zwischen 15 cm und 50 cm entlang der zweiten Richtung erstrecken.

Alternativ oder ergänzend kann vorgesehen sein, dass der betrachtete Bereich ein Volumenbereich, vorzugsweise mit wenigstens 50 cm$^3$ und/oder mit höchstens 30000 cm$^3$, ist, und/oder der betrachtete Bereich beim Einsatz der Vorrichtung zumindest teilweise zwischen Vorrichtung und Hautpartie liegt.

Wenn der betrachtete Volumenbereich entsprechend dimensioniert ist, eignet sich die Vorrichtung besonders gut für eine Wärmebehandlung einer Hautpartie des Rückens oder im Kniebereich. Auf diese Weise können nämlich auch großflächigere Wärmebehandlungen sicher und zuverlässig durchgeführt werden, da in einem entsprechend großen Volumenbereich.

Der betrachtete Volumenbereich kann sich dann entlang der ersten und zweiten Richtung sowie einer dritten Richtung erstrecken. Beispielsweise stehen alle drei Richtungen senkrecht aufeinander. Die dritte Richtung kann beispielsweise parallel zu einer Sagittalebene des Lebewesens verlaufen.

Wenn beispielsweise die erste oder zweite Richtung gekrümmt verläuft, kann auch der Volumenbereich eine entsprechend gekrümmte Form aufweisen. Damit kann der Volumenbereich sehr gut an die Gegebenheiten angepasst werden und somit ist dann auch die Wärmestrahlung in einem für diese Gegebenheit sinnvollem Bereich homogen.

Beispielsweise kann ein Koordinatensystem, etwa ein kartesisches Koordinatensystem, betrachtet werden, dessen Nullpunkt mittig, beispielsweise am Schwerpunkt, in der Vorrichtung liegt. Dann kann vorzugsweise definiert sein, dass die erste Richtung (beispielsweise in Form einer Abstandsrichtung) entlang der x-Achse, die zweite Richtung entlang der y-Achse und die dritte Richtung entlang der z-Achse verläuft. Beim Einsatz der Vorrichtung erstreckt sich der betrachtete Bereich dann vorzugsweise (a) zumindest abschnittsweise zwischen den absoluten Werten von +1 cm und +35 cm, vorzugsweise von +1 cm und +20 cm, entlang der x-Achse, (b) zumindest abschnittsweise zwischen den absoluten Werten von +50 cm und -50 cm, vorzugsweise von +32,5 cm und -32,5 cm, entlang der y-Achse, und/oder (c) zumindest abschnittsweise zwischen den absoluten Werten von +20 cm und -20 cm, vorzugsweise von +10 cm und - 10 cm, entlang der z-Achse.

Der betrachtete Bereich kann also einem Volumenbereich entsprechen, der sich entlang der x-Achse von +5 cm bis +20 cm erstreckt (also sozusagen "vor" der Vorrichtung und zwischen Hautpartie und Vorrichtung liegt, wenn die positive x-Achse in Richtung der Hautpartie zeigt), der sich entlang der y-Achse (symmetrisch) von +30 cm bis -30 cm erstreckt, und der sich entlang der z-Achse (symmetrisch) von -10 cm bis +10 cm erstreckt.

Die Ausführungen können selbstverständlich auch auf den Fall angewendet werden, dass der betrachtete Bereich zweidimensional ist, wobei dann eine Achse, etwa die z-Achse, des kartesischen Koordinatensystems unberücksichtigt bleibt.

Beispielsweise beginnt der betrachtete Bereich 1 cm vor dem äußersten Element der Vorrichtung.

Im Fall eines Volumenbereichs liegt eine homogene Intensität der Wärmestrahlung in diesem Bereich vorzugsweise dann vor, wenn innerhalb des betrachteten Bereichs, insbesondere entlang der ersten, der zweiten Richtung und der dritten Richtung, die Schwankung der Intensität der Wärmestrahlung entsprechend der oben genannten Ungleichung begrenzt ist.

Alternativ oder ergänzend kann vorgesehen sein, dass der betrachtete Bereich sich seitlich entlang zumindest zweier benachbarter Infrarotheizmodule zumindest teilweise erstreckt.

Der betrachtete Bereich kann sich dann beispielsweise seitlich zur Haupterstreckung der Vorrichtung erstrecken. Dadurch weist also mit anderen Worten der betrachtete Bereich auch zumindest einen Übergangsbereich entlang zumindest zweier Infrarotheizmodule auf. Der Übergangsbereich befindet sich also lateral zu (also neben) der Vorrichtung.

Das ist sehr vorteilhaft, da sich auch die Hautpartie entlang mehrere Infrarotheizmodule erstreckt.

Dadurch ist also auch in solch einem Übergangsbereich eine Schwankung der Intensität der Wärmestrahlung ausgeschlossen oder zumindest begrenzt. Dadurch kann die Vorrichtung besonders einfach aus mehreren einzelnen Modulen aufgebaut sein, ohne dass es zu lokalen Hotspots auf der Hautpartie kommt. Dies wiederum ermöglicht es, den Verlauf der Hautpartie durch mehrere Infrarotheizmodule besonders gut nachzubilden. Dadurch steigert sich die Effizienz der möglichen Wärmebehandlung.

Alternativ oder ergänzend kann vorgesehen sein, dass beim Einsatz der Vorrichtung die Betriebstemperatur der einzelnen Infrarotheizmodule jeweils eingestellt oder einstellbar ist auf zwischen 100 °C und 400 °C, vorzugsweise zwischen 100 °C und 300 °C, vorzugsweise zwischen 150 °C und 300 °C, vorzugsweise zwischen 150 °C und 250 °C oder zwischen 200 °C und 300 °C.

Beispielsweise kann sich die Betriebstemperatur der beiden, vorzugsweise in der spezifischen Transversalebene, seitlichen Infrarotheizmodule jeweils zwischen 5 °C und 100 °C, vorzugsweise zwischen 5 °C und 50 °C, vorzugsweise zwischen 5 °C und 30 °C, von der höchsten Betriebstemperatur der übrigen Infrarotheizmodule unterscheiden. Damit wird eine besonders homogene Wärmebehandlung erreicht.

Alternativ oder ergänzend kann vorgesehen sein, dass beim Einsatz der Vorrichtung die Betriebstemperatur der beiden, vorzugsweise in der spezifischen Transversalebene, seitlichen Infrarotheizmodule jeweils (a) bis zu 50 %, vorzugsweise bis zu 40 %, vorzugsweise bis zu 30 %, vorzugsweise bis zu 20 %, vorzugsweise bis zu 10 %, größer eingestellt oder einstellbar ist als die höchste Betriebstemperatur der übrigen Infrarotheizmodule und/oder (b) 1% oder mehr, vorzugsweise 5 % oder mehr, vorzugsweise 10 % oder mehr, größer eingestellt oder einstellbar ist als die höchste Betriebstemperatur der übrigen Infrarotheizmodule.

Auf diese Weise kann besonders zuverlässig ein homogenes Wärmefeld erhalten werden.

Es ist dabei bevorzugt, dass die äußersten Infrarotheizmodule die höchste Betriebstemperatur aller Infrarotheizmodule aufweisen und dass die Betriebstemperaturen der übrigen Infrarotheizmodule nach innen hin abnimmt.

Alternativ oder ergänzend kann vorgesehen sein, dass die Betriebstemperatur eines jeden Infrarotheizmoduls mittels jeweils eines Bedienelements, wie einem Schiebeschalter, einem Drehknopf, einem Druckschalter oder einem berührungsempfindlichen Bedienfeld, an dem jeweiligen Infrarotheizmodul einstellbar ist.

Mit anderen Worten kann jedes Infrarotheizmodul ein solches Bedienelement aufweisen. Dadurch kann die Betriebstemperatur eines jeden Infrarotheizmoduls individuell für sich einstellbar sein.

Beispielsweise kann über ein erfindungsgemäßes Bedienelement eine Betriebstemperatur-Stufe zwischen 1 und 5 für jedes Infrarotheizmodul einstellbar sein. Jede Stufe entspricht dabei beispielsweise einer Betriebstemperatur innerhalb eines bestimmten Temperaturbereichs, der beispielsweise von 100 °C bis 400 °C reicht.

Alternativ oder ergänzend kann vorgesehen sein, dass die Vorrichtung ein Kontrollmodul aufweist, das dazu eingerichtet ist, die Betriebstemperaturen der einzelnen Infrarotheizmodulen, vorzugsweise basierend auf zumindest einem von einem Anwender ausgewählten oder auswählbaren Betriebsmodus, zu kontrollieren, insbesondere zu regeln oder zu steuern.

Indem die Betriebstemperaturen durch ein entsprechend eingerichtetes Kontrollmodul kontrolliert werden, also insbesondere eingestellt werden, kann der Betrieb der Vorrichtung zuverlässiger gestalten werden. Denn die Verantwortung für die richtige Einstellung der Betriebstemperaturen wird dem Benutzer abgenommen. Das ermöglicht nicht nur eine sichere Anwendung, sondern ist auch komfortabel für den Benutzer.

Beispielsweise kann der Benutzer einen Betriebsmodus der Wärmebehandlung auswählen, der für ihn eine leicht verständliche Bezeichnung hat, wie etwa "Schonend" oder "Anregend". Das Kontrollmodul ist dann dazu eingerichtet, ein damit verknüpftes Kontrollieren der Betriebstemperaturen durchzuführen. Also beispielsweise auf einer eher geringen absoluten Intensität, oder einer eher hohen absoluten Intensität der Wärmestrahlung innerhalb des betrachteten Bereichs.

Grundsätzlich, also egal auf welche Weise die Betriebstemperatur einstellbar ist, kann es in einer Ausführungsform vorteilhaft sein, dass die Betriebstemperatur der einzelnen Infrarotheizmodule jeweils zwischen 100 °C und 400 °C, vorzugsweise zwischen 100 °C und 300 °C, vorzugsweise zwischen 150 °C und 300 °C, vorzugsweise zwischen 150 °C und 250 °C oder zwischen 200 °C und 300 °C, liegt. Dadurch besteht ein ausreichend großer Spielraum, um zuverlässig ein homogenes Wärmefeld zu erreichen, und wobei gleichzeitig die absoluten Intensitäten innerhalb akzeptabler Grenzwerte einstellbar sind.

Alternativ oder ergänzend kann vorgesehen sein, dass das Kontrollmodul dazu eingerichtet ist, wenigstens die beiden beim Einsatz der Vorrichtung seitlichen Infrarotheizmodule auf einer gleichen Betriebstemperatur zu betreiben und/oder wenigstens zwei der Infrarotheizmodule mit einer unterschiedlichen Betriebstemperatur zu betreiben.

Alternativ oder ergänzend kann vorgesehen sein, dass das Kontrollmodul dazu eingerichtet ist, die beiden beim Einsatz der Vorrichtung seitlichen Infrarotheizmodule mit gleicher Betriebstemperatur zu betreiben, die größer ist als jede der Betriebstemperaturen, auf denen die übrigen Infrarotheizmodule betrieben werden.

Indem die seitlichen Infrarotheizmodule auf einer höheren Betriebstemperatur als die übrigen Infrarotheizmodule betrieben werden, kann ein Abfall der bereitgestellten Intensität der Wärmestrahlung zu den Seiten hin zuverlässig reduziert oder vermieden werden. Außerdem kann auf diese Weise im Übergangsbereich von einem vorletzten Infrarotheizmodul zu dem letzten, also seitlichen Infrarotheizmodul, überraschenderweise mit besonders einfachen Mitteln zuverlässig ein homogenes Wärmefeld bereitgestellt werden.

Dabei kann es vorteilhaft sein, wenn der betrachtete Bereich sich dann zumindest teilweise entlang zumindest eines der seitlichen Infrarotheizmodul, vorzugsweise beider seitlicher Infrarotheizmodule, erstreckt.

Die seitlichen Infrarotheizmodule können dabei die in der spezifischen Transversalebene äußersten Infrarotheizmodule sein.

Beispielsweise kann die Betriebstemperatur der beiden seitlichen Infrarotheizmodule bis zu 50 %, vorzugsweise bis zu 40 %, vorzugsweise bis zu 30 %, vorzugsweise bis zu 20 %, vorzugsweise bis zu 10 %, größer sein als die höchste Betriebstemperatur der übrigen Infrarotheizmodule. Optional ist die Betriebstemperatur der beiden seitlichen Infrarotheizmodule aber 5 % oder mehr, vorzugsweise 10 % oder mehr, größer als die höchste Betriebstemperatur der übrigen Infrarotheizmodule.

Alternativ oder ergänzend kann vorgesehen sein, dass die Vorrichtung einen oder mehrere Sensoren aufweist, die dazu eingerichtet sind, jeweils eine lokale Temperatur der Hautpartie zu messen und wobei das Kontrollmodul dazu eingerichtet ist, zumindest basierend auf den gemessenen Temperaturwerten die Betriebstemperaturen der einzelnen Infrarotheizmodule zu kontrollieren, insbesondere zu regeln oder zu steuern.

Indem die Betriebstemperaturen basierend auf der konkreten Hauttemperatur kontrolliert werden, lassen sich lokale Hotspots auf der Hautpartie zuverlässig vermeiden oder zumindest reduzieren.

Beispielsweise kann durch den Sensor ein, insbesondere ausreichend großer, vernarbter Bereich der Hautpartie erkannt werden und die Betriebstemperaturen der Infrarotheizmodule entsprechend angepasst werden, um eine zu hohe Belastung des vernarbten Hautbereichs zu vermeiden. So lässt sich damit etwa kontrolliert die Intensität der Wärmestrahlung und damit die der Haut zugeführten Wärme reduzieren. Dies kann mit den Temperatursensoren sehr zuverlässig erkannt werden, da vernarbte Hautpartien eine andere Temperatur aufweisen als intakte Hautpartien.

Vorzugsweise ist also das Kontrollmodul dazu eingerichtet, die bereitgestellte Intensität der Wärmestrahlung basierend auf der Temperatur des empfindlichsten Hautbereichs einzustellen.

Dadurch kann sich zwar die absolute Intensität der Wärmestrahlung in dem betrachteten Bereich insgesamt reduzieren, also auch für an sich intakte Bereiche der Hautpartie; Allerdings kann dies vorteilhaft sein, um geschädigte Hautpartien schonend mit Wärme behandeln zu können.

So kann das Kontrollmodul beispielsweise dazu eingerichtet sein, die Betriebstemperaturen der einzelnen Infrarotheizmodule derart zu kontrollieren, dass die gemessenen Temperaturen eine Mindest-Temperatur nicht unterschreiten und/oder eine Maximal-Temperatur nicht überschreiten. Die Temperaturen stammen dabei von dem oder den Sensoren. Die Vorrichtung kann dadurch im Einsatz noch zuverlässiger und sicherer gemacht werden.

Das homogene Wärmefeld im betrachteten Bereich führt dabei zu einer höheren Sicherheit, dass eine nur lokal gemessene Temperatur der Hauptpartie auch für andere Regionen der Hautpartie Gültigkeit hat und dadurch eine zuverlässige Wärmebehandlung durchführbar ist.

Beispielsweise kann der Sensor in einer Ausführungsform dazu eingerichtet sein, die Hauptpartie abzuscannen und dadurch vorzugsweise mehrere ortsaufgelöste Temperaturwerte bereitzustellen.

Die Sensoren können beispielsweise in Form von Infrarottemperatursensoren ausgebildet sein. Dabei kann jeder Sensor bei dem Einsatz der Vorrichtung ein bestimmtes Sichtfeld auf die Hautpartie haben. Die Sichtfelder einiger Sensoren können sich jeweils zumindest teilweise überlappen. Dadurch können die lokalen Temperaturen der Hautpartie zuverlässig gemessen werden.

Beispielsweise können die Sensoren zusammen einen, vorzugsweise zusammenhängenden, Bereich der Hautpartie von bis zu 600 mm in einer Richtung und von bis zu 180 mm in eine andere Richtung erfassen. Alternativ oder ergänzend können die Temperaturen auf der Hautpartie für Hautbereiche mit einer längsten Aus-

... dehnung im sub-Zentimeterbereich, vorzugsweise im sub-Millimeterbereich, erfasst werden.

Vorzugsweise sind die Sensoren verschiebbar innerhalb der Vorrichtung vorgesehen. Dadurch kann besonders einfach der überwachte Hautbereich an den jeweiligen Anwendungsfall angepasst werden. Beispielsweise können die Sensoren vertikal und/oder horizontal verschiebbar sein.

Alternativ oder ergänzend kann vorgesehen sein, dass die Sensoren matrixförmig angeordnet sind.

Dadurch lässt sich die Hautpartie sozusagen flächig abrastern, da jeder Sensor die Temperatur eines bestimmten Bereichs der Hautpartie ermittelt. Vorzugsweise sind die Erfassungsbereiche benachbarter Sensoren zumindest teilweise überlappend. Dadurch kann eine vollständige Erfassung der Hautpartie erreicht werden.

Anstelle einiger oder sogar aller der matrixförmig angeordneten Sensoren könnte auch ein 2D-Sensor vorgesehen sein, vorzugsweise weist dieser dann eine entsprechend große Auflösung auf. Mit anderen Worten, der 2D-Sensor könnte alternativ oder ergänzend zu matrixförmig angeordneten Sensoren vorgesehen sein. Es können aber auch mehrere 2D-Sensoren matrixförmig angeordnet sein.

Alternativ oder ergänzend kann vorgesehen sein, dass wenigstens zwei der Infrarotheizmodule relativ zueinander verschiebbar sind, insbesondere in einer Richtung, die beim Einsatz der Vorrichtung parallel zu der spezifischen Transversalebene verläuft.

Dadurch kann die Vorrichtung für verschiedene Körperbereiche des Lebewesens verwendet werden, da die Module leicht anpassbar sind. Auch ist es dadurch möglich, je nach Körperstatur, die Vorrichtung in eine passende Konfiguration zu überführen.

Optional kann die Vorrichtung zumindest ein Führungselement aufweisen, mittels dessen die Einheiten relativ zueinander veränderlich positionierbar sind, insbesondere (a) das Führungselement ein Teleskopauszug aufweist und die Einheiten an unterschiedlichen Segmenten des Teleskopauszugs angeordnet sind und/oder (b) das Führungselement eine Schiene aufweist, entlang derer die Einheiten zumindest abschnittsweise bewegbar und/oder mittels einer Rast-, Klemm-, und/oder Reibverbindung arretierbar sind.

Alternativ oder ergänzend kann vorgesehen sein, dass wenigstens zwei, vorzugsweise drei oder mehr als drei, der Infrarotheizmodule relativ zueinander neigbar sind.

Alternativ oder ergänzend kann vorgesehen sein, dass wenigstens zwei, vorzugsweise drei oder mehr als drei, der Infrarotheizmodule relativ zueinander abwinkelbar sind.

Die Möglichkeit, Infrarotheizmodule gegeneinander zu neigen oder abzuwinkeln ist gerade für die Wärmebehandlung von gekrümmten Hautpartien, wie auf dem Rücken oder am Knie, besonders vorteilhaft.

Der Krümmungswinkel liegt dabei vorzugsweise in der spezifischen Transversalebene.

Alternativ oder ergänzend kann vorgesehen sein, dass wenigstens zwei benachbarte Infrarotheizmodule, vorzugsweise alle paarweise benachbarten Infrarotheizmodule, thermisch voneinander entkoppelt sind, vorzugsweise durch einen Luftspalt, ein thermisches Isoliermaterial, und/oder ein Aerogel, das beispielsweise zwischen den Infrarotheizmodulen zumindest bereichsweise angeordnet ist.

Durch die Entkopplung kann ein Wärmefluss zwischen benachbarten Infrarotheizmodulen vermieden oder zumindest reduziert werden. Dadurch kann das homogene Wärmefeld, also eine homogene Intensität der Wärmestrahlung, noch besser und gezielter innerhalb des betrachteten Bereichs ausgebildet werden, da störende Einflüsse durch einen Wärmefluss zwischen den Modulen nicht oder in nicht mehr nennenswertem Ausmaß bestehen und ein solcher Wärmefluss daher nicht näher berücksichtigt werden muss.

Die Wärmeleitfähigkeit des thermischen Entkopplungsmittels beträgt vorzugsweise 0.05 W / mK oder weniger, vorzugsweise 0.03 W / mK oder weniger, vorzugsweise 0.02 W / mK oder weniger, vorzugsweise 0.01 W / mK oder weniger.

Alternativ oder ergänzend kann vorgesehen sein, dass jedes Infrarotheizmodul zumindest ein Infrarotstrahlerelement, insbesondere in Form eines Heizdrahtes, einer Folienheizung, eines Heizgewebes, von Mikanit, einer Dickschichtheizung und/oder einer Silikonheizmatte, aufweist oder darstellt. Insbesondere kann jedes Infrarotheizmodul zumindest ein Infrarotstrahlerelement in Form eines Carbongewebes aufweisen.

Ein Infrarotstrahlerelement ist vorliegend vorzugsweise ein Infrarotstrahler oder weist diesen auf. Ein Heizdraht ist ein günstiger und gleichzeitig robuster und zuverlässiger Infrarotstrahler. Ein Beispiel für eine Folienheizung ist eine Infrarot Wärmefolie.

Ein Infrarotstrahlerelement kann ein Teil mit unterschiedlichen Energiezonen sein oder mehrere Teile mit gleicher oder unterschiedlicher Leistung. Beispielsweise ist ein Infrarotstrahlerelement mit einer Elektronik ansteuerbar oder angesteuert und/oder oder jede Zone ist einzeln angesteuert oder ansteuerbar.

Je nach erforderlicher Betriebstemperatur können unterschiedliche Realisierungen bevorzugt sein. Eine Silikonheizmatte kann für Temperaturen bis 250 °C bevorzugt sein. Eine Folienheizung kann für Temperaturen bis 150 °C bevorzugt sein. Mikanit kann für Temperaturen bis 450 °C bevorzugt sein.

Ein Infrarotstrahlerelement kann dabei eine Ausgangs-Wärmestrahlung des jeweiligen Infrarotheizmoduls bereitstellen. Die Ausgangs-Wärmestrahlung kann qualitativ und/oder quantitativ identisch zu der Wärmestrahlung sein, die das Infrarotheizmodul seinerseits abstrahlt. Dies kann beispielsweise der Fall sein, wenn das Infrarotheizmodul identisch zu dem Infrarotstrahlerelement ist. Die Ausgangs-Wärmestrahlung kann auch in einem oder mehreren qualitativen oder quantitativen Punkten unterschiedlich zu der Wärmestrahlung sein, mit der das Infrarotheizmodul seinerseits abstrahlt. Dies kann beispielsweise der Fall sein, wenn das Infrarotheizmodul die von dem Infrarotstrahlerelement bereitgestellte Ausgangs-Wärmestrahlung modifiziert, etwa mittels Filter, Schirmungen, Isolierungen und/oder anderer Mechanismen wie ein durch die Wärmestrahlung aufwärmbares Element, das dann seinerseits Wärmestrahlung abstrahlt. Dieses Modifizieren kann ein Umlenken der Wärmestrahlung, ein Abschwächen der Wärmestrahlung und/oder ein Filtern von spektralen Anteilen der Wärmestrahlung aufweisen. Alternativ oder ergänzend kann ein Infrarotstrahlerelement eine Ausgangs-Wärmequelle des jeweiligen Infrarotheizmoduls darstellen. Das Infrarotstrahlerelement kann dann ein anderes Element des Infrarotheizmoduls aufwärmen, indem Wärme durch Konduktion von dem Infrarotstrahlerelement auf das andere Element übertragbar ist und/oder übertragen wird. Das andere Element kann dann beispielsweise seinerseits Wärmestrahlung abstrahlen und damit die von dem Infrarotheizmodul ausgestrahlte Wärmestrahlung ganz oder teilweise bereitstellen und/oder beeinflussen.

Indem das Infrarotheizmodul seinerseits ein Infrarotstrahlerelement aufweist, kann also vorteilhafterweise die von dem Infrarotheizmodul bereitgestellte, insbesondere an die Hautpartie zugeführte, Wärmestrahlung ausgehend von der Ausgangs-Wärmestrahlung und/oder von der Ausgangs-Wärmequelle des Infrarotstrahlerelement einstellbar sein.

Das Infrarotstrahlerelement stellt daher in einer Ausführungsform zumindest einen Teil der von dem Infrarotheizmodul abgestrahlten, und insbesondere an die Hautpartie zugeführten, Wärmestrahlung direkt oder indirekt (zum Beispiel durch Wärmestrahlung oder Konduktion) bereit.

In einer Ausführungsform ist das Infrarotheizmodul aber identisch zu dem Infrarotstrahlerelement. Dadurch ist die Vorrichtung kompakt im Aufbau.

Im Fall eines Heizdrahtes ist der Heizdraht vorzugsweise auf einem Trägermaterial, wie beispielsweise eine Textilie, angeordnet.

Es kann aber auch vorteilhaft sein, wenn das Infrarotstrahlerelement symmetrisch um die Mittelachse des Infrarotheizmoduls angeordnet ist. Besonders bevorzugt ist es dann, wenn die Temperatur, mit der das Infrarotstrahlerelement abstrahlt, entlang der Mittelachse die geringste Temperatur aufweist, die nach außen hin zunimmt.

Beispielsweise kann jedes Infrarotheizmodul zwei, drei, vier, fünf, sechs, sieben, acht, neun, zehn oder mehr als zehn Infrarotstrahlerelemente aufweisen. Dabei kann jedes Infrarotheizmodul die gleiche Anzahl Infrarotstrahlerelemente aufweisen, oder jedes Infrarotheizmodul kann eine individuelle Anzahl, insbesondere gemäß den vorstehenden Zahlenwerten, Infrarotstrahlerelemente aufweisen.

Beispielsweise können drei Infrarotheizmodule vorgesehen sein und jedes davon weist ein oder mehrere Infrarotstrahlerelemente auf. Die Infrarotstrahlerelemente der beiden seitlichen Infrarotheizmodule werden derart betrieben, dass die Infrarotheizmodule auf der gleichen Betriebstemperatur betrieben werden. Diese kann beispielsweise 250 °C betragen. Das oder die Infrarotstrahlerelemente des mittleren Infrarotheizmoduls wird derart betrieben, dass das Infrarotheizmodul auf einer kleineren Betriebstemperatur betrieben wird als die übrigen beiden. Diese kann beispielsweise 200 °C betragen.

Alternativ oder ergänzend kann vorgesehen sein, dass jedes Infrarotheizmodul ein Gehäuse oder Gehäuseabschnitt aufweist, innerhalb dessen vorzugsweise das jeweilige Infrarotstrahlerelement wie zuvor beschrieben angeordnet ist.

Das Gehäuse macht die Vorrichtung robust und damit sicher im Einsatz und ermöglicht ein sicheres Unterbringen aller, auch elektrischen, Komponenten.

Das Gehäuse kann eine Öffnung oder einen Bereich aufweisen, von der bzw. von dem ausgehend Wärmestrahlung in Richtung der Hautpartie abstrahlbar ist und/oder beim Einsatz der Vorrichtung abgestrahlt wird. Beim Einsatz der Vorrichtung ist dann vorzugsweise diese Öffnung oder dieser Bereich zumindest bereichsweise vor der Hautpartie angeordnet.

Das Gehäuse kann beispielsweise PU-Schaum aufweisen oder daraus bestehen. In dem Gehäuse können Sensoren und andere Elektronik integriert sein. Das Gehäuse kann auch einen Schweißablauf aufweisen.

In einer Ausführungsform ist eine thermische Isolierung zwischen dem Infrarotstrahlerelement und einem Abschnitt des Gehäuses vorgesehen. Beispielsweise kann hierzu ein Aerogel, insbesondere eine Schicht aus Aerogel, vorgesehen sein. Die thermische Isolierung, insbesondere die Aerogelschicht, kann eine Dicke von 5-20 mm, beispielsweise 10 mm, aufweisen. Durch die thermische Isolierung kann das Aufwärmen des Gehäuses vermieden oder zumindest reduziert werden. Dadurch wird die Effizient des Infrarotheizmoduls gesteigert sowie die Handhabung sicherer gestaltet.

Alternativ oder ergänzend kann vorgesehen sein, dass die Vorrichtung ein Gehäuse aufweist, innerhalb dessen die Infrarotheizmodule angeordnet sind.

Das Gehäuse der gesamten Vorrichtung macht die Vorrichtung robust und damit sicher im Einsatz.

Gemäß einem zweiten Aspekt ist ein Infrarotheizmodul zum Zuführen von Wärme an eine Hautpartie eines Lebewesens offenbart, aufweisend ein Gehäuse, zumindest ein innerhalb des Gehäuses angeordnetes Infrarotstrahlerelement, zumindest ein Arretierungsmittel und ein mit dem Arretierungsmittel lösbar in und/oder an dem Gehäuse und/oder an dem Infrarotstrahlerelement anordenbares Abstrahlelement.

Der Erfindung liegt damit die überraschende Erkenntnis zugrunde, dass der Hautpartie die Wärmestrahlung besonders kontrolliert bereitgestellt werden kann, indem die Abstrahlcharakteristik des Infrarotheizmoduls passend eingestellt wird. Dadurch kann zuverlässig eine definierte Wärmebehandlung der Hautpartie erreicht werden, im Einklang mit der gewünschten Anwendung. Dadurch wird eine verbesserte Wärmeanwendung ermöglicht.

Die Erfinder haben insoweit erkannt, dass eine anpassbare Abstrahlcharakteristik des Infrarotheizmoduls besonders einfach dadurch erreicht werden kann, indem ein Abstrahlelement vorgesehen ist. Dieses kann die von dem Infrarotstrahlerelement bereitgestellte Wärmestrahlung und/oder die von dem Infrarotstrahlerelement auf das Abstrahlelement per Konduktion übertragene Wärme beispielsweise aufnehmen, insbesondere, indem es durch diese Strahlung und/oder Wärme erwärmt wird, und anschließend seinerseits Wärmestrahlung abstrahlen. Dadurch ist mittels des Abstrahlelements die Abstrahlcharakteristik des Infrarotheizmoduls anpassbar und/oder einstellbar.

Damit ist durch das Infrarotheizmodul eine Wärmestrahlung bereitstellbar, die beispielsweise auch hinsichtlich einer möglichst homogenen Intensität innerhalb eines Bereichs, wie einem Volumenbereich, der beim Einsatz des Infrarotheizmoduls zumindest teilweise zwischen Infrarotheizmodul und Hautpartie ist, einstellbar ist.

So kann, je nach Anwendungsfall, ein Abstrahlelement vorgesehen werden, das zu einer jeweils passenden Wärmestrahlung führt. Beispielsweise kann die Wärmestrahlung hinsichtlich Abstrahlrichtung und/oder Intensität innerhalb eines Bereichs, der vorzugsweise beim Einsatz des Infrarotheizmoduls zwischen Infrarotheizmodul und Hautpartie liegt, durch das Abstrahlelement veränderlich sein.

Da das Abstrahlelement lösbar an dem Gehäuse angeordnet ist, lässt sich das Abstrahlelement besonders einfach und ohne großen Aufwand austauschen und so die Abstrahlcharakteristik an einen neuen Anwendungsfall anpassen. Je nach Wahl des Arretierungsmittels ist dies sogar ohne zusätzliches Werkzeug möglich, wodurch die Handhabung sehr einfach ist. Das Infrarotheizmodul lässt sich dadurch schnell für einen neuen Anwendungsfall umrüsten.

Daher ist vorzugsweise das Abstrahlelement mit dem Arretierungsmittel ohne zusätzliches Werkzeug in und/oder an dem Gehäuse anordenbar.

Das Abstrahlelement erlaubt es auch, den Einfluss des konkret vorgesehenen Infrarotstrahlerelements und/oder der konkreten Anordnung des Infrarotstrahlerelements innerhalb des Gehäuses zu reduzieren. Denn das Abstrahlelement kann die Charakteristiken des Infrarotstrahlerelements anpassen. Beispielsweise kann ein linienförmiges Infrarotstrahlerelement eingesetzt werden, für das mittels des Abstrahlelements sehr einfach ein eher flächiges Abstrahlen erreicht werden kann. Dies ermöglicht es, dass in die Wahl des Infrarotstrahlerelements sowie dessen Anordnung innerhalb des Gehäuses auch andere Kriterien als die zur Erreichung einer konkreten Abstrahlcharakteristik fließen können. Dadurch können beispielsweise günstigere Elemente ausgewählt werden.

Das Abstrahlelement kann beispielsweise aus einem Metall, wie etwa Aluminium, sein. Dadurch ist eine besonders gute Wärmeleitfähigkeit gegeben, so dass das Abstrahlelement gut erwärmbar ist. Optional weist das Abstrahlelement ein Ofenlack und/oder eine Eloxalschicht auf oder ist damit zumindest bereichsweise beschichtet.

Vorzugsweise liegt beim Einsatz des Infrarotheizmoduls und von der Hautpartie aus betrachtet das Infrarotstrahlerelement hinter dem Abstrahlelement.

Vorzugsweise erstreckt sich das Abstrahlelement entlang seiner Haupterstreckungsrichtung 5 cm oder mehr, vorzugsweise 10 cm oder mehr, vorzugsweise 15 cm oder mehr, vorzugsweise 20 cm oder mehr, vorzugsweise 25 cm oder mehr, vorzugsweise 30 cm oder mehr, vorzugsweise 40 cm oder mehr, vorzugsweise 50 cm oder mehr, vorzugsweise 60 cm oder mehr, vorzugsweise 70 cm oder mehr, vorzugsweise 70 cm oder mehr.

Vorzugsweise erstreckt sich das Abstrahlelement entlang seiner Haupterstreckungsrichtung 90 cm oder weniger, vorzugsweise 80 cm oder weniger, vorzugsweise 70 cm oder weniger, vorzugsweise 60 cm oder weniger, vorzugsweise 50 cm oder weniger, vorzugsweise 40 cm oder weniger, vorzugsweise 30 cm oder weniger, vorzugsweise 20 cm oder weniger, vorzugsweise 15 cm oder weniger, vorzugsweise 10 cm oder weniger.

Beispielsweise hat das Abstrahlelement entlang seiner Haupterstreckungsrichtung eine Abmessung von zwischen 5 cm und 40 cm.

Außerdem ist einer oder mehrere der folgenden Aspekte besonders vorteilhaft:

- Das Abstrahlelement ist derart innerhalb des Gehäuses angeordnet, dass es von dem Infrarotstrahlerelement, insbesondere durch dessen beim Einsatz des Infrarotstrahlerelements abgestrahlte Wärmestrahlung und/oder von diesem per Konduktion übertragene Wärme, aufwärmbar ist. Dadurch kann es anschließend wieder Wärmestrahlung abgeben.

- Das Abstrahlelement ist zumindest teilweise vor, innerhalb und/oder hinter einer Öffnung des Gehäuses angeordnet und/oder verschließt eine Öffnung des Gehäuses zumindest teilweise. Dadurch kann das Abstrahlelement sozusagen "Innen" von "Außen" trennen.

- Das Abstrahlelement ist zumindest bereichsweise membranförmig und/oder flächig ausgebildet.

Vorzugsweise ist beim Einsatz des Infrarotheizmoduls dieses relativ zu und/oder an dem Lebewesen abgeordnet. Alternativ oder ergänzend ist beim Einsatz des Infrarotheizmoduls das Infrarotstrahlerelement mit einer Spannung beaufschlagt. Dadurch strahlt dieses Wärmestrahlung ab, insbesondere gemäß der jeweils bestehenden Betriebstemperatur des Infrarotstrahlerelements.

Es kann besonders bevorzugt sein, wenn das Infrarotstrahlerelement Merkmale des in Bezug auf den ersten Aspekt der Erfindung beschriebenen Infrarotstrahlerelements aufweist.

Alternativ oder ergänzend kann vorgesehen sein, dass beim Einsatz des Infrarotheizmoduls das Abstrahlelement zumindest bereichsweise zwischen dem Infrarotstrahlerelement und der Hautpartie angeordnet ist.

Beispielsweise kann dazu das Abstrahlelement zumindest teilweise vor, innerhalb und/oder hinter einer Öffnung des Gehäuses angeordnet und/oder das Abstrahlelement eine Öffnung des Gehäuses zumindest teilweise verschließen.

Alternativ oder ergänzend kann vorgesehen sein, dass das Arretierungsmittel in Form eines Schnellspannrahmens ausgebildet ist und vorzugsweise der Schnellspannrahmen mit dem Gehäuse verliersicher verbunden ist und/oder von dem Gehäuse bereitgestellt ist.

Mit einem Schnellspannrahmen kann das Abstrahlelement sehr schnell und einfach gewechselt werden.

Beispielsweise kann das Abstrahlelement dazu innerhalb eines Gehäusebereichs, der als Schnellspannrahmen fungiert, eingespannt werden. Mit anderen Worten, das Abstrahlelement kann von außen, insbesondere durch die Öffnung, innerhalb des Gehäuses anordenbar sein, indem es an einer vorgesehenen Stelle innerhalb des Gehäuses einspannbar ist. Dadurch ist das Abstrahlelement sehr einfach und ohne Werkzeug auswechselbar.

Beispielsweise kann der Schnellspannrahmen mehrteilig ausgeführt sein und vorzugsweise ein oder mehrere Komponenten des Infrarotheizmoduls zumindest bereichsweise sandwichartig zwischen den einzelnen Teilen des Schnellspannrahmens einspannen.

Der Schnellspannrahmen kann beispielsweise aus Blech sein oder ein Blech aufweisen.

Alternativ oder ergänzend kann vorgesehen sein, dass beim Einsatz des Infrarotheizmoduls das Abstrahlelement und das Infrarotstrahlerelement derart zueinander angeordnet sind, dass das Abstrahlelement durch das Infrarotstrahlerelement aufgewärmt wird und dann seinerseits Wärmestrahlung abgibt, insbesondere zumindest teilweise in Richtung der Hautpartie beim Einsatz des Infrarotheizmoduls, wobei vorzugsweise das Aufwärmen des Abstrahlelements zumindest teilweise durch zumindest einen Teil der von dem Infrarotstrahlerelement abgestrahlten Wärmestrahlung und/oder durch Konduktion von dem Infrarotstrahlerelement auf das Abstrahlelement erfolgt.

Indem das Abstrahlelement also eine gewissermaßen vermittelnde Stellung zwischen "Innen" und "Außen" einnimmt, ist die von dem Infrarotheizmodul, insbesondere in Richtung der Hautpartie, abgegebene Wärmestrahlung überraschenderweise besonders einfach und dennoch wirkungsvoll beeinflussbar.

Das Abstrahlelement ist also beispielsweise durch die Wärmestrahlung des Infrarotstrahlerelements und/oder durch per Konduktion von dem Infrarotstrahlerelement auf das Abstrahlelement übertragene Wärme erwärmbar. Wenn das Abstrahlelement erwärmt ist, strahlt es dann seinerseits Wärmestrahlung ab.

Alternativ oder ergänzend kann vorgesehen sein, dass das Arretierungsmittel von einer Entnahmestellung, in der vorzugsweise das Abstrahlelement von dem Gehäuse lösbar ist, in eine Arretierstellung, in der vorzugsweise das Abstrahlelement form-, reib- und/oder kraftschlüssig an dem Gehäuse und/oder an dem Infrarotstrahlerelement angeordnet ist und/oder mit dem Infrarotstrahlerelement zumindest bereichsweise in Kontakt steht, und umgekehrt überführbar ist.

Durch die definierten Stellungen des Arretierungsmittels ist eine sichere Handhabung des Infrarotheizmoduls möglich.

Ein besonders guter konduktiver Wärmeübertrag zwischen Infrarotstrahlerelement und Abstrahlelement ist möglich, indem diese in Kontakt stehen. Ein indirekter Kontakt kann dabei beispielsweise bestehen, wenn zwischen Infrarotstrahlerelement und Abstrahlelement beispielsweise eine Schutzschicht oder ein Verbindungsmittel vorgesehen ist. Beispiele hierfür ist eine thermisch leitende Schicht.

Alternativ oder ergänzend kann vorgesehen sein, dass das Arretierungsmittel eine Anlagefläche aufweist, die mit einem Oberflächenbereich des Abstrahlelements in Kontakt bringbar ist und wobei vorzugsweise das Infrarotheizmodul dazu ausgebildet ist, dass, wenn das Arretierungsmittel von der Entnahmestellung in die Arretierstellung überführt wird, das Abstrahlelement durch die mit ihm in Kontakt stehende Anlagefläche gegen das Gehäuse und/oder das Infrarotstrahlerelement gedrückt und dadurch das Abstrahlelement, während sich das Arretierungsmittel in der Arretierstellung befindet, form-, reib- und/oder kraftschlüssig an dem Gehäuse und/oder dem Infrarotstrahlerelement gehalten und/oder in direkten oder indirekten Kontakt mit dem Infrarotstrahlerelement gebracht wird.

Vorzugsweise ist das Abstrahlelement also zumindest bereichsweise sandwichartig zwischen einem Gehäuseteil und dem Arretierungsmittel gehalten, insbesondere wenn das Arretierungsmitel in der Arretierstellung ist. Alternativ oder ergänzend ist das Abstrahlelement zumindest bereichsweise sandwichartig zwischen dem Infrarotstrahlerelement und dem Arretierungsmittel gehalten, insbesondere wenn das Arretierungsmitel in der Arretierstellung ist, wobei vorzugsweise das Abstrahlelement und das Infrarotstrahlerelement in unmittelbarem Kontakt miteinander stehen.

Alternativ oder ergänzend kann vorgesehen sein, dass das Abstrahlelement scheibenförmig, insbesondere in runder oder eckiger Form, ausgebildet ist.

Dies lässt sich besonders einfach herstellen und zudem auch gut handhaben.

Alternativ oder ergänzend kann vorgesehen sein, dass das Gehäuse eine Öffnung mit einem, vorzugsweise ringförmigen, Randbereich aufweist, und vorzugsweise das Abstrahlelement an dem Randbereich angeordnet ist, insbesondere wenn das Arretierungsmittel in der Arretierstellung ist.

Der Randbereich kann damit als derjenige Bereich definiert sein, an dem das Abstrahlelement festlegbar ist und/oder an dem das Abstrahlelement, wenn das Arretierungsmittel in der Arretierstellung ist, festgelegt ist.

Alternativ oder ergänzend kann vorgesehen sein, dass das Infrarotheizmodul einen Berührschutz des Abstrahlelements aufweist, wodurch ein unbeabsichtigtes Berühren des Abstrahlelements verhinderbar ist.

Der Berührschutz kann verhindern, dass ein Anwender mit dem Abstrahlelement unbeabsichtigterweise in Kontakt kommt. Da das Abstrahlelement während des Einsatzes des Infrarotheizmoduls warm oder sogar heiß werden kann, schützt der Berührschutz vor Verbrennungen und erhöht damit die Sicherheit.

Beispielsweise ist der Berührschutz großmaschig ausgestaltet und/oder weist eine Größe, insbesondere eine Öffnungsgröße, von 120 mm x 40 mm auf.

Der Berührschutz weist vorzugsweise eine Wärmeleitfähigkeit von weniger als 1 W/mK, vorzugsweise von weniger als 0,8 W/mK, vorzugsweise von weniger als 0,5 W/mK, vorzugsweise von weniger als 0,4 W/mK, vorzugsweise von weniger als 0,25 W/mK, vorzugsweise von weniger als 0,1 W/mK, auf. Dadurch kann besonders zuverlässig sichergestellt sein, dass sich der Berührschutz nicht oder nur wenig durch die Infrarotstrahlung aufwärmt und dadurch auch wenig Wärme an das berührende Körperteil abgibt.

Alternativ oder ergänzend kann vorgesehen sein, dass der Berührschutz einstückig mit zumindest einem Teil des Arretierungsmittels und/oder des Gehäuses ausgebildet ist.

Indem der Berührschutz entsprechend ausgebildet ist, ist der Berührschutz immer vorgesehen, wenn das Infrarotheizmodul im Betrieb ist. Damit ist ein besonders sicherer Betrieb des Infrarotheizmoduls möglich.

Gemäß einem dritten Aspekt ist eine Vorrichtung gemäß dem ersten Aspekt der Erfindung offenbart, wobei zumindest eines der Infrarotheizmodule der Vorrichtung ein Infrarotheizmodul gemäß dem zweiten Aspekt der Erfindung aufweist oder darstellt.

Der Erfindung liegt damit die überraschende Erkenntnis zugrunde, dass der Hautpartie die Wärmestrahlung besonders kontrolliert bereitgestellt werden kann, indem die Abstrahlcharakteristik der einzelnen Infrarotheizmodule berücksichtigt wird. Dadurch kann sichergestellt werden, dass eine lokale Überhitzung der Hautpartie in Folge eines zu hohen Wärmebeitrags mehrerer Infrarotheizmodule zuverlässig vermieden werden kann. Gleichermaßen können aber auch Bereiche mit zu geringer Wärmezufuhr vermieden werden, wodurch eine zuverlässigere Wärmeanwendung möglich ist.

Die Erfinder haben insoweit erkannt, dass die unterschiedlichen Abstrahlcharakteristiken besonders einfach aber dennoch wirkungsvoll dadurch berücksichtigt werden können, indem die Infrarotheizmodule mit unterschiedlicher Temperatur betrieben werden. Dadurch ist es überraschend einfach möglich, innerhalb des betrachteten Bereichs eine homogene Intensität der Wärmestrahlung, die sich aus überlagerten Beiträgen auch mehrerer Infrarotheizmodule ergeben kann, bereitzustellen.

Zur bevorzugten Definition der homogenen Intensität kann auf die Ausführungen im Zusammenhang mit dem ersten Aspekt der Erfindung verwiesen werden, die hier ganz entsprechend gelten.

Das homogene Wärmefeld (also eine homogene Intensität der Wärmestrahlung) ermöglicht dabei nicht nur eine, vor allem hinsichtlich der zulässigen Grenzwerte, besonders kontrollierte Wärmebehandlung. Zusätzlich erlaubt ein Bereich mit einem homogenen Wärmefeld auch eine Toleranz gegenüber einer relativen Bewegung von Vorrichtung und Hautpartie. Denn auch bei einer relativen Verschiebung ist eine gute Wärmebehandlung durchführbar.

Indem außerdem auch ein austauschbares Abstrahlelement in wenigstens einem der Infrarotheizmodule vorgesehen ist, kann die Abstrahlcharakteristik zumindest des einen Infrarotheizmoduls individuell angepasst werden.

Durch das Zusammenspiel des ersten und des zweiten Erfindungsaspekts wurde durch die Erfinder überraschend eine Möglichkeit gefunden, die von der Vorrichtung bereitgestellte Wärmestrahlung, welcher beispielsweise dann die Hautpartie aussetzbar ist, noch besser definieren zu können, und damit ein noch zuverlässiger eine homogene Intensität der Wärmestrahlung bereitgestellt werden kann.

Mit anderen Worten, durch die individuelle Temperatureinstellung der Infrarotheizmodule einerseits und den Einsatz des Abstrahlelements andererseits lassen sich nach Erkenntnissen der Erfinder Synergieeffekte aus beiden Maßnahmen erzielen, die zu einer Wärmestrahlung mit besonders vorteilhaften, da homogenen, Intensitätsverteilung, führen.

Gemäß einem vierten Aspekt ist eine Einheit zur Zuführung von Wärme an eine Hautpartie eines Lebewesens offenbart, insbesondere als zumindest ein Infrarotheizmodul der Infrarotheizmodule der Vorrichtung gemäß dem ersten Aspekt der Erfindung. Die Einheit umfasst ein Gehäuse und zumindest eine innerhalb des Gehäuses angeordnete Wärmequelle in Form eines Infrarotstrahlers zur Abgabe von Wärme an die Umgebung,

einen Temperatursensor, der dazu angepasst ist, die Temperatur von zumindest Teilen der beim Gebrauch der Einheit vor der Einheit befindlichen Hautpartie zu messen,

und/oder ein Steuermodul, das dazu eingerichtet ist, die während des Gebrauchs der Einheit von der Wärmequelle pro Zeiteinheit abgegebene Wärme nach Maßgabe bzw. basierend zumindest auf der gemessenen Temperatur zu steuern.

Der Erfindung liegt damit die überraschende Erkenntnis zugrunde, dass ein manuelles eingreifen oder gar Verändern des Abstandes zwischen Infrarotstrahler und Person entfallen kann, indem die Hauttemperatur der Person überwacht wird.

Denn auf Grundlage der Haupttemperatur kann die von der Wärmequelle abgegebene Wärme besonders gut kontrolliert und ihre Intensität ggf. angepasst werden. So kann eine zu starke Erwärmung der Haut zuverlässig vermieden werden. Vor allem ist die vorgeschlagene Einheit mittels eines festen Aufbaus möglich, da die Regulierung des Wärmeeintrags elektrisch und ohne Abstandsänderung erfolgt. Dadurch kann die Einheit sehr kompakt ausgebildet werden. Außerdem ist sie universell für verschiedene Personen einsetzbar.

Aufgrund der überraschend einfachen Realisierung lassen sich auch bestehende Infrarotstrahler leicht und besonders kostengünstig erfindungsgemäß anpassen. Daher ist die vorgeschlagene Einheit auch wirtschaftlich vorteilhaft.

Es kann also die Bestrahlungsstärke der Hautpartie automatisch gesteuert werden. Dadurch wird die Einheit zudem besonders sicher in der Anwendung. Der Anwender kann daher in den Genuss der Wirkungen der (lokalen) Wärmeanwendung in einer bisher nicht verfügbaren Qualität kommen, bei gleichzeitig hohem Sicherheitsstandard.

Dabei ist die Steuerung besonders einfach und dennoch zuverlässig möglich. Aufgrund der einfachen Ausgestaltbarkeit, ist die Steuerung weniger fehleranfällig und daher die Einheit insgesamt zuverlässiger und sicherer im Betrieb sowie kostengünstiger im Unterhalt. Die Steuerung lässt sich aber auf Wunsch auch besonders einfach erweitern und bietet daher ein hohes Maß an Flexibilität, um künftige Entwicklungen besonders einfach berücksichtigen zu können.

Die gemessene Hauttemperatur ist dabei vorzugsweise eine Hauttemperatur auf der Oberfläche und/oder im Oberflächenbereich der Hautpartie.

Das Steuern der von der Wärmequelle abgegebenen Wärme kann beispielsweise das Anpassen, insbesondere Verringern oder Erhöhen, der von der Wärmequelle abgegebenen Wärme pro Zeiteinheit aufweisen.

Der Temperatursensor kann über ein Datenkabel mit dem Steuermodul verbunden sein. Der Temperatursensor kann auf einer Platine angeordnet sein. Die Platine kann Abmessungen von beispielsweise 13x11x8 mm$^3$ aufweisen.

Die vorgeschlagene Einheit ist dabei vielseitig einsetzbar. Die Einheit kann universell für stationäre und mobile Wärmeanwendungen eingesetzt werden. Die Einheit kann zum Einbau in Kabinen, Saunaanlagen, hyperbaren Anlagen und hypobaren Anlagen eingesetzt werden. Die Einheit kann auch als mobiler Infrarotstrahler eingesetzt werden.

In einer Ausführungsform ist die Einheit in einer ergonomischen Form ausgeführt, wobei sie sich vorzugsweise flexibel an verschiedene Rückenformen anpasst.

Der Infrarotstrahler kann dabei so ausgelegt sein, dass er auf der Hautpartie zumindest bereichsweise eine homogene Bestrahlungsstärke bereitstellt. Vorzugsweise erfolgt dies in einem Bereich, der sich vertikal und/oder horizontal und/oder in einem Abstand von zwischen 5 cm und 20 cm von der Einheit entfernt erstreckt.

Vorzugsweise beziehen sich dabei die Angaben "vertikal" und "horizontal" auf Richtungen bezüglich der Einheit während ihres Gebrauchs. Der Abstand wird vorzugsweise senkrecht zu einer äußeren Oberfläche der Einheit gemessen.

In einer Ausführungsform wird die Wärmequelle mit einer Schutzkleinspannung von maximal 42 V DC und/oder einer elektrischen Leistung von 350 W oder weniger betrieben. Dadurch ist die elektrische Sicherheit gewährleistet. Außerdem werden baubiologische EMF-Grenzwerte eingehalten. Die Spannung kann über ein externes Netzteil oder über einen Akku bereitgestellt werden.

Ein Akku ist besonders bevorzugt, da es dadurch möglich ist, die Einheit auch mobil und unabhängig von der Verfügbarkeit eines Stromnetzes zu betreiben. Der Akku kann beispielsweise fest verbaut sein oder zerstörungsfrei austauschbar sein.

Die Steuerung der Wärmequelle kann dabei vorzugsweise derart erfolgen, dass die Leistung des Strahlers zu einer definierten oder definierbaren Hauttemperatur (vegetative Stimulation) führt, aber Grenzwerte (thermische Hautschädigung) nicht überschritten werden.

In einer Ausführungsform kann in jeder Richtung die maximale Abmessung der Einheit 40 cm oder weniger, vorzugsweise 35 cm oder weniger, vorzugsweise 30 cm oder weniger, vorzugsweise 25 cm oder weniger, vorzugsweise 20 cm oder weniger, vorzugsweise 15 cm oder weniger, vorzugsweise 10 cm oder weniger, betragen. Optional beträgt sie 1 cm oder mehr, vorzugsweise 5 cm oder mehr, vorzugsweise 10 cm oder mehr, vorzugsweise 20 cm oder mehr, vorzugsweise 30 cm oder mehr.

Das Steuermodul kann in Software, in Hardware oder aus einer Kombination von beidem realisiert sein. Beispielsweise kann das Steuermodul einen Prozessor oder Mikrokontroller aufweisen, der entsprechend konfiguriert ist.

Das Lebewesen kann beispielsweise ein Mensch und/oder ein Tier sein.

Alternativ oder ergänzend kann vorgesehen sein, dass die Einheit zumindest ein, insbesondere von der Wärmequelle aufwärmbares, Abstrahlelement aufweist,

und wobei das Abstrahlelement und die Wärmequelle derart zueinander angeordnet sind, dass die von der Wärmequelle abgegebene Wärme zumindest teilweise über das Abstrahlelement an die Umgebung, insbesondere außerhalb der Einheit, abgegeben wird, vorzugsweise, indem das Abstrahlelement von der Wärmequelle aufgewärmt wird und dann seinerseits Wärme abstrahlt.

Die Erfinder haben erkannt, dass das Vorsehen eines Abstrahlelements eine homogene Wärmeabgabe selbst über einen ausgedehnten Flächenbereich in besonders einfacher Weise ermöglicht und sich daher in Verbindung mit einer entsprechenden Steuerung der Wärmequelle, die von der Einheit an die Hautpartie abgegebene Wärme besonders zuverlässig und effizient einstellen lässt.

Die Erfinder haben vor allem erkannt, dass durch das Abstrahlelement eine deutlich größere Unabhängigkeit von der Wahl der Wärmequelle erreicht werden kann. Denn die homogene Wärmeabstrahlung kann zu einem größeren Teil auch durch das Abstrahlelement erreicht werden und muss nicht alleine durch die Wärmequellen selbst erreicht werden.

Dabei wird das Abstrahlelement sozusagen von der Wärmequelle aufgeheizt und strahlt dann wiederum in den Raum ab. Daher kann die Anzahl, die Art und die Positionierung der Wärmequelle(n) nach anderen Gesichtspunkten als primär denen zum Erreichen einer homogenen Abstrahlung gewählt werden. Dies erlaubt auch die Wahl von günstigeren Wärmequellen. Beispielsweise können so räumlich enger begrenzte, also etwa im Wesentlichen linienförmige oder sogar punktförmige, Wärmequellen eigesetzt werden. Denn die homogene und vor allem flächenhafte Abstrahlung wird durch das Abstrahlelement gefördert. Dadurch kann die Einheit kompakter gestaltet werden, wodurch Materialkosten und Gewicht eingespart und die Handhabung nicht nur angenehmer, sondern vor allem auch sicherer gestaltet werden kann.

Werden mehrere Wärmequellen eingesetzt, dann tragen sozusagen alle Wärmequellen durch Überlagerung ihres Wärmebeitrages in diffuser Weise zur Abstrahlung der Wärme entlang der Fläche bei. Daher lässt sich eine Anpassung der von der Einheit an die Hautpartie zugeführten Wärmemenge, sowohl nach oben als auch nach unten, und damit die Temperatur auf der Hautpartie sehr effizient durch Anpassen auch nur einzelner Wärmequellen einstellen. Beispielsweise kann die abgegebene Wärmemenge von nur einer einzigen Wärmequelle angepasst werden. Da die Wärme dieser Wärmequelle zumindest näherungsweise betrachtet über die gesamte Fläche des Abstrahlelements, wie auch die Wärme der anderen Wärmequellen abgegeben wird, kann dadurch die abgestrahlte Wärmemenge unter weitestgehender Beibehaltung einer homogenen Abstrahlung reduziert werden.

Alternativ oder ergänzend kann vorgesehen sein, dass das Abstrahlelement zumindest teilweise vor, innerhalb und/oder hinter einer Öffnung des Gehäuses angeordnet ist und/oder das Abstrahlelement die Öffnung zumindest teilweise verschließt.

Eine entsprechende Anordnung des Abstrahlelements ermöglicht eine günstige Herstellung von sowohl Gehäuse als auch Abstrahlelement, beispielsweise mittels Spritzguss. Die beiden Teile können dann vorzugsweise mit bekannten Mitteln, wie Ultraschallschweißen oder Verkleben, miteinander verbunden sein.

Das Verschließen der Öffnung durch das Abstrahlelement schützt das Innere des Gehäuses, wie etwa die Wärmequelle und andere Elektronik, vor eindringender Feuchtigkeit, wie insbesondere Schweiß.

Alternativ oder ergänzend kann vorgesehen sein, dass das Abstrahlelement zumindest bereichsweise einstückig mit dem Gehäuse ausgebildet ist, insbesondere das Abstrahlelement einen Bereich eines ersten Gehäuseabschnitts aufweist oder darstellt.

Die Einheit ist besonders stabil im Einsatz und günstig in der Herstellung, wenn das Abstrahlelement als Teil des Gehäuses ausgebildet wird. Dadurch kann auch die Dichtigkeit der Einheit erhöht und das Eindringen von Feuchtigkeit, wie insbesondere Schweiß, besonders gut und zuverlässig verhindert oder zumindest reduziert werden.

Alternativ oder ergänzend kann vorgesehen sein, dass das Abstrahlelement zumindest bereichsweise membranförmig und/oder flächig ausgebildet ist.

Eine flächige Ausbildung erzielt einen besonders hohen Wirkungsgrad, da nur eine geringe Materialdicke von der Wärmequelle erwärmt werden muss. Gleichzeitig strahlt das Abstrahlelement die Wärme mit einer vergleichsweise hohen Homogenität wieder ab, selbst wenn es seinerseits nur bereichsweise von der Wärmequelle direkt bestrahlt, mithin erwärmt wird.

Eine Membran lässt sich besonders einfach herstellen und innerhalb des Gehäuses anordnen.

Auch ein Abstrahlelement mit einer bestimmten Dicke kann im Sinne der vorliegenden Anmeldung ein flächiges Abstrahlelement sein. Ein flächiges Abstrahlelement liegt insbesondere dann vor, wenn die räumliche Erstreckung des Abstrahlelements in zwei senkrecht zueinander verlaufende Erstreckungsrichtungen, der Erstreckung des Abstrahlelements in eine zu diesen senkrecht stehende dritte Erstreckungsrichtung deutlich übersteigt, insbesondere die Ausbreitung in die erste und zweite Richtung jeweils um einen Faktor 3 oder mehr, vorzugsweise 5 oder mehr, vorzugsweise 10 oder mehr, vorzugsweise 20 oder mehr, größer ist als die in die dritte Richtung.

Beispielsweise kann ein Abstrahlelement scheibenartig ausgeführt sein.

Beispielsweise kann das Abstrahlelement einen zumindest bereichsweise gekrümmten Verlauf aufweisen. Ein gekrümmter Verlauf ist besonders bevorzugt, um eine an den Verlauf der Hautpartie angepasste und damit ergonomische Form zu erreichen. Damit lässt sich eine besonders schonende Behandlung der Hautpartie ermöglichen. Beispielsweise kann das Abstrahlelement entlang einer Längsachse der Einheit, insbesondere des Abstrahlelements, an die Form einer Wirbelsäule eines zu bestrahlenden Lebewesens angepasst sein.

Beispielsweise kann das Abstrahlelement auch einen zumindest bereichsweise ebenen Verlauf aufweisen.

In einer Ausführungsform weist das Abstrahlelement eine Erstreckung in eine erste Richtung von (a) 400 mm oder weniger, vorzugsweise von 300 mm oder weniger, vorzugsweise von 250 mm oder weniger, vorzugsweise von 200 mm oder weniger, vorzugsweise von 150 mm oder weniger, vorzugsweise von 100 mm oder weniger, vorzugsweise von 50 mm oder weniger, (b) 1 mm oder mehr, vorzugsweise von 10 mm oder mehr, vorzugsweise von 50 mm oder mehr, vorzugsweise von 100 mm oder mehr, vorzugsweise von 200 mm oder mehr, vorzugsweise von 250 mm oder mehr, und/oder (c) zwischen 1 mm und 400 mm, vorzugsweise zwischen 10 mm und 350 mm, vorzugsweise zwischen 50 mm und 300 mm, vorzugsweise zwischen 100 mm und 300 mm, vorzugsweise zwischen 150 mm und 300 mm, vorzugsweise zwischen 200 mm und 300 mm, auf.

In einer Ausführungsform weist das Abstrahlelement eine Erstreckung in eine zweite Richtung von (a) 900 mm oder weniger, vorzugsweise von 800 mm oder weniger, vorzugsweise von 750 mm oder weniger, vorzugsweise von 700 mm oder weniger, vorzugsweise von 650 mm oder weniger, vorzugsweise von 600 mm oder weniger, vorzugsweise von 500 mm oder weniger, (b) 1 mm oder mehr, vorzugsweise von 100 mm oder mehr, vorzugsweise von 300 mm oder mehr, vorzugsweise von 400 mm oder mehr, vorzugsweise von 500 mm oder mehr, vorzugsweise von 600 mm oder mehr, und/oder (c) zwischen 1 mm und 900 mm, vorzugsweise zwischen 200 mm und 800 mm, vorzugsweise zwischen 500 mm und 800 mm, vorzugsweise zwischen 550 mm und 750 mm, vorzugsweise zwischen 600 mm und 700 mm, auf.

Beispielsweise beträgt die Erstreckung in die erste Richtung 180 mm und/oder in die zweite Richtung 650mm.

Alternativ oder ergänzend kann vorgesehen sein, dass das Abstrahlelement als Material X, ..., Y, und/oder Z aufweist oder daraus besteht.

Die genannten Materialien sind widerstandsfähig gegen Wärme und damit formstabil. Daher eignen sich die Materialien besonders zum Einsatz bei der vorliegenden Einheit, die zur Wärmebehandlung von Hautpartien eingesetzt wird.

Alternativ oder ergänzend kann vorgesehen sein, dass das Abstrahlelement zumindest bereichsweise eine Wärmeleitfähigkeit von X W/(mK) oder mehr, vorzugsweise von Y W/(mK) oder mehr, aufweist.

Alternativ oder ergänzend kann vorgesehen sein, dass das Steuermodul die Wärmequelle derart steuert, dass eine definierte oder definierbare maximal zulässige Hauttemperatur, zumindest lokal, nicht überschritten wird und/oder das Steuermodul die Wärmequelle derart steuert, dass die von der Wärmequelle abgegebene Wärme pro Zeiteinheit so lange, insbesondere mit einer definierten oder definierbaren Änderungsrate, zunimmt, bis, zumindest lokal, die maximal zulässige Hauttemperatur erreicht wird.

Die gemessene Temperatur kann daher auf vorteilhafte Weise für die Steuerung eingesetzt werden.

Indem die Maximaltemperatur auf der Hauptpartie kontrolliert wird, ist ein besonders sicherer Betrieb der Einheit möglich.

Indem die Temperatur auf der Hautpartie kontrolliert gesteigert wird, ist es besonders zuverlässig möglich, die maximal zulässige Hauttemperatur nicht zu überschreiten. Zum Beispiel kann beim Einschalten der Einheit die Wärmequelle entsprechend gesteuert werden. Aber auch anschließend ist es alternativ oder ergänzend vorteilhaft, die von der Wärmequelle abgegebene Wärme kontrolliert zu steigern, um innerhalb des zulässigen Bereichs zu bleiben.

In einer Ausführungsform wird die Abstrahlung der Wärmequelle kontinuierlich angepasst, um eine, zumindest innerhalb eines definierten oder definierbaren Temperatur-Schwankungsbereichs, gleichbleibende Hauttemperatur zu erreichen.

Alternativ oder ergänzend kann vorgesehen sein, dass die Einheit ferner eine zumindest teilweise innerhalb des Gehäuses angeordnete Wärmeisolierung aufweist, wobei die Wärmeisolierung vorzugsweise zwischen einem, insbesondere dem ersten Gehäuseabschnitt zumindest bereichsweise gegenüberliegenden, zweiten Gehäuseabschnitt und der Wärmequelle angeordnet ist, und insbesondere die Innenseite des zweiten Gehäuseabschnitts mit der Wärmeisolierung in unmittelbarem oder mittelbarem Kontakt steht.

Die Wärmeisolierung kann beispielsweise auch angeordnet sein der Einheit und einer benachbarten Einheit. Hierdurch können benachbarte und miteinander verknüpfte Einheiten thermisch voneinander entkoppelt ausgebildet sein. Die Wärmeisolierung kann beispielsweise ein Aerogel umfassen, insbesondere ein Hochtemperaturaerogel. Die Wärmeisolierung kann eine Wärmeleitfähigkeit von 0,02 W/(m·K) oder weniger haben.

Durch das Vorsehen der Wärmeisolierung kann das Erwärmen von Gehäuseteilen der Einheit vermieden oder zumindest reduziert werden. Dadurch kann die Handhabung der Einheit deutlich sicherer und angenehmer gemacht werden.

So ist nämlich sichergestellt, dass der Wärmeübergang zwischen der Wärmequelle und dem Gehäuse gering ist, so dass das Gehäuse so wenig wie möglich aufgewärmt wird.

Die Wärmeisolierung kann beispielsweise durch ein Aerogel erfolgen. Hier sind Dicken von 5 mm oder mehr, vorzugsweise von 10 mm oder mehr, von 30 mm oder weniger, vorzugsweise von 25 mm oder weniger, vorzugsweise von 20 mm oder weniger, und/oder von zwischen 5 mm und 30 mm, vorzugsweise von zwischen 10 mm und 25 mm, vorzugsweise von 10 mm und 15 mm, bevorzugt.

Außerdem kann die Wärmeisolierung dazu beitragen, dass die Wärme zu einem großen Teil in Richtung des Abstrahlelements oder jedenfalls zur Hautpartie geleitet wird und nicht für den Zweck der Einheit, nämlich Wärme in Richtung der Hautpartie abzustrahlen, verloren ist.

Dazu weist die Wärmeisolierung vorzugsweise ein Reflexionselement auf oder stellt dieses dar, das seinerseits beispielsweise dazu angepasst ist, die von der Wärmequelle einfallende Wärme zumindest teilweise in Richtung des Abstrahlelements zu reflektieren. Beispielsweise kann das Reflexionselement eine beschichtete Folie sein oder aufweisen, die Infrarotstrahlung reflektiert.

Alternativ oder ergänzend kann vorgesehen sein, dass die Wärmeisolierung ein Aerogel umfassen, insbesondere ein Hochtemperaturaerogel aufweist oder daraus besteht.

Die genannten Materialien sind besonders effizient im Einsatz.

Alternativ oder ergänzend kann vorgesehen sein, dass das Gehäuse Abschnitte mit unterschiedlicher Wärmeleitfähigkeit aufweist und der erste Gehäuseabschnitt eine, vorzugsweise mittlere, Wärmeleitfähigkeit aufweist, die höher ist, insbesondere 10 % oder mehr, vorzugsweise 30 % oder mehr, vorzugsweise 50 % oder mehr, vorzugsweise 100 % oder mehr, vorzugsweise 200 % oder mehr, als die Wärmeleitfähigkeit von wenigstens des zweiten Gehäuseabschnitts, vorzugsweise als die Wärmeleitfähigkeit aller übrigen Abschnitte.

Indem Gehäuseteile unterschiedliche Wärmeleitfähigkeiten aufweisen, geben diese unterschiedlichen Bereiche des Gehäuses die von der Wärmequelle empfangene Wärme sozusagen unterschiedlich stark nach außen ab. Dadurch lässt sich die Richtung, in die die Einheit Wärme überwiegend abstrahlt besonders einfach und zuverlässig einstellen. Beispielsweise kann ein solches Gehäuse besonders einfach und kostengünstig mit Mehrkomponenten-Spritzgießen hergestellt werden.

Der Einsatz eines solchen Gehäuses ist gerade im Zusammenspiel mit der oben genannten Wärmeisolierung besonders effizient.

Alternativ oder ergänzend kann vorgesehen sein, dass der Temperatursensor ein Infrarottemperatursensor ist, innerhalb des Gehäuses angeordnet ist, und/oder zumindest teilweise, insbesondere aus Sicht der Hautpartie, hinter dem Abstrahlelement angeordnet ist, und vorzugsweise das Abstrahlelement zumindest bereichsweise durchdringt.

Indem die Temperaturmessung berührungslos erfolgt, lassen sich alle elektronischen Komponenten sicher innerhalb des Gehäuses und vor Berührung geschützt unterbringen. Dies erhöht die Sicherheit. Auch die Reinigung der Einheit wird erleichtert und dadurch ihre Lebensdauer erhöht.

Gleichzeitig ermöglichen die Infrarottemperatursensoren die Messung parallel zum laufenden Betrieb und sogar in nächster Nähe zum Abstrahlelement durchzuführen. Dies ermöglicht eine kompakte Implementierung der Sensoren innerhalb des Gehäuses.

Alternativ oder ergänzend kann vorgesehen sein, dass der Temperatursensor ein Sichtbereich von 10° oder mehr, vorzugsweise von 30° oder mehr, vorzugsweise von 40° oder mehr, vorzugsweise von 50° oder mehr, vorzugsweise von 60° oder mehr, vorzugsweise von 60° oder mehr, vorzugsweise von 70° oder mehr, vorzugsweise von 80° oder mehr, vorzugsweise von

90° oder mehr, vorzugsweise von 100° oder mehr, vorzugsweise von 120° oder mehr, vorzugsweise von 150° oder mehr, aufweist und/oder der Temperatursensor beim Gebrauch der Einheit einen Abstand von der Hautpartie von 30 cm oder weniger, vorzugsweise von 20 cm oder weniger, vorzugsweise von 10 cm oder weniger, vorzugsweise von 5 cm oder weniger, vorzugsweise von 3 cm oder weniger, vorzugsweise von 1 cm oder weniger, aufweist.

Optional kann der Temperatursensor einen Sichtbereich von weniger als 180°, vorzugsweise von weniger als 150°, vorzugsweise von weniger als 120°, vorzugsweise von weniger als 100°, vorzugsweise von weniger als 90°, vorzugsweise von weniger als 80°, vorzugsweise von weniger als 60°, vorzugsweise von weniger als 50°, vorzugsweise von weniger als 30°, vorzugsweise von weniger als 10°, aufweisen.

Ein entsprechender Sichtbereich ermöglicht die Erfassung eines ausreichend großen aber gleichzeitig nicht zu großen Bereichs der Hautpartie. Dadurch wird eine zuverlässige Messung der Temperatur der Hautpartie ermöglicht.

Die angegebenen Abstände sind dabei vorzugsweise die senkrechten Abstände zwischen Sensor und Hautpartie.

Aus der Kombination eines Abstands und eines Sichtbereich kann sich der von dem Sensor erfasste Flächenbereich einer (ebenen) Hautpartie ermittelbar sein.

Alternativ oder ergänzend kann vorgesehen sein, dass die Einheit zwei, drei, vier oder mehr als vier Temperatursensoren aufweist,

und die Temperatursensoren derart angeordnet und/oder ausgewählt sind, dass jeweils zwei der Temperatursensoren die Temperatur von zumindest teilweise verschiedenen Bereichen der beim Gebrauch der Einheit vor dem Abstrahlelement befindlichen Hautpartie messen,

und wobei vorzugsweise das Steuermodul dazu eingerichtet ist, die Wärmequelle nach Maßgabe der höchsten von den Temperatursensoren gemessenen Temperatur zu steuern.

Durch mehrere Temperatursensoren mit unterschiedlichem Erfassungsbereich auf der Hautpartie lassen sich Temperaturen auch auf größeren Hauptbereichen mit hoher Genauigkeit überwachen.

Indem die höchste, mithin maximale, Temperatur zur Steuerung der Wärmequelle verwendet wird, ist ein sichererer und zuverlässiger Betrieb der Einheit möglich. Beispielsweise können die einzelnen Temperatursensoren unterschiedliche Temperaturwerte für die einzelnen Bereiche der Hautpartie messen. Wenn von diesen unterschiedlichen Temperaturwerten der mit der höchsten Temperatur für die Steuerung der Wärmequelle verwendet wird, kann sichergestellt werden, dass sich die Temperatur auf den überwachten Bereichen der Hautpartie innerhalb zulässiger Grenzen bewegt. Oder mit anderen Worten ausgedrückt, indem die Steuerung nach Maßgabe der maximalen Temperatur erfolgt, kann vermieden werden, dass lokal eine zu hohe Temperatur auftritt.

Alternativ oder ergänzend kann vorgesehen sein, dass das Steuermodul dazu eingerichtet ist, die Wärmequelle derart zu steuern, dass bei Benutzung der Einheit die am Ort der im Abstand von 10 cm von dem Temperatursensor befindliche Hautpartie einer Wärme-Intensität von 150 mW/cm² oder weniger ausgesetzt wird, wobei vorzugsweise die Wärme-Intensität anhand der gemessenen Temperatur ermittelt wird oder werden kann.

Dadurch ist ein sicherer und zuverlässiger Betrieb der Einheit möglich.

Beispielsweise kann bei Erreichen dieses Maximalwertes das Steuermodul dazu eingerichtet sein, eine weitere Steigerung der von der Wärmequelle abgegebenen Wärme selbst dann zu unterlassen, wenn die Temperatur der Hautpartie noch nicht die definierte oder definierbare maximal zulässige Hauttemperatur erreicht hat. Damit kann sozusagen ein Schutzschalter vorgesehen werden. Die von der Wärmequelle abgegebene Wärme kann beispielsweise mit Hilfe einer mittels eines weiteren innerhalb des Gehäuses vorgesehenen Temperatursensors gemessenen Temperatur und einer Kalibriertabelle ermittelt werden.

Optional kann das Steuermodul dazu eingerichtet sein, die Wärmequelle derart zu steuern, dass die Hautpartie im Abstand von 10 cm einer Wärme-Intensität von 1 mW/cm² oder mehr ausgesetzt wird.

Alternativ oder ergänzend kann vorgesehen sein, dass das Steuermodul dazu eingerichtet ist, mittels einer externen Bedienvorrichtung, wie einem Smartphone, zu kommunizieren, insbesondere Befehle von dieser zu empfangen und Daten der Einheit an die Bedienvorrichtung zu senden.

Dadurch lassen sich Informationen über die Einheit besonders einfach auswerten.

Alternativ oder ergänzend kann vorgesehen sein, dass der Infrarotstrahler zumindest einen Heizleiter, vorzugsweise aus Keramik, einer Dickschichtleitung oder Carbon, aufweist.

Ein Heizleiter ist günstig in der Herstellung und lässt sich besonders einfach und zuverlässig in der Einheit anbringen.

Beispielsweise kann der Heizleiter innerhalb der Einheit entlang einer geraden oder gekrümmten Linie verlaufen. Der Heizleiter kann auch mäanderförmig verlaufen. Dabei ist es jeweils bevorzugt, dass der Heizleiter zumindest abschnittsweise in einer Ebene verläuft, die parallel zu einer Oberfläche des Abstrahlelements ist. Der konstante Abstand kann eine gleichmäßige Erwärmung des Abstrahlelements verbessern und damit gleichermaßen das Bereitstellen eines homogenen Wärmefeldes außerhalb der Einheit.

Alternativ oder ergänzend kann vorgesehen sein, dass das Gehäuse als Material X, ..., Y, und/oder Z aufweist oder daraus besteht.

Die genannten Materialien sind zum einen widerstandsfähig gegen Wärme und damit formstabil. Andererseits sind sie auch bei Kontakt mit selbst empfindlicher Haut unkritisch. Daher eignen sich die Materialien besonders zum Einsatz bei der vorliegenden Einheit, die zur Wärmebehandlung von Hautpartien eingesetzt wird.

Alternativ oder ergänzend kann vorgesehen sein, dass das Gehäuse, insbesondere der beim Gebrauch der Einheit der Hautpartie abgewandte Gehäuseabschnitt und/oder der zweite Gehäuseabschnitt, zumindest bereichsweise eine Wärmeleitfähigkeit von X W/(mK) oder weniger, vorzugsweise von Y W/(mK) oder weniger, aufweist.

Dadurch lässt sich das Gehäuse sicher handhaben. Insbesondere kann der rückseitige Gehäuseabschnitt entsprechend gewählt sein.

Alternativ oder ergänzend kann vorgesehen sein, dass die Einheit ferner zumindest eine innerhalb des Gehäuses angeordnete Lichtquelle, insbesondere in Form einer LED oder einer Laserdiode, aufweist.

Die Lichtquelle kann insbesondere in einem Verbindungsabschnitt zwischen zwei benachbarten Einheiten einer Anordnung vorgesehen sein. Der Verbindungsabschnitt kann zur thermischen Entkopplung zwischen benachbarten Einheiten vorgesehen und ausgebildet sein.

Dadurch lässt sich der Innenraum besser inspizieren.

Alternativ oder ergänzend kann vorgesehen sein, dass die Einheit ferner zumindest einen Berührungsschutz, insbesondere des Abstrahlelements, aufweist, der vorzugsweise (a) zumindest bereichsweise beabstandet zu einer beim Gebrauch der Einheit der Hautpartie zugewandten Seite des Abstrahlelements vorgesehen ist und/oder (b) gleichzeitig eine Auflagefläche für die Hauptpartie bereitstellt oder ausbildet,

und wobei vorzugsweise der Berührungsschutz mit dem Gehäuse verbunden und/oder zumindest bereichsweise einstückig mit diesem ausgebildet ist und/oder der Berührungsschutz zumindest bereichsweise eine Wärmeleitfähigkeit von X W/(mK) oder weniger, vorzugsweise von Y W/(mK) oder weniger, aufweist.

Der Berührungsschutz verhindert zuverlässig, dass die Hautpartie mit dem Abstrahlelement oder anderen Elementen der Einheit in Berührung kommt. Der Berührungsschutz kann gleichzeitig auch eine Auflagefläche für die Hauptpartie bereitstellen oder ausbilden, so dass das Abstrahlelement in definiertem Abstand zu der Hauptpartie gehalten werden kann.

Die Auflagefläche kann durchgehend sein, die Auflagefläche kann aber auch gelocht sein oder andere Öffnungen aufweisen. Die Auflagefläche kann auch durch eine Gitterstruktur bereitgestellt sein.

Der Berührungsschutz kann zumindest bereichsweise durch ein Polster realisiert sein oder dieses aufweisen. Beispielsweise kann die Auflagefläche durch das Polster ausgebildet sein. Das Polster kann die Hautpartie zuverlässig und schonend abstützen. Dies ermöglicht eine sichere Benutzung der Einheit.

Beispielsweise kann der Berührungsschutz einen zumindest bereichsweise gekrümmten Verlauf aufweisen. Ein gekrümmter Verlauf ist besonders bevorzugt, um eine an den Verlauf der Hautpartie angepasste und damit ergonomische Form zu erreichen. Damit lässt sich eine besonders schonende Behandlung der Hautpartie ermöglichen. Beispielsweise kann der Berührungsschutz entlang einer Längsachse der Einheit, insbesondere des Berührungsschutzes, an die Form einer Wirbelsäule eines zu bestrahlenden Lebewesens angepasst sein.

Der Berührungsschutz kann als Material Kunststoff aufweisen. Die genannten Materialien sind zum einen widerstandsfähig gegen Wärme und damit formstabil. Andererseits sind sie auch bei Kontakt mit selbst empfindlicher Haut unkritisch. Daher eignen sich die Materialien besonders zum Einsatz bei der vorliegenden Einheit, die zur Wärmebehandlung von Hautpartien eingesetzt wird.

Alternativ oder ergänzend kann vorgesehen sein, dass die Einheit ferner zumindest eine Auffang- und/oder Abführvorrichtung zum Auffangen und Abführen von von der Hautpartie tropfenden Schweißes aufweist.

Dadurch lässt sich die Einheit vor Flüssigkeit schützen. Außerdem lässt sich die so aufgefangene Flüssigkeit einer späteren Analyse unterziehen.

Alternativ oder ergänzend kann vorgesehen sein, dass die Einheit ferner zumindest einen Abstandshalter aufweist, der während der Benutzung die Hautpartie beabstandet zu Teilen der übrigen Einheit, insbesondere dem Berührungsschutz und/oder dem Abstrahlelement, hält, aufweist und/oder der gleichzeitig eine Auflagefläche für die Hautpartie bereitstellt oder ausbildet,

und wobei vorzugsweise der Abstandshalter mit dem Gehäuse verbunden und/oder zumindest bereichsweise einstückig mit diesem ausgebildet ist.

Der Abstandshalter verhindert zuverlässig, dass die Hautpartie mit dem Abstrahlelement in Berührung kommt. Der Abstandshalter kann gleichzeitig auch eine Auflagefläche für die Hauptpartie bereitstellen oder ausbilden, so dass das Abstrahlelement in definiertem Abstand zu der Hauptpartie gehalten werden kann.

Die Auflagefläche kann durchgehend sein, die Auflagefläche kann aber auch gelocht sein oder andere Öffnungen aufweisen. Die Auflagefläche kann auch durch eine Gitterstruktur bereitgestellt sein.

Der Abstandshalter kann zumindest bereichsweise durch ein Polster realisiert sein oder dieses aufweisen. Beispielsweise kann die Auflagefläche durch das Polster ausgebildet sein. Das Polster kann die Hautpartie zuverlässig und schonend abstützen. Dies ermöglicht eine sichere Benutzung der Einheit.

Beispielsweise kann der Abstandshalter einen zumindest bereichsweise gekrümmten Verlauf aufweisen. Ein gekrümmter Verlauf ist besonders bevorzugt, um eine an den Verlauf der Hautpartie angepasste und damit ergonomische Form zu erreichen. Damit lässt sich eine besonders schonende Behandlung der Hautpartie ermöglichen. Beispielsweise kann der Abstandshalter entlang einer Längsachse der Einheit, insbesondere des Abstandhalters, an die Form einer Wirbelsäule eines zu bestrahlenden Lebewesens angepasst sein.

Alternativ oder ergänzend kann vorgesehen sein, dass die Einheit eine Vielzahl von Wärmequellen, vorzugsweise zwei, drei, vier oder mehr als vier Wärmequellen, jeweils in Form eines Infrarotstrahlers aufweist.

Wenn mehrere Wärmequellen vorgesehen sind, sind diese vorzugsweise alle geeignet zu dem Abstrahlelement angeordnet, so dass das Abstrahlelement von diesen erwärmbar ist.

Beispielsweise kann jede Wärmequelle gleichartig ausgebildet sein. Damit ist die Einheit besonders günstig realisierbar. Denn für jede Wärmequelle kann das gleiche Bauteil eingesetzt werden.

Alternativ oder ergänzend kann vorgesehen sein, dass zumindest zwei, vorzugsweise drei, mehr als drei und/oder alle, der Vielzahl von Wärmequellen, insbesondere entlang einer ersten Richtung, beabstandet zueinander angeordnet sind und/oder ihre Haupterstreckungsrichtung jeweils parallel zueinander, und insbesondere parallel zu einer zur ersten Richtung senkrechten zweiten Richtung, verläuft.

Die Wärmequellen können zumindest teilweise einen gleichen Abstand zueinander aufweisen, beispielsweise entlang der ersten Richtung. Damit kann die homogene Wärmeabstrahlung der Einheit verbessert werden.

Die erste Richtung kann eine beim Gebrauch der Einheit laterale Richtung darstellen, insbesondere verlaufend senkrecht zur Haupterstreckungsrichtung der Wirbelsäule des Lebewesens.

Die zweite Richtung kann eine beim Gebrauch der Einheit vertikale Richtung darstellen, insbesondere verlaufend parallel zur Haupterstreckungsrichtung der Wirbelsäule des Lebewesens.

Alternativ oder ergänzend kann vorgesehen sein, dass das Steuermodul dazu eingerichtet ist, die Vielzahl von Wärmequellen derart zu steuern, dass sie, insbesondere in Abhängigkeit voneinander und/oder von der gemessenen Temperatur, zumindest teilweise unterschiedliche Wärmemengen pro Zeiteinheit bereitstellen.

Dadurch ist es besonders einfach möglich, mehrere Zonen unterschiedlicher Temperatur innerhalb der Einheit zu erreichen. Dadurch können die Abstrahlcharakteristik sowie die Einbausituation der einzelnen Wärmequellen innerhalb des Gehäuses berücksichtigt werden.

Dies kann letztlich wiederum zu einer besonders homogenen Wärmeverteilung auf der Hautpartie führen.

Beispielsweise kann die Steuerung dazu führen, dass die Wärmequellen, die näher am Gehäuserand angeordnet sind, mit einer höheren Temperatur abstrahlen, als diejenigen Wärmequellen, die einen größeren Abstand davon aufweisen, insbesondere zentral innerhalb des Gehäuses angeordnet sind. Da womöglich die Wärme, die von den an den Rändern angeordneten Wärmequellen abgegeben wird nicht in gleich hohem Maße der Hautpartie zugeführt wird, kann durch eine größere Wärmeabgabe der näher am Gehäuserand angeordneten Wärmequellen eine insgesamt homogene Abstrahlung hergestellt werden.

Wenn drei Wärmequellen eingesetzt werden, kann beispielsweise eine zentral angeordnete Wärmequelle mit 180°C und die beiden anderen, näher am Gehäuserand angeordneten, Wärmequellen mit 230°C abstrahlen.

In einer Ausführungsform ist das Steuermodul dazu eingerichtet, wenigstens zwei Wärmequellen abhängig voneinander derart zu steuern, dass sie unterschiedliche Mengen an Wärme pro Zeiteinheit bereitstellen. Vorzugsweise ist dabei das Verhältnis von der größeren Wärmemenge pro Zeiteinheit der ersten Wärmequelle und der kleineren Wärmemenge pro Zeiteinheit der zweiten Wärmequelle (a) 1,1 oder mehr, vorzugsweise 1,2 oder mehr, vorzugsweise 1,3 oder mehr, vorzugsweise 1,4 oder mehr, vorzugsweise 1,5 oder mehr, vorzugsweise 1,8 oder mehr, vorzugsweise 2,0 oder mehr, vorzugsweise 2,5 oder mehr, vorzugsweise 3 oder mehr aufweisen, (b) 5 oder weniger, vorzugsweise 4 oder weniger, vorzugsweise 3,5 oder weniger, vorzugsweise 3 oder weniger, vorzugsweise 2,5 oder weniger, vorzugsweise 2 oder weniger, vorzugsweise 1,5 oder weniger, vorzugsweise 1,4 oder weniger, vorzugsweise 1,3 oder weniger, vorzugsweise 1,2 oder weniger, vorzugsweise 1,1 oder weniger, und/oder (c) zwischen 1,1 und 5, vorzugsweise zwischen 1,1 und 3, vorzugsweise zwischen 1,2 und 2. Alternativ oder ergänzend strahlt dabei die erste Wärmequelle mit einer Temperatur von zwischen 100 °C und 300 °C, vorzugsweise zwischen 150 °C und 250 °C, ab, und/oder die zweite Wärmequelle mit einer Temperatur von zwischen 50 °C und 250 °C, vorzugsweise zwischen 100 °C und 200 °C, ab.

Beispielsweise können zwei, drei, vier, fünf oder mehr als fünf Zonen unterschiedlicher Temperatur bestehen und dazu beispielsweise entsprechend viele, also zwei, drei, vier, fünf oder mehr als fünf, Wärmequellen eingesetzt sein, die zumindest teilweise unterschiedliche Wärmemengen pro Zeiteinheit bereitstellen.

Grundsätzlich können beispielsweise die Vielzahl von Wärmequellen zumindest teilweise entlang der ersten Richtung beabstandet zueinander angeordnet sein. Wenn die erste Richtung eine beim Gebrauch der Einheit laterale Richtung darstellt, kann damit zuverlässig ein homogenes Wärmefeld auf beiden Seiten der Wirbelsäule des Lebewesens erreicht werden.

Alternativ oder ergänzend kann vorgesehen sein, dass das Steuermodul dazu eingerichtet ist, die Vielzahl von Wärmequellen, insbesondere in Abhängigkeit voneinander und/oder von der gemessenen Temperatur, derart zu steuern, dass zumindest bereichsweise innerhalb einer außerhalb der Einheit verlaufenden Ebene und/oder zumindest bereichsweise innerhalb einer Fläche, die mit einer spezifischen Oberfläche der Einheit zusammenfällt, ein homogenes Wärmefeld bereitgestellt wird, insbesondere entlang der ersten Richtung und/oder der zweiten Richtung.

Ein homogenes Wärmefeld ermöglicht eine besonders zuverlässige und schonende Behandlung der Hautpartie.

Beispielsweise ist es bevorzugt, dass das Steuermodul dazu eingerichtet ist, die Wärmequellen derart zu steuern, dass die näher an einem seitlichen Gehäuseabschnitt angeordneten Wärmequellen mehr Wärmemengen bereitstellen als, die weiter davon entfernt angeordneten Wärmequellen.

Bei einer entsprechenden Konfiguration kann überraschenderweise ein besonders homogenes Wärmefeld auf der Hautpartie erreicht werden. Die Erfinder erklären sich dies damit, dass von der Wärme, die von einer Wärmequelle bereitgestellt wird, ein größerer Anteil nach außerhalb der Einheit zur Hautpartie gelangt, wenn diese weiter von dem seitlichen Gehäuseabschnitt entfernt angeordnet ist. Gewissermaßen treten für die einzelnen Wärmequellen unterschiedliche Abschattungseffekte infolge des Gehäuses ein.

Alternativ oder ergänzend kann vorgesehen sein, dass die spezifische Oberfläche eine Oberfläche einer Auflagefläche der Einheit ist, auf der die Hautpartie bei Benutzung der Einheit anordenbar oder angeordnet ist, die spezifische Oberfläche insbesondere eine Oberfläche der von dem Berührungsschutz oder dem Abstandshalter bereitgestellten oder ausgebildeten Auflagefläche ist.

Dadurch kann eine homogene Wärmeverteilung an der Hautpartie erreicht werden. Dabei kann die spezifische Oberfläche auch gekrümmt sein.

Gemäß einem fünften Aspekt ist eine Anordnung offenbart, umfassend (i) zwei oder mehr als zwei, vorzugsweise drei, vier, fünf oder mehr als fünf, Infrarotheizmodule gemäß dem zweiten Aspekt der Erfindung und/oder (ii) zwei oder mehr als zwei, vorzugsweise drei, vier, fünf oder mehr als fünf, Einheiten gemäß dem vierten Aspekt der Erfindung.

Indem mehrere Einheiten gemeinsam verwendet werden, lässt sich eine sichere und zudem auch homogene Wärmebehandlung auch entlang eines größeren Hautbereichs zuverlässig erreichen.

Die Einheiten können mittels elektrischer Verbindungskabel elektrisch miteinander verbunden sein. Hierüber können beispielsweise Steuersignale und/oder die Versorgungsspannung verteilt werden.

Alternativ oder ergänzend kann vorgesehen sein, dass die Anordnung zumindest ein Führungselement aufweist, mittels dessen die Einheiten relativ zueinander fix oder veränderlich positionierbar sind,

insbesondere (a) das Führungselement einen Teleskopauszug aufweist und die Einheiten an unterschiedlichen Segmenten des Teleskopauszugs angeordnet sind und/oder (b) das Führungselement eine Schiene aufweist, entlang derer die Einheiten zumindest abschnittsweise bewegbar und/oder mittels einer Rast-, Klemm-, und/oder Reibverbindung arretierbar sind.

Beispielsweise können die Einheiten entlang der Schiene bewegt werden und an unterschiedlichen Positionen können von der Einheit aufgewiesene Zapfen in Öffnungen der Schiene rastend eingreifen, um die Einheit somit an einer Position zu arretieren.

Alternativ oder ergänzend kann vorgesehen sein, dass die Steuermodule der einzelnen Einheiten von einem von der Anordnung aufgewiesenen gemeinsamen Steuermodul zumindest teilweise bereitgestellt sind,

und wobei vorzugsweise das gemeinsame Steuermodul ferner dazu eingerichtet ist, die Wärmequellen der einzelnen Einheiten derart zu steuern, dass bei Benutzung der Anordnung die am Ort einer im Abstand von 10 cm von den Temperatursensoren befindliche Hautpartie auch zwischen den einzelnen Einheiten einer homogene Wärme-Intensität ausgesetzt wird, insbesondere indem die von den einzelnen Wärmequellen pro Zeiteinheit abgegebenen Wärmemengen zumindest teilweise aufeinander abgestimmt werden.

Dadurch kann auch im Übergangsbereich zwischen zwei Einheiten ein besonders homogene Wärmefeld erreicht werden. Dies ermöglicht eine besonders gute Wärmebehandlung.

Ein homogenes Wärmefeld kann in einer Ausführungsform dadurch erreicht werden, dass eine aktive Temperaturregelung mittels mehrerer Sensoren realisiert ist und dass Bereiche der Anordnung unterschiedliche Abstrahltemperaturen aufweisen. Insbesondere können für seitlich bzw. außen angeordnete Einheiten höhere Abstrahltemperaturen vorgesehen sein als für zwischen diesen liegende mittlere Einheit.

Die Anordnung kann dazu ausgebildet und derart geregelt sein, dass ein homogenes Wärmefeld in lateraler bzw. horizontaler Richtung und in Strahlrichtung erzeugt wird. Dies kann mittels der unterschiedlichen Abstrahltemperaturen zwischen den Elementen, der sensorgesteuerten Regelung und/oder einer strukturellen Ausgestaltung der Anordnung realisiert sein. Beispielsweise können drei Einheiten miteinander verbunden, jedoch thermisch voneinander entkoppelt sein, wobei zwei seitliche bzw. äußere Einheiten eine mittlere Einheit einschließen. Die beiden seitlichen bzw. äußeren Einheiten können mit eine höhere Abstrahltemperatur als die mittlere Einheit aufweisen und/oder die beiden seitlichen bzw. äußeren Einheiten können zumindest teilweise in Richtung des zu bestrahlenden Objekts geneigt sein. Mit anderen Worten kann der Abstand zwischen dem zu bestrahlenden Objekt und jeder der beiden seitlichen bzw. äußeren Einheiten geringer sein als der Abstand zwischen dem zu bestrahlenden Objekt und der mittleren Einheit.

Alternativ oder ergänzend kann vorgesehen sein, dass eine Vorrichtung gemäß dem ersten Aspekt der Erfindung, eine Einheit gemäß dem vierten Aspekt der Erfindung oder eine Anordnung gemäß dem fünften Aspekt der Erfindung ferner eine Rückenlehne und/oder eine Kopfstütze aufweist und vorzugsweise diese mit dem Gehäuse zumindest einer Einheit verbunden ist, wobei vorzugsweise die Rückenlehne mehrteilig ausgeführt ist und die Teile horizontal und/oder vertikal, insbesondere auf Schienen, relativ zueinander verschiebbar sind, und/oder die Rückenlehne angepasst ist, Vibrationen und/oder oszillierende Bewegungen eines innerhalb der Rückenlehne verfahrbaren Elements zu erzeugen.

Eine Rückenlehne sowie eine Kopfstütze ermöglichen einen besonders sicheren Einsatz der Anordnung.

Rückenlehne sowie Kopfstütze kann jeweils ein geschäumtes Kunststoffteil aufweisen. Alternativ kann auch ein Schaumstoff mit einem Kunstlederbezug verwendet werden.

Die Rückenlehne hat beispielsweise eine Breite von 20 cm oder weniger, vorzugsweise von 15 cm oder weniger, und/oder von 5 cm oder mehr, vorzugsweise von 10 cm oder mehr. Beispielsweise hat sie eine Breite von 10 cm.

Indem die Rückenlehne mehre Teile hat, beispielsweise eine linke Hälfte und eine rechte Hälfte, kann die Rückenlehne an die Ergonomie eines Benutzers sehr gut angepasst werden.

Indem die Rückenlehne Vibrationen erzeugt, kann die Wärmebehandlung durch die Entspannung der Muskulatur unterstützt werden. Eine Massagefunktion ist vor allem auch durch die oszillierenden Bewegungen des innerhalb der Rückenlehne verfahrbaren Elements möglich. Durch das verfahrbare Element können unterschiedliche Regionen der Hautpartie mechanisch stimuliert werden und dadurch die Wärmebehandlung positiv beeinflusst werden.

In einer Ausführungsform ist die Rückenlehne und/oder die Kopfstütze dazu eingerichtet, eine Hautwiderstandsmessung durchzuführen, insbesondere um eine Anwendererkennung durchzuführen und vorzugsweise anhand eines erkannten Anwenders verschiedene medizinzisch-physiologische Parameter zu laden, auszuwerten und/oder dem Anwender anzubieten. Dies ermöglicht einen besonders zuverlässigen Betrieb.

Gemäß einem sechsten Aspekt ist eine Kabine offenbart, insbesondere Sauna, aufweisend zumindest eine Vorrichtung gemäß dem ersten Aspekt der Erfindung, zumindest ein Infrarotheizmodul gemäß dem zweiten Aspekt der Erfindung, zumindest eine Vorrichtung gemäß dem dritten Aspekt der Erfindung, zumindest eine Einheit gemäß dem vierten Aspekt der Erfindung oder zumindest eine Anordnung gemäß dem fünften Aspekt der Erfindung.

Die Wärmebehandlung lässt sich besonders gut durchführen, wenn die Einheit oder die Anordnung in einer Kabine integriert ist. Insbesondere in einer Sauna führt die Tiefenwirkung der von der oder den Einheiten abgegebenen Infrarotstrahlung in Kombination mit der in der Sauna bestehenden Wärme zu einer besonders vorteilhaften Wirkung.

Gemäß einem siebenten Aspekt ist eine Wärmestaudecke offenbart, zumindest eine Vorrichtung gemäß dem ersten Aspekt der Erfindung, zumindest ein Infrarotheizmodul gemäß dem zweiten Aspekt der Erfindung, zumindest eine Vorrichtung gemäß dem dritten Aspekt der Erfindung, zumindest eine Einheit gemäß dem vierten Aspekt der Erfindung oder zumindest eine Anordnung gemäß dem fünften Aspekt der Erfindung.

Gemäß einem achten Aspekt ist eine Tragevorrichtung offenbart, umfassend zumindest eine Vorrichtung gemäß dem ersten Aspekt der Erfindung, zumindest ein Infrarotheizmodul gemäß dem zweiten Aspekt der Erfindung, zumindest eine Vorrichtung gemäß dem dritten Aspekt der Erfindung, zumindest eine Einheit gemäß dem vierten Aspekt der Erfindung oder zumindest eine Anordnung gemäß dem fünften Aspekt der Erfindung sowie zumindest ein Befestigungsmittel, wobei die Einheit oder Anordnung mittels des Befestigungsmittels an einer Hautpartie, vorzugsweise des Rückens, eines Lebewesens anordenbar ist.

Dadurch kann die Einheit / Anordnung mittels der Tragevorrichtung besonders einfach an dem Lebewesen angeordnet werden. Beispielsweise kann die Einheit / Anordnung damit sozusagen wie ein Rucksack an dem Rücken eines Lebewesens angeordnet werden. Dadurch ist der Einsatz besonders einfach möglich. Bei mehreren Einheiten kann damit ihre Positionierung entlang der Hautpartie ergonomisch erfolgen.

Es kann vorteilhafterweise vorgesehen sein, dass zumindest eines, vorzugsweise mehr als eines und/oder alle, der drei oder mehr als drei Infrarotheizmodule der Vorrichtung gemäß dem ersten Aspekt der Erfindung jeweils durch eine Einheit gemäß dem vierten Aspekt der Erfindung realisiert ist oder sind.

In einer Ausführungsform kann das Kontrollmodul der Vorrichtung gemäß dem ersten Aspekt der Erfindung durch ein Steuermodul einer Einheit gemäß dem vierten Aspekt der Erfindung, insbesondere ganz oder zumindest teilweise, realisiert sein.

Es kann vorteilhafterweise vorgesehen sein, dass die Wärmequelle in Form eines Infrarotstrahlers der Einheit gemäß dem vierten Aspekt der Erfindung durch ein Infrarotheizmodul gemäß dem zweiten Aspekt der Erfindung, insbesondere ganz oder zumindest teilweise, realisiert ist.

Beispielsweise kann die Anordnung gemäß dem fünften Aspekt der Erfindung eine Vorrichtung gemäß dem ersten Aspekt der Erfindung sein oder aufweisen und umgekehrt. In einer Ausführungsform kann das Kontrollmodul der Vorrichtung gemäß dem ersten Aspekt der Erfindung durch ein gemeinsames Steuermodul der Anordnung gemäß dem fünften Aspekt der Erfindung, insbesondere ganz oder zumindest teilweise, realisiert sein.

In einer Ausführungsform können einzelne Aspekte der Erfindung kombiniert werden und/oder der eine Aspekt Details des anderen Aspekts ganz oder teilweise spezifizieren, wobei die bei den unterschiedlichen Aspekten der Erfindung gleich benannten Merkmale vorzugsweise identisch sind. Beispielsweise kann das Abstrahlelement der Einheit gemäß dem vierten Aspekt der Erfindung identisch zu dem Abstrahlelement des Infrarotheizmoduls gemäß dem zweiten Aspekt der Erfindung sein. Auch ungleich benannte Merkmale verschiedener Aspekte der Erfindung können vorzugsweise identisch sein, was sich dann für den Fachmann insbesondere jeweils aus dem Zusammenhang ergeben kann. Dementsprechend können die einzelnen Aspekte optional kombiniert werden.

Kurzbeschreibung der Figuren

Weitere Merkmale und Vorteile der Erfindung ergeben sich aus der nachfolgenden Beschreibung, in der bevorzugte Ausführungsformen der Erfindung anhand schematischer Zeichnungen erläutert werden.

Dabei zeigen:

Fig. 1a    eine schematische Querschnittsansicht einer erfindungsgemäßen Vorrichtung in einer ersten Konfiguration;

Fig. 1b    die Ansicht aus Fig. 1a mit eingezeichnetem Betrachtungsbereich;

Fig. 2    eine schematische Frontalansicht der Vorrichtung aus Fig. 1a;

Fig. 3    eine schematische Querschnittsansicht der erfindungsgemäßen Vorrichtung aus Fig. 1a in einer zweiten Konfiguration;

Fig. 4    eine schematische Querschnittsansicht eines erfindungsgemäßen Infrarotheizmoduls in einer ersten Ausführungsform;

Fig. 5     eine schematische Frontalansicht des Infrarotheizmoduls aus Fig. 4;

Fig. 6a    eine schematische Seitenansicht eines erfindungsgemäßen Infrarotheizmoduls in einer zweiten Ausführungsform;

Fig. 6b    eine vergrößerte Darstellung eines Ausschnitts aus Fig. 6a;

Fig. 7     eine schematische Frontalansicht des Infrarotheizmoduls aus Fig. 6;

Fig. 8     einen Schnellspannrahmen;

Fig. 9     eine schematische Frontalansicht einer erfindungsgemäßen Vorrichtung mit einem erfindungsgemäßen Infrarotheizmodul;

Fig. 10a   eine noch weitere Ausführungsform einer erfindungsgemäßen Vorrichtung in einer perspektivischen Ansicht von schräg vorne;

Fig. 10b   die Vorrichtung aus Fig. 10a in einer perspektivischen Ansicht von schräg hinten;

Fig. 10c   einen Ausschnitt mit einem oberen Teil der Vorrichtung aus Fig. 10a in einer kompakten Konfiguration in einer perspektivischen Ansicht von schräg hinten;

Fig. 10d   die Vorrichtung aus Fig. 10c mit hervorgehobenen den Infrarotheizmodulen in einer perspektivischen Ansicht von vorne;

Fig. 11    ein Abstützelement in einer vergrößerten Darstellung;

Fig. 12    eine alternative Ausführung eines Podestelements;

Fig. 13    eine schematische Querschnittsansicht einer erfindungsgemäßen Einheit gemäß dem vierten Aspekt der Erfindung;

Fig. 14    eine schematische Aufsicht auf Teile einer erfindungsgemäßen Einheit gemäß dem vierten Aspekt der Erfindung;

Fig. 15a   eine schematische Ansicht einer erfindungsgemäßen Anordnung gemäß dem fünften Aspekt der Erfindung in einer ersten Konfiguration von vorne;

Fig. 15b   eine schematische Ansicht der erfindungsgemäßen Anordnung aus Fig. 15a in einer zweiten Konfiguration von vorne;

Fig. 15c   eine schematische Ansicht der erfindungsgemäßen Anordnung aus Fig. 15a in der ersten Konfiguration von hinten;

Fig. 15d   eine schematische Ansicht der erfindungsgemäßen Anordnung aus Fig. 15b in der zweiten Konfiguration von hinten; und

Fig. 16    eine schematische Ansicht einer erfindungsgemäßen Tragevorrichtung gemäß dem achten Aspekt der Erfindung.

**Beispiele**

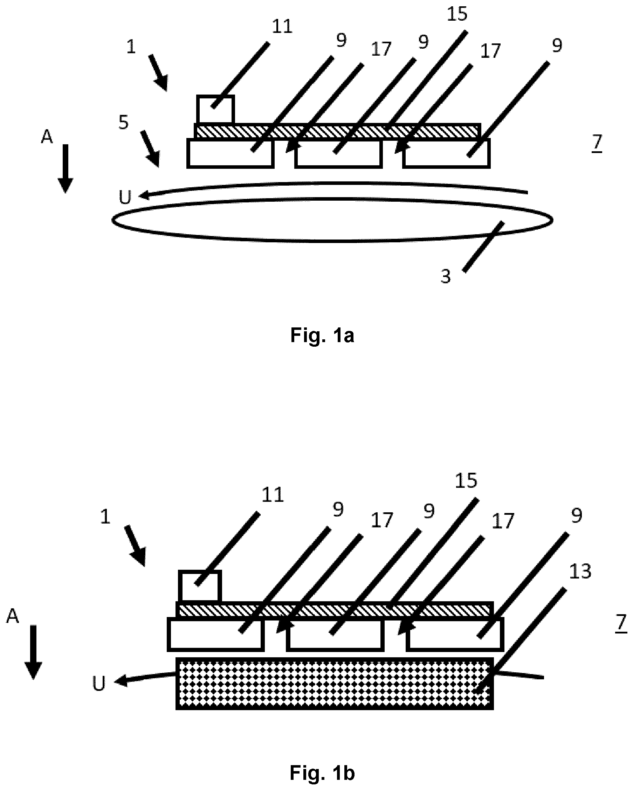

Figur 1 zeigt eine schematische Querschnittsansicht einer erfindungsgemäßen Vorrichtung 1 in einer ersten Konfiguration.

Die Vorrichtung 1 ist in Fig. 1 bei einem Einsatz gezeigt, bei dem sie relativ zu und an einem Körperteil, hier einem Rumpf 3, eines Menschen angeordnet ist. Der Rumpf 3 weist eine Hautpartie 5 des Rückens des Menschen auf. Die Zeichenebene der Figur 1 und damit die Schnittebene der Vorrichtung 1 entspricht einer spezifischen Transversalebene 7 des Menschen.

Die Vorrichtung 1 weist insgesamt drei Infrarotheizmodule 9 auf. Alle der Infrarotheizmodule 9 weisen eine Schnittfläche mit der spezifischen Transversalebene 7 auf. Mit anderen Worten ausgedrückt, sind also die drei Infrarotheizmodule 3 entlang einer Umfangsrichtung U an dem Rumpf 3, genauer gesagt, dem Rücken des Menschen, angeordnet.

Jedes der Infrarotheizmodule 9 ist auf einer individuellen Temperatur betreibbar. Diese Betriebstemperatur bestimmt die von dem jeweiligen Infrarotheizmodul abgegebene Wärmestrahlung. Vorliegend ist hauptsächlich die in Richtung der Hautpartie 5 abgegebene Wärmestrahlung relevant.

Die Vorrichtung 1 weist auch ein Kontrollmodul 11 auf. Dieses ist dazu eingerichtet, die Betriebstemperaturen der einzelnen Infrarotheizmodule 9 zu kontrollieren. Dies erfolgt dabei derart, dass die beiden seitlichen Infrarotheizmodule (also die in Fig. 1 links und rechts befindlichen Infrarotheizmodule 9) auf einer gleichen Betriebstemperatur betrieben werden, also entsprechend kontrolliert werden. Demgegenüber wird das mittlere der drei Infrarotheizmodule 9 in Fig. 1 auf einer niedrigeren Temperatur betrieben, also von dem dazu eingerichteten Kontrollmodul 11 entsprechend kontrolliert.

Vorliegend beträgt die Betriebstemperatur der beiden seitlichen Infrarotheizmodule 9 250 °C und die Betriebstemperatur des mittleren Infrarotheizmoduls beträgt 200 °C.

Indem also die beiden seitlichen Infrarotheizmodule 9 auf gleicher Temperatur betrieben werden, und das mittlere Infrarotheizmodul 9 auf einer niedrigeren Betriebstemperatur betrieben wird, ist innerhalb eines betrachteten Bereichs 13 (vgl. Fig. 1b) entlang einer Abstandsrichtung A zwischen Vorrichtung 1 und Rumpf 3 und der Umfangsrichtung U die beim Einsatz der Vorrichtung 1 von der Vorrichtung 1 bereitgestellte Intensität der Wärmestrahlung zum Zuführen an die Hautpartie 5 homogen.

In einer Ausführungsform weist die Vorrichtung 1 außerdem ein (in Fig. 1a nicht gezeigtes) Abstützelement zum zumindest teilweisen Abstützen der Vorrichtung 1 an dem Menschen auf.

Figur 1b zeigt dabei identisch die Vorrichtung 1 der Fig. 1a, wobei zusätzlich der betrachtete Bereich 13 als Überlagerung illustriert ist. Der betrachtete Bereich 13 erstreckt sich seitlich entlang des mittleren und jeweils Teilen der beiden seitlichen Infrarotheizmodule 9. Der betrachtete Bereich 13 liegt (ganz oder teilweise) auch zwischen der Vorrichtung 1 und dem Rumpf 3.

Das heißt, innerhalb des betrachteten Bereichs 13 ist entlang der Umfangsrichtung U und der Abstandsrichtung A die Schwankung der Intensität der Wärmestrahlung begrenzt. Ausgehend von jedem Punkt innerhalb des Bereichs 13 kann mithin in Abstandsrichtung A und/oder in Umfangsrichtung U die homogene Wärmestrahlung festgestellt werden. Das heißt für die Vorrichtung 1, dass die maximale und die minimale Intensität der Wärmestrahlung, $I_{max}$ und $I_{min}$, im Be-reich 13 der Ungleichung   $2\frac{Imax-Imin}{Imax+Imin} \leq 1,9$   genügen.

Das ermöglicht vorliegend eine zuverlässige Wärmebehandlung des Rumpfes 3 und dort insbesondere der Hautpartie 5. Denn trotz der Krümmung des Rückens und damit trotz des gekrümmten Verlaufs der Hauptpartie 5, kann in dem Bereich 13 eine homogene Wärmezufuhr zu der Hautpartie 5 ermöglicht werden. Außerdem ist die Vorrichtung 1 dadurch unempfindlich gegenüber relativen Verschiebungen zwischen Rumpf 3 und Vorrichtung 1.

Wie der Fig. 1 entnommen werden kann, sind Abstandsrichtung A und Umfangsrichtung U beide parallel zur Transversalebene 7. Der Bereich 13 (Fig. 1b) könnte sich außerdem auch teilweise entlang der Richtung senkrecht zur Zeichenebene erstrecken, was jedoch vorliegend nicht näher betrachtet wird. Im gesamten Volumenbereich (von dem der Bereich 13 die Schnittfläche mit der spezifischen Transversalebene 7 / Zeichenebene darstellt) könnte dann entlang der beiden Richtungen A und U die homogene Intensität der Wärmestrahlung festgestellt werden.

Die einzelnen Infrarotheizmodule 9 sind relativ zueinander entlang eines von der Vorrichtung aufgewiesenen Führungselements 15 verschiebbar und sind damit relativ zueinander veränderlich positionierbar.

Die einzelnen Infrarotheizmodule 9 sind durch Luftspalte 17 thermisch voneinander entkoppelt. Außerdem sind die einzelnen Infrarotheizmodule 9 durch eine (in Fig. 1a nicht dargestellte) Isolierung thermisch von dem Führungselement 15 entkoppelt.

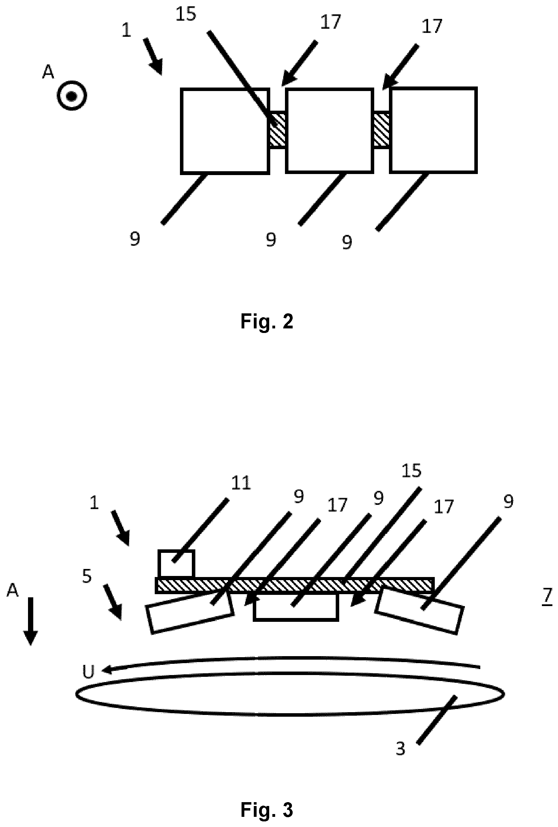

Fig. 2 zeigt eine schematische Frontalansicht der Vorrichtung 1 aus Fig. 1a, entgegen der Richtung A.

Fig. 3 zeigt eine schematische Querschnittsansicht der erfindungsgemäßen Vorrichtung 1 aus Fig. 1a in einer zweiten Konfiguration.

In der zweiten Konfiguration sind die beiden äußeren Infrarotheizmodule 9 gegenüber dem mittleren Infrarotheizmodul 9 abgewinkelt. Dadurch lässt sich die Form des Rumpfes 3 und damit die Krümmung der Hauptpartie 5 besser nachbilden und die Wärmestrahlung besser der Hautpartie 5 zuführen. Der Winkel zwischen der Normalen eines seitlichen und des mittleren Infrarotheizmoduls 9 liegt innerhalb der spezifischen Ebene 7.

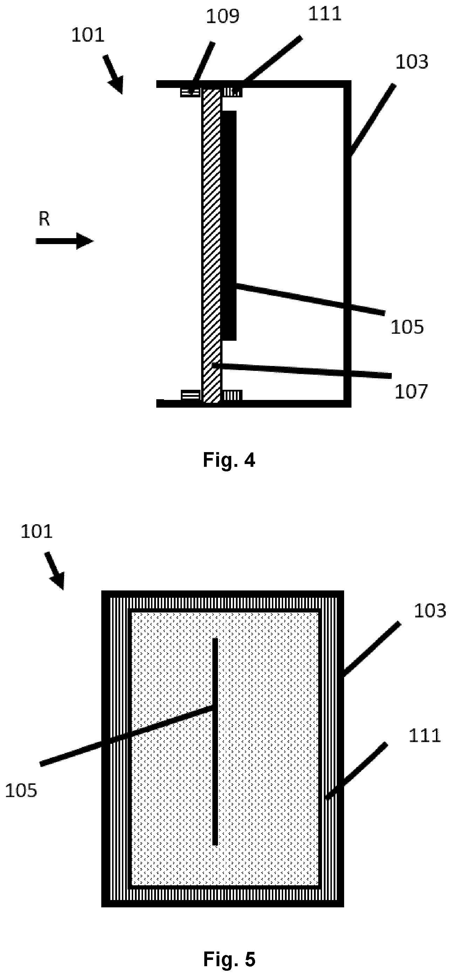

Fig. 4 zeigt eine schematische Querschnittsansicht eines erfindungsgemäßen Infrarotheizmoduls 101 in einer ersten Ausführungsform.

Das Infrarotheizmodul 101 weist ein Gehäuse 103 auf und ein innerhalb des Gehäuses angeordnetes Infrarotstrahlerelement 105 in Form eines Heizdrahtes. Außerdem weist das Infrarotheizmodul 101 ein scheibenförmiges, rechteckiges Abstrahlelement 107 auf, das an dem Gehäuse 103 lösbar angeordnet ist.

Genauer gesprochen ist das Abstrahlelement 107 von einem Arretierungsmittel 109 lösbar an einem Rand 111 des Gehäuses 103 gehalten, indem das Arretierungsmittel 109 in der in Fig. 4 gezeigten Arretierstellung rastend in das Gehäuse 103 eingreift und die auf dem Abstrahlelement 107 aufliegende Auflagefläche des Arretierungsmittels 109 das Abstrahlelement 107 an das Gehäuse 103, nämlich an dessen Rand 111, und an das Infrarotstrahlerelement 105 drückt. Dadurch wird das Abstrahlelement 107 form- und kraftschlüssig an dem Gehäuse 103 gehalten. Außerdem steht es auch in direktem Kontakt mit dem Infrarotstrahlerelement 105.

Indem das Arretierungsmittel 109 von dem Gehäuse 103 gelöst wird, das Arretierungsmittel 109 also in eine (in der Fig. 4 nicht dargestellten) Entnahmestellung überführt wird, lässt sich auch das Abstrahlelement 107 aus dem Gehäuse 103 entnehmen und zum Beispiel gegen ein anderes Abstrahlelement austauschen.

Fig. 5 zeigt eine schematische Frontalansicht des Infrarotheizmoduls 101 aus Fig. 4, und zwar entlang der Blickrichtung R in Fig. 4. Allerdings ist in Fig. 5 das Arretierungsmittel 109 und das Abstrahlelement 107 entfernt, so dass der Blick in das Innere des Gehäuses 103 frei ist. Gut zu erkennen ist dadurch der Rand 111 des Gehäuses 103, auf dem das Abstrahlelement 107 zum Aufliegen kommt. Nicht dargestellt ist in Fig. 5 eine Befestigung des Infrarotstrahlerelements 105 innerhalb des Gehäuses 103.

Mit dem Infrarotheizmodul 101 kann einer Hautpartie, die sich vor dem Abstrahlelement 107 befindet, Wärme zugeführt werden. Dazu wird beim Einsatz des Infrarotheizmoduls 101 der Heizdraht 105 mit Spannung beaufschlagt, wodurch dieser durch Konduktion das Abstrahlelement 107 aufwärmt. Dieses gibt dann seinerseits Wärmestrahlung ab.

Das Abstrahlelement 107 ermöglicht es dadurch, die Wärmestrahlung zu beeinflussen. Beispielsweise kann die Wärme über einen eher flächigen Bereich abgestrahlt werden (im Gegensatz zu dem eher linienförmigen Heizdraht 105). Das Abstrahlen erfolgt dann beispielsweise in Richtung der vor dem Infrarotheizmodul 101 befindlichen Hautpartie, also insbesondere entgegen der Richtung R.

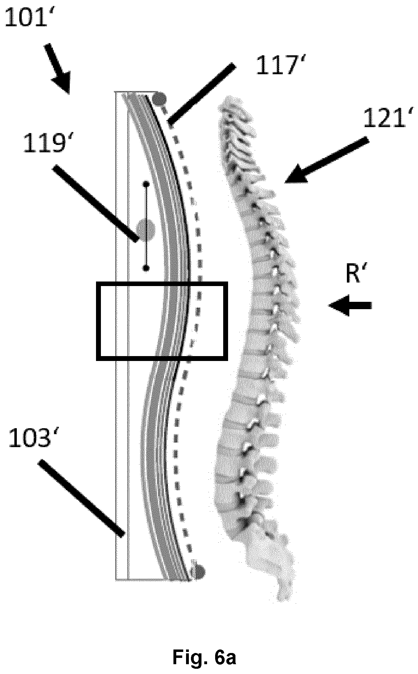

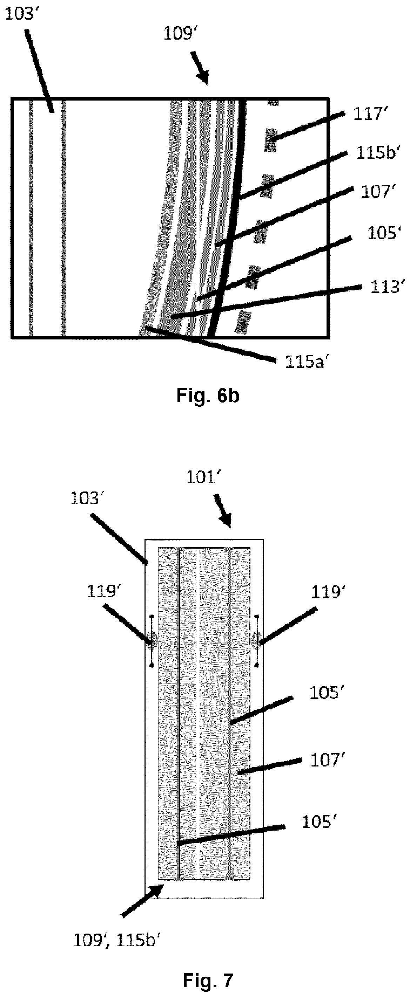

Fig. 6a zeigt eine schematische Seitenansicht eines erfindungsgemäßen Infrarotheizmoduls 101' in einer zweiten Ausführungsform. Fig. 6b zeigt den in Fig. 6a durch einen Kasten markierten Ausschnitt in einer vergrößerten Darstellung.

Merkmale des Infrarotheizmoduls 101', die funktional oder konstruktiv ähnlich zu denen des in Bezug auf die Figs. 4 und 5 besprochenen Infrarotheizmoduls 101 sind, sind mit gleichen, jedoch einfach gestrichenen Bezugszeichen versehen. Es kann daher in Bezug auf diese Merkmale ergänzend auch auf die oben in Bezug auf Figuren 4 und 5 gemachten Ausführungen verwiesen werden, sofern sich aus dem Zusammenhang nichts anderes ergibt.

Das Infrarotheizmodul 101' weist ein Gehäuse 103' auf, innerhalb dessen die einzelnen Komponenten des Infrarotheizmodul 101' angeordnet sind. Insbesondere also das Abstrahlelement 107' mit dahinter angeordneten Infrarotstrahlerelementen 105' sowie einer dahinter angeordneten thermischen Isolierung 113' in Form einer, beispielsweise 10 mm dicken, Aereogelschicht (alles entlang einer Blickrichtung R' von rechts in den Figuren 6a und 6b betrachtet). Insbesondere ist also das Infrarotstrahlerelementen 105' direkt an dem Abstrahlelement 107' angeordnet, so dass Wärme von dem Infrarotstrahlerelementen 105' an das Abstrahlelement 107' per Konduktion übertragen werden kann. Es wäre auch grundsätzlich eine beabstandete Anordnung von Infrarotstrahlerelementen 105' und Abstrahlelement 107' denkbar, wobei dann auch oder ausschließlich die von dem Infrarotstrahlerelementen 105' abgegebene Wärmestrahlung zu einer Aufwärmung des Abstrahlelements 107' führen würde.

Abstrahlelement 107', Infrarotstrahlerelement 105' und Isolierung 113' sind sandwichartig zwischen einem hinteren Teil 115a' und einem vorderen Teil 115b' eines Arretierungsmittels 109' in Form eines Schnellspannrahmens innerhalb des Gehäuses 103' vorgesehen.

Durch den Spannrahmen 109' lässt sich vor allem das Abstrahlelement 107' leicht auswechseln und so die Abstrahlcharakteristik des Infrarotheizmoduls 101' besonders einfach anpassen.

Außerdem weist das Infrarotheizmodul 101' einen Berührungsschutz 117' auf, der verhindert, dass das Abstrahlelement 107' versehentlich berührt wird (insbesondere dort, wo kein Spannrahmen 115b' sozusagen "im Weg" ist). Ferner weist das Infrarotheizmodul 101' auch Temperatursensoren 119' auf. Die Temperatursensoren 119' sind vertikal in der Höhe verschiebbar.

In Figur 6a ist außerdem der S-förmig gekrümmte Verlauf einer Wirbelsäule 121' eines Menschen dargestellt. Wie sich schön zeigt, sind die Komponenten 117', 115b', 107', 105', 113' und 115a' derart geformt, dass sie den Verlauf der Wirbelsäule 121' annähernd nachbilden. Dadurch ist das Infrarotheizmodul 101' sehr ergonomisch, passt sich dem Verlauf des Rückens eines Menschen an und ermöglicht dadurch eine zuverlässige und effektive Wärmebehandlung einer Hautpartie des Rückens.

Fig. 7 zeigt eine schematische Frontalansicht des Infrarotheizmoduls 101' aus Fig. 6a, und zwar entlang der Blickrichtung R' in Fig. 6a.

Anhand Fig. 7 ist erkennbar, dass das Infrarotheizmodul 101' zwei Temperatursensoren 119' aufweist (in Form von Infrarot-Temperatursensoren). Die beiden Infrarotstrahlerelemente 105' sind als linienförmige Heizdrähte ausgestaltet. Da sich diese (aus der Blickrichtung von vorne) hinter dem Abstrahlelement 107' befinden, dürften die beiden in Fig. 7 eigentlich nicht sichtbar sein. Jedoch sind sie zur Illustration dennoch in Fig. 7 dargestellt. Der Übersichtlichkeit halber nicht dargestellt ist in Fig. 7 hingegen der Berührungsschutz 117'.

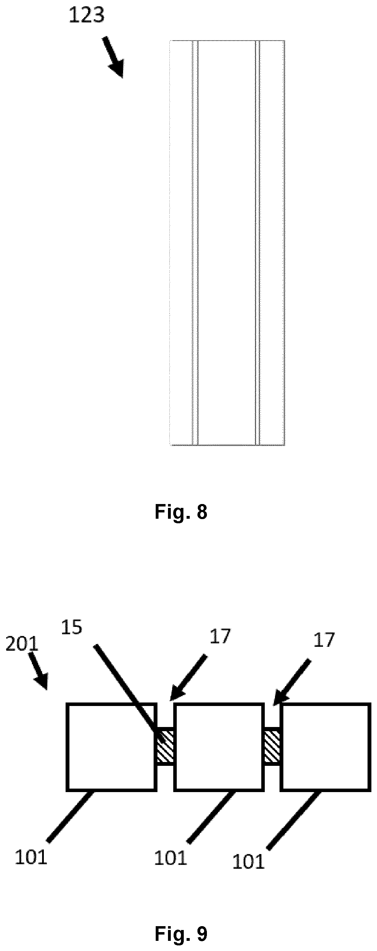

Fig. 8 zeigt einen Schnellspannrahmen 123, wie er beispielsweise in dem Infrarotheizmodul 101, das in Bezug auf Figuren 4 und 5 besprochen wurde oder in dem Infrarotheizmodul 101', das in Bezug auf Figuren 6 und 7 besprochen wurde, eingesetzt werden kann.

Der Schnellspannrahmen 123 ist aus Blech.

Fig. 9 zeigt eine schematische Frontalansicht einer erfindungsgemäßen Vorrichtung 201 mit drei erfindungsgemäßen Infrarotheizmodulen 101.

Die Vorrichtung 201 ist dabei vorliegend wie die Vorrichtung 1 aufgebaut, wobei die Infrarotheizmodule 9 der Vorrichtung 1 durch Infrarotheizmodule 101 realisiert sind. Daher kann hinsichtlich der weiteren Beschreibung der Vorrichtung 201 auf die oben gemachten Ausführungen zur Vorrichtung 1 und zum Infrarotheizmodul 101 verwiesen werden.

In einer Ausführungsform weist die Vorrichtung 201 außerdem ein (in Fig. 9 nicht gezeigtes) Abstützelement zum zumindest teilweisen Abstützen der Vorrichtung 201 an dem Menschen auf.

Die Vorrichtung 201 ermöglicht damit eine homogene Intensität der Wärmestrahlung, indem die einzelnen Infrarotheizmodule 101 mit individuellen Betriebstemperaturen betrieben werden, wobei die Homogenität durch das austauschbare Abstrahlelement der Infrarotheizmodule 101 besonders auf das jeweilige Anwendungsszenario abgestimmt werden kann.

Eine weitere Ausführungsform einer erfindungsgemäßen Vorrichtung ist ganz ähnlich zur in Fig. 9 dargestellten Vorrichtung 201, mit jedoch dem Unterschied, dass die Infrarotheizmodule durch Infrarotheizmodule 101' realisiert sind.

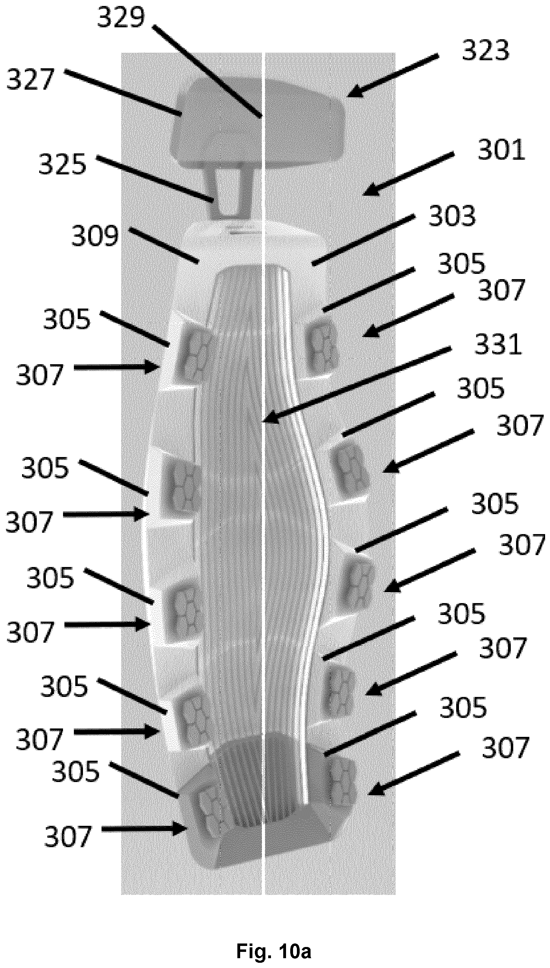

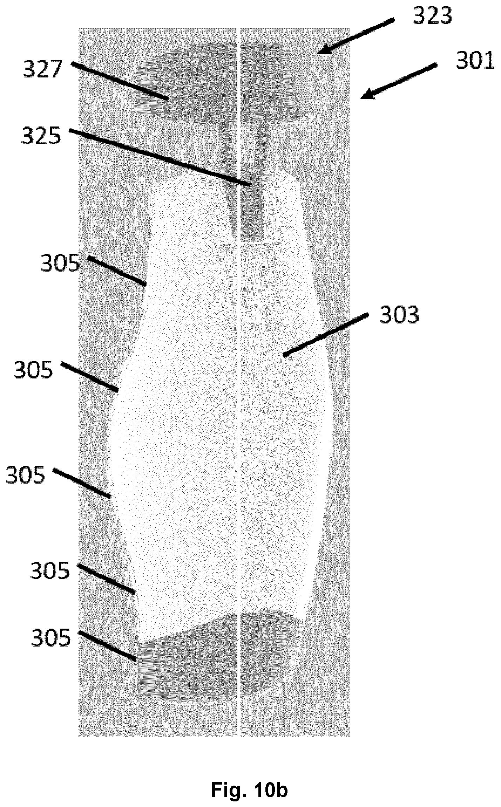

Fig. 10a zeigt eine noch weitere Ausführungsform einer erfindungsgemäßen Vorrichtung 301 in einer perspektivischen Ansicht von schräg vorne. Fig. 10b zeigt die Vorrichtung 301 in einer perspektivischen Ansicht von schräg hinten.

Die Vorrichtung 301 weist ein Gehäuse 303 auf, innerhalb dessen drei (in Figs. 10a und 10b nicht dargestellte) Infraroheizmodule angeordnet sind.

Die Vorrichtung 301 weist an der Frontpartie des Gehäuses 303 zehn pyramidenstumpfförmige Podestelemente 305 auf. Diese bilden eine Erhöhung aus.

Die Vorrichtung 301 weist außerdem zehn gleichartige Abstützelemente 307 auf. Diese bilden eine Rückenlehne aus und dienen daher dazu, die Vorrichtung 301 an einem Lebewesen, an dessen Hautpartie mit der Vorrichtung 301 Wärme zugeführt werden soll, abstützen zu können. Jedes Abstützelement ist auf jeweils einem der Podestelemente 305 und damit auch indirekt an der Frontpartie 309 des Gehäuses 303 angeordnet.

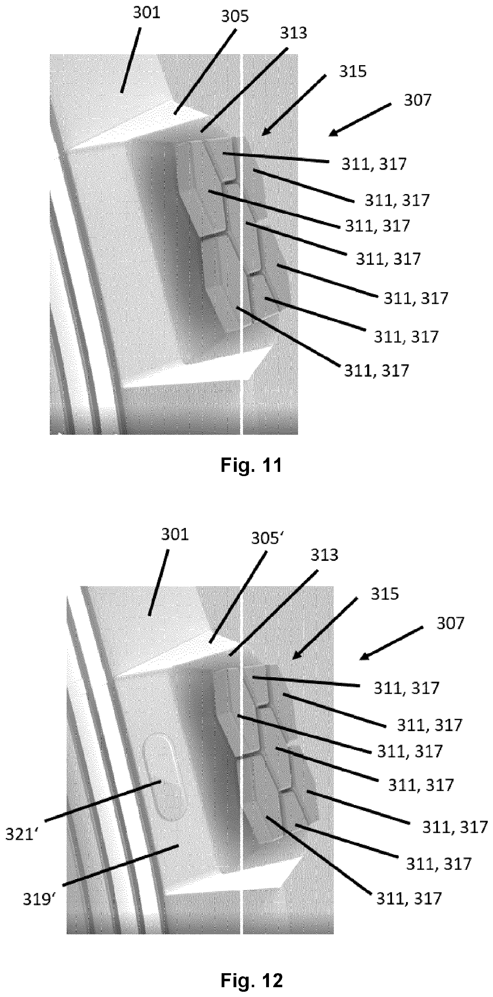

Fig. 11 zeigt ein Abstützelement 307 in vergrößerter Darstellung. Da alle Abstützelemente 307 in dieser Ausführungsform gleich sind, genügt es, ein einziges davon exemplarisch anhand Fig. 11 näher zu erläutern.

Das Abstützelement 307 (siehe Fig. 11) weist jeweils mehrere Einzelelemente 311 auf, die stoffschlüssig auf einem von dem Abstützelement 307 aufgewiesenen elastischen Grundelement 313 aus Silikon nebeneinander angeordnet sind. Werden die starren Einzelelemente 311 durch das Lebewesen belastet, sind diese gegeneinander bewegbar, da die Einzelelemente 311 in die Silikonschicht des elastischen Grundelements 313 gedrückt werden können und dadurch jeweils ihre Position und Lage individuell verändert werden kann.

Dadurch ist der Verlauf der von dem Abstützelement 307 bereitgestellten spezifischen Oberfläche 315 an den Verlauf einer Oberfläche des Lebewesens (wie die eines Rückenbereichs dessen) anpassbar. Jedes Einzelelement 311 weist dabei einen Oberflächenbereich 317 auf, der einen Teil der spezifischen Oberfläche 315 des Abstützelements 307 bereitstellt. Fünf der sieben Einzelelemente 311 des Abstützelements 305 stellen insofern eine wabenförmige, also sechseckige, Oberfläche bereit. Die anderen beiden jeweils eine viereckige Oberfläche.

In Fig. 12 ist eine alternative Ausführung zu den zuvor beschriebenen Podestelementen 305 mit darauf angeordnetem Abstützelement 307 dargestellt. Innerhalb des Podestelements 305' ist ein Infrarot-Temperatursensor vorgesehen, der dazu eingerichtet ist eine lokale Hauttemperatur des Lebewesens zu messen. Die Seitenfläche 319' des Podestelements 305' ist in einem Bereich 321' für die Infrarot-Temperaturmessung durchlässig. Indem die Seitenfläche 319' leicht schräg verläuft, hat der Infrarot-Temperatursensor einen guten Blickwinkel auf die Hautpartie.

Wieder in Bezug auf Fig. 10a und Fig. 10b. Die Vorrichtung 301 weist ferner eine Kopfstütze 323 auf, die mit einem Haltemittel 325 beabstandet von und an dem Gehäuse 303 befestigt ist. Die Kopfstütze 323 weist dabei eine Plastikschale 327 und ein daran angeordnetes flexibles Netzgewebe 329 auf, das eine Öffnung der Plastikschale 327 überspannt.

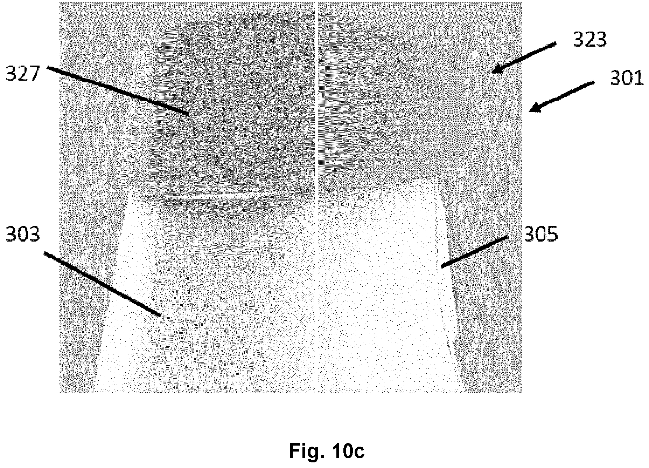

Fig. 10c zeigt einen Ausschnitt mit einem oberen Teil der Vorrichtung 301 aus Fig. 10a in einer kompakten Konfiguration in einer perspektivischen Ansicht von schräg hinten. In dieser kompakten Konfiguration (Fig. 10c) ist die Kopfstütze 323 als Gehäuseabdeckung im oberen Bereich der Vorrichtung 301 an dem Gehäuse 305 lösbar befestigt, etwa da die Vorrichtung nicht im Einsatz ist.

Die Vorrichtung 301 weist einen gitterförmigen Berührschutz 331 auf, der ein unbeabsichtigtes Berühren der Infraroheizmodule verhindert.

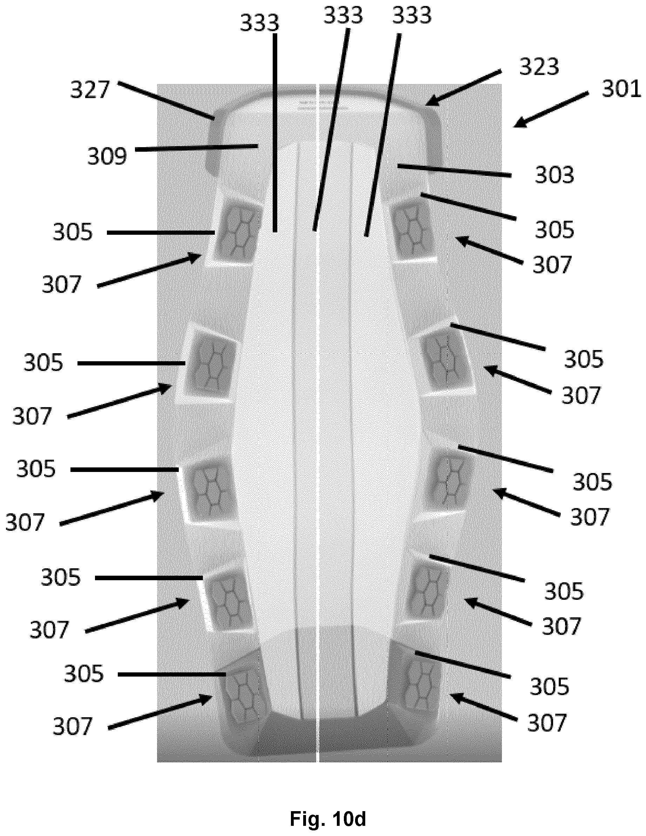

Fig. 10d zeigt die Vorrichtung 301 in der kompakten Konfiguration aus Fig. 10c ohne den Berührschutz aber mit den drei Infraroheizmodulen 333. Diese erstrecken sich beim Einsatz der Vorrichtung 301 streifenförmig entlang einer zu einer Transversalebene des Lebewesens senkrecht verlaufenden Richtung.

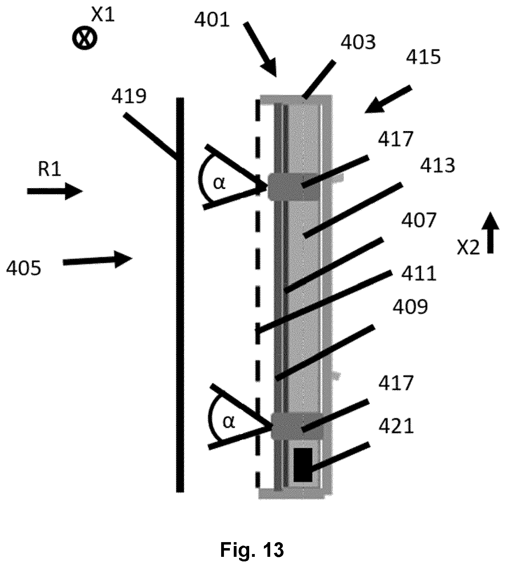

Figur 13 zeigt eine erfindungsgemäße Einheit 401 gemäß dem vierten Aspekt der Erfindung in einer schematischen Querschnittsansicht.

Die Einheit 401 weist ein Gehäuse 403 auf, das auf einer Seite eine Öffnung 405 hat. Innerhalb des Gehäuses 403 ist eine Wärmequelle 407 angeordnet. Die Wärmequelle 407 ist ein Infrarotstrahler, der einen Heizleiter aus Carbon aufweist. Der Heizleiter ist linienförmig und erstreckt sich in Fig. 13 vertikal von unten nach oben entlang einer Richtung X2. Von außen durch die Öffnung 405 entlang Richtung R1 in das Gehäuse 403 blickend, ist vor der Wärmequelle 407 ein von der Einheit 401 aufgewiesenes Abstrahlelement 409 innerhalb des Gehäuses 403 angeordnet.

Außerdem weist die Einheit 401 einen Berührungsschutz 411 auf, der mit dem Gehäuse 403 durch eine Schweißverbindung verbunden ist. Der Berührungsschutz 411 verhindert ein Berühren des Abstrahlelements 409, indem er beabstand zur der dem Berührungsschutz zugewandten Seite des Abstrahlelements 409 innerhalb des Gehäuses 403 angeordnet ist.

Wird die Wärmequelle 407 mit einer Spannung beaufschlagt, so gibt sie Wärme durch Wärmestrahlung ab. Ein Teil der abgestrahlten Wärme trifft auf das Abstrahlelement 409, wodurch dieses über einen ausgedehnten Flächenbereich hinweg erwärmt wird. Das Abstrahlelement 409 strahlt dann seinerseits Wärme an die Umgebung der Einheit 401 ab.

Die Einheit 401 weist außerdem auch eine Wärmeisolierung 413 auf, die zwischen einem der Öffnung 405 gegenüberliegenden Gehäuseabschnitt 415 und der Wärmequelle 407 angeordnet ist. Die Wärmeisolierung 413 verringert die Aufheizung des Gehäuses 403 im Bereich des Abschnitts 415 durch die von der Wärmequelle nicht in Richtung des Abstrahlelements 409 abgestrahlte Wärme.

Innerhalb des Gehäuses 403 sind zwei Infrarottemperatursensor 417 angeordnet, die in der Zeichenebene der Fig. 13 jeweils einen Sichtbereich (field of view) mit einem Winkel $\alpha$ aufweisen. Die Temperatursensoren 417 sind in Richtung X2 zueinander beabstandet angeordnet. Möglicherweise könnten sie auch in einer dazu senkrecht verlaufenden Richtung X1 einen Abstand zueinander aufweisen. Wenn die Einheit 401 in Gebrauch ist, also auch mit Spannung beaufschlagt ist, und sich eine Hautpartie 419 eines Lebewesens in einem gewissen Abstand vor der Öffnung 405 der Einheit 401 und damit vor den Sensoren 417 befindet, können die entsprechend dazu angepassten Temperatursensoren 417 die Temperatur von Teilen der Hauptpartie 417 messen. Genauer gesagt, misst jeder Temperatursensor 417 in der Zeichenebene der Fig. 13 jeweils einen Teilbereich der Hautpartie 419, dessen Größe durch den Winkel $\alpha$ und den Abstand zur Hautpartie 19 definiert wird. Offensichtlich, erfassen die beiden Sensoren 417 jeweils unterschiedliche Teile der Hautpartie 419, haben also kein überlappendes Erfassungsfeld.

Die Messdaten der Temperatursensoren 417 werden von einem von der Einheit 401 aufgewiesenen und entsprechend eingerichteten Steuermodul 421 dazu verwendet, die Wärmequelle 407 zu steuern, etwa die von ihr pro Zeiteinheit abgegebene Wärme anzupassen, insbesondere zu verringern oder zu erhöhen. Die Wärmequelle 407 wird dabei anhand der höchsten von den Temperatursensoren 417 gemessenen Temperatur gesteuert.

Wenn also ein von den Temperatursensoren 417 erfasster Bereich eine zu hohe Temperatur aufweist (etwa anhand eines vordefinierten Maximalwertes), kann die von der Wärmequelle 407 pro Zeiteinheit abgegebene Wärmemenge reduziert werden. Da vorliegend die höchste Temperatur zur Steuerung verwendet wird, ist sichergestellt, dass in keinem der mit den Temperatursensoren 417 überwachten Bereiche der Hautpartie 419 dauerhaft eine zu hohe Temperatur besteht.

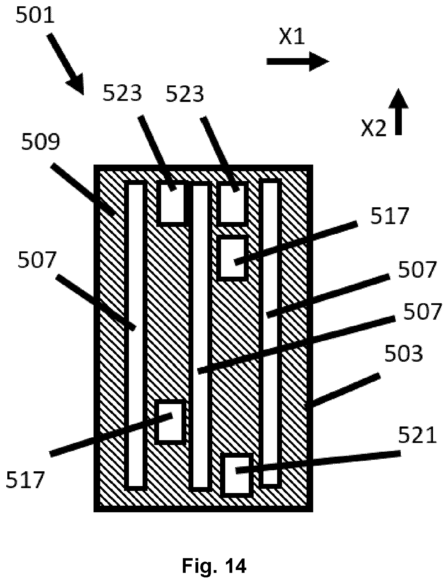

Fig. 14 zeigt eine schematische Aufsicht auf Teile einer erfindungsgemäßen Einheit 501 gemäß dem vierten Aspekt der Erfindung. Die Einheit 501 ist ähnlich zu der in Bezug auf Fig. 13 beschriebenen Einheit 401. Daher sind gleiche Merkmale mit gleichen, jedoch um den Wert 100 erhöhten Bezugszeichen versehen.

Die Aufsicht in Fig. 14 entspricht gewissermaßen in Fig. 13 einem Blick vom Innern des Gehäuses 403 entgegen der Richtung R.

Aus Fig. 14 geht eine mögliche relative Anordnung des Abstrahlelements 509 und der, vorliegend, drei Wärmequellen 507 der Einheit 501 deutlich hervor. Genauer gesprochen, sind die Carbon-Heizleiter der drei Wärmequellen 507 entlang einer ersten Richtung X1 äquidistant beabstandet zueinander angeordnet. Die Haupterstreckungsrichtung der Heizleiter 507 verläuft entlang einer zur ersten Richtung X1 senkrecht verlaufenden zweiten Richtung X2. Das Abstrahlelement weist entlang einer Richtung senkrecht zur Zeichenebene einen Abstand zu den Heizleitern 507 auf. In einer anderen Ausführungsform könnten die Heizleiter 507 aber auch direkt auf dem Abstrahlelement angeordnet sein.

Das Steuermodul 521 ist dazu eingerichtet, die drei Wärmequellen 507 auf teilweise unterschiedliche Temperaturen einzustellen, so dass diese teilweise unterschiedliche Wärmemengen pro Zeiteinheit bereitstellen. Vorliegend steuert das Steuermodul 521 die Wärmequellen 507 derart, dass die beiden in Fig. 14 äußeren der Wärmequellen 507 rund 30 % mehr Wärme bereitstellen als die mittlere der Wärmequellen 507.

Indem die Wärmequellen 507 abhängig voneinander gesteuert werden, kann am Ort einer in Fig. 14 hinter dem Abstrahlelement 509 angeordnete (in Fig. 14 nicht dargestellte) Hautpartie ein homogenes Wärmefeld erreicht werden. Dessen absolute Temperatur kann wiederum anhand der Messwerte der Temperatursensoren 517 angepasst werden.

Die Einheit 501 weist außerdem zwei innerhalb des Gehäuses 503 angeordneten LEDs 523 auf. Damit kann das Innere des Gehäuses 503 beleuchtet werden.

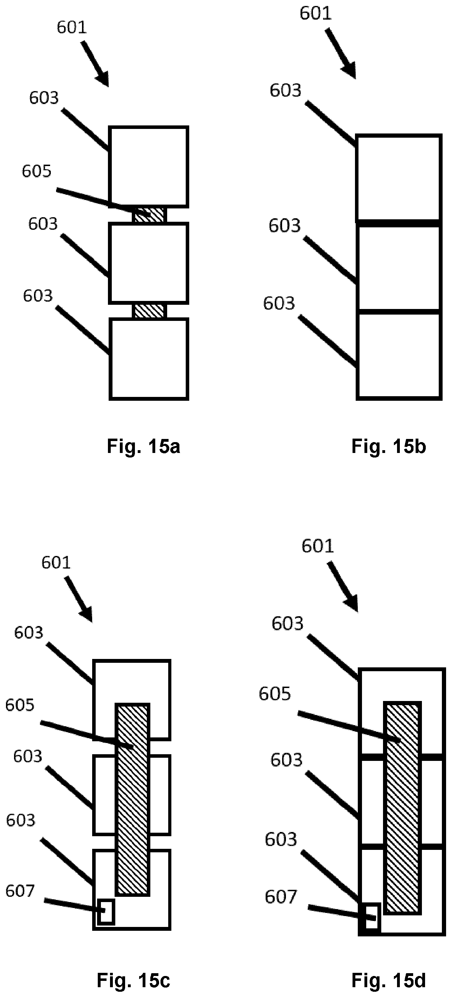

Fig. 15a zeigt eine schematische Ansicht einer erfindungsgemäßen Anordnung 601 gemäß dem fünften Aspekt der Erfindung in einer ersten Konfiguration von vorne.

Die Anordnung 601 weist drei Einheiten 603 gemäß dem vierten Aspekt der Erfindung auf. Jede der Einheiten 603 kann beispielsweise eine Einheit 401, wie sie in Bezug auf Fig. 13 beschrieben wurde, oder eine Einheit 501, wie sie in Bezug auf Fig. 14 beschrieben wurde, aufweisen oder darstellen.

Die Anordnung 601 weist ein Führungselement 605 auf, mittels dessen die Einheiten 603 relativ zueinander veränderlich positionierbar sind.

In der in Fig. 15a gezeigten ersten Konfiguration sind die Einheiten 603 so relativ zueinander positioniert, dass sie entlang des Führungselements 605 einen Abstand zueinander aufweisen.

Fig. 15b zeigt eine schematische Ansicht der Anordnung 603 in einer zweiten Konfiguration von vorne. In dieser weisen die einzelnen Einheiten 605 entlang des Führungselements 205 einen geringeren Abstand zueinander auf als in der ersten Konfiguration, so dass das Führungselements 605 in Fig. 15b auch nicht mehr zu sehen ist.

Die Fig. 15c zeigt die Anordnung 601 in der ersten Konfiguration von hinten und Fig. 15d zeigt die Anordnung 601 in der zweiten Konfiguration von hinten. Hierbei ist jeweils der Akku 607 erkennbar, der die Anordnung 601 mit Energie versorgt.

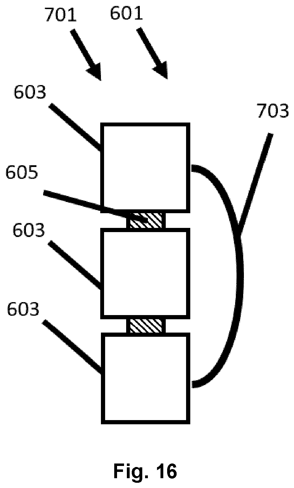

Fig. 16 zeigt eine schematische Ansicht einer Tragevorrichtung 701 gemäß dem achten Aspekt der Erfindung. Diese weist eine Anordnung gemäß dem fünften Aspekt der Erfindung, nämlich vorliegend Anordnung 601, wie sie in Bezug auf die Figs. 15a-d beschrieben wurde, auf. Außerdem weist die Tragevorrichtung 701 Befestigungsmittel 703 auf, womit die Anordnung 601 an einer Hautpartie des Rückens eines Menschen anordenbar ist.

Bezugszeichenliste

| 1, 201 | Vorrichtung |
| 3 | Rumpf |
| 5 | Hautpartie |
| 7 | Transversalebene |
| 9 | Infrarotheizmodul |
| 11 | Kontrollmodul |
| 13 | Bereich |
| 15 | Führungselement |
| 17 | Luftspalt |
| 101, 101' | Infrarotheizmodul |
| 103, 103' | Gehäuse |
| 105, 105' | Infrarotstrahlerelement |
| 107, 107' | Abstrahlelement |
| 109, 109' | Arretierungsmittel |
| 111 | Rand |
| 113' | Thermische Isolierung |
| 115a', 115b' | Teile eines Spannrahmens |
| 117' | Berührungsschutz |
| 119' | Temperatursensor |
| 121' | Wirbelsäule |
| 123 | Schnellspannrahmen |
| 301 | Vorrichtung |
| 303 | Gehäuse |
| 305, 305' | Podestelement |
| 307 | Abstützelement |
| 309 | Frontpartie |
| 311 | Einzelelement |
| 313 | Elastisches Grundelement |
| 315 | Spezifischen Oberfläche |
| 317 | Oberfläche |
| 319' | Seitenfläche |
| 321' | Bereich |
| 323 | Kopfstütze |
| 325 | Haltemittel |
| 327 | Plastikschale |
| 329 | Netzgewebe |
| 331 | Berührschutz |
| 333 | Infrarotheizmodul |
| 401, 501 | Einheit |
| 403, 503 | Gehäuse |
| 405 | Öffnung |
| 407, 507 | Wärmequelle |
| 409,509 | Abstrahlelement |
| 411 | Berührungsschutz |
| 413 | Wärmeisolierung |
| 415 | Gehäuseabschnitt |
| 417,517 | Temperatursensor |
| 419 | Hautpartie |
| 421,521 | Steuermodul |
| 523 | LED |
| 601 | Anordnung |
| 603 | Einheit |
| 605 | Führungselement |
| 607 | Akku |
| 701 | Tragevorrichtung |
| 703 | Befestigungsmittel |
| α | Winkel |
| R1 | Richtung |
| X1 | Richtung |
| X2 | Richtung |
| A | Abstandsrichtung |
| R, R' | Richtung |
| U | Umfangsrichtung |

**Patentansprüche**

1. Vorrichtung (1; 201; 301) zum Zuführen von Wärme an eine Hautpartie (5) eines Lebewesens, wobei die Vorrichtung (1; 201; 301) drei oder mehr als drei Infrarotheizmodule (9; 101, 101'; 333; 401; 501; 603) aufweist, die beim Einsatz der Vorrichtung (1; 201; 301) derart relativ zu und/oder an dem Lebewesen angeordnet sind, dass für eine definierte oder definierbare spezifische Transversalebene (7) des Lebewesens jedes der Infrarotheizmodule (9; 101, 101'; 333; 401; 501; 603) eine Schnittfläche mit der spezifischen Transversalebene (7) hat, **dadurch gekennzeichnet, dass**

   jedes Infrarotheizmodul (9; 101, 101'; 333; 401; 501; 603) auf einer individuellen Betriebstemperatur betreibbar ist, und
   wobei beim Einsatz der Vorrichtung (1; 201; 301) die Betriebstemperaturen der Infrarotheizmodule (9; 101, 101'; 333; 401; 501; 603) derart zumindest teilweise unterschiedlich eingestellt oder einstellbar sind, dass die Intensität der von der Vorrichtung (1; 201; 301) bereitgestellten Wärmestrahlung zum Zuführen an die Hautpartie (5) zumindest entlang einer ersten und einer zweiten jeweils parallel zur spezifischen Transversalebene (7) verlaufenden Richtung (A, U) innerhalb zumindest eines betrachteten Bereichs (13) außerhalb der Vorrichtung (1; 201; 301) homogen ist.

2. Vorrichtung nach Anspruch 1, wobei beim Einsatz der Vorrichtung (1; 201; 301) (a) die erste Richtung parallel zu einer definierten oder definierbaren Sagittalebene des Lebewesens ist und/oder die erste Richtung eine Abstandsrichtung (A) von der Vorrichtung (1; 201; 301) zu der Hautpartie ist, und sich vorzugsweise der betrachtete Bereich (13) 1 cm oder mehr und/oder 30 cm oder weniger entlang der ersten Richtung erstreckt, und/oder (b) die zweite Richtung eine Umfangsrichtung (U) des die Hautpartie (5) aufweisenden Körperteils oder Körperbereichs (3) des Lebewesens ist und/oder sich der betrachtete Bereich (13) 10 cm oder mehr und/oder 100 cm oder weniger entlang der zweiten Richtung erstreckt.

3. Vorrichtung nach einem der vorangehenden Ansprüche, wobei (a) der betrachtete Bereich (13) ein Volumenbereich, vorzugsweise mit wenigstens 50 cm³ und/oder mit höchstens 30000 cm³, ist, und/oder der betrachtete Bereich (13) beim Einsatz der Vorrichtung (1; 201; 301) zumindest teilweise zwischen Vorrichtung (1; 201; 301) und Hautpartie (5) liegt, und/oder (b) der betrachtete Bereich (13) sich seitlich entlang zumindest zweier benachbarter Infrarotheizmodule (9; 101, 101'; 333; 401; 501; 603) zumindest teilweise erstreckt.

4. Vorrichtung nach einem der vorangehenden Ansprüche, wobei beim Einsatz der Vorrichtung (1; 201; 301) die Betriebstemperatur der einzelnen Infrarotheizmodule (9; 101, 101'; 333; 401; 501; 603) jeweils eingestellt oder einstellbar ist auf zwischen 100 °C und 400 °C, vorzugsweise zwischen 100 °C und 300 °C, vorzugsweise zwischen 150 °C und 300 °C, vorzugsweise zwischen 150 °C und 250 °C oder zwischen 200 °C und 300 °C, und/oder wobei beim Einsatz der Vorrichtung (1; 201; 301) die Betriebstemperatur der beiden, vorzugsweise in der spezifischen Transversalebene (7), seitlichen Infrarotheizmodule (9; 101, 101'; 333; 401; 501; 603) jeweils (a) bis zu 50 %, vorzugsweise bis zu 40 %, vorzugsweise bis zu 30 %, vorzugsweise bis zu 20 %, vorzugsweise bis zu 10 %, größer eingestellt oder einstellbar ist als die höchste Betriebstemperatur der übrigen Infrarotheizmodule (9; 101, 101'; 333; 401; 501; 603) und/oder (b) 1% oder mehr, vorzugsweise 5 % oder mehr, vorzugsweise 10 % oder mehr, größer eingestellt oder einstellbar ist als die höchste Betriebstemperatur der übrigen Infrarotheizmodule (9; 101, 101'; 333; 401; 501; 603).

5. Vorrichtung nach einem der vorangehenden Ansprüche, wobei die Vorrichtung (1; 201; 301) ein Kontrollmodul (11) aufweist, das dazu eingerichtet ist, die Betriebstemperaturen der einzelnen Infrarotheizmodule (9; 101, 101'; 333; 401; 501; 603), vorzugsweise basierend auf zumindest einem von einem Anwender ausgewählten oder auswählbaren Betriebsmodus, zu kontrollieren, insbesondere zu regeln oder zu steuern, wobei, optional:

(a) das Kontrollmodul (11) dazu eingerichtet ist, wenigstens die beiden beim Einsatz der Vorrichtung (1; 201; 301) seitlichen Infrarotheizmodule (9; 101, 101'; 333; 401; 501; 603) auf einer gleichen Betriebstemperatur zu betreiben und/oder wenigstens zwei der Infrarotheizmodule (9; 101, 101'; 333; 401; 501; 603) mit einer unterschiedlichen Betriebstemperatur zu betreiben, und/oder (b) das Kontrollmodul (11) dazu eingerichtet ist, die beiden beim Einsatz der Vorrichtung (1; 201; 301) seitlichen Infrarotheizmodule (9; 101, 101'; 333; 401; 501; 603) mit gleicher Betriebstemperatur zu betreiben, die größer ist als jede der Betriebstemperaturen, auf denen die übrigen Infrarotheizmodule (9; 101, 101'; 333; 401; 501; 603) betrieben werden, und/oder

die Vorrichtung (1; 201; 301) einen oder mehrere Sensoren (119'; 417; 517) aufweist, die dazu eingerichtet sind, jeweils eine lokale Temperatur der Hautpartie (5) zu messen und wobei das Kontrollmodul (11) dazu eingerichtet ist, zumindest basierend auf den gemessenen Temperaturwerten die Betriebstemperaturen der einzelnen Infrarotheizmodule (9; 101, 101'; 333; 401; 501; 603) zu kontrollieren, insbesondere zu regeln oder zu steuern, und wobei vorzugsweise die Sensoren (119'; 417; 517) matrixförmig angeordnet sind.

6. Vorrichtung nach einem der vorangehenden Ansprüche, wobei (a) wenigstens zwei der Infrarotheizmodule (9; 101, 101'; 333; 401; 501; 603) relativ zueinander verschiebbar sind, insbesondere in einer Richtung, die beim Einsatz der Vorrichtung (1; 201; 301) parallel zu der spezifischen Transversalebene (7) verläuft, (b) wenigstens zwei, vorzugsweise drei oder mehr als drei, der Infrarotheizmodule (9; 101, 101'; 333; 401; 501; 603) relativ zueinander neigbar sind und/oder wobei wenigstens zwei, vorzugsweise drei oder mehr als drei, der Infrarotheizmodule (9; 101, 101'; 333; 401; 501; 603) relativ zueinander abwinkelbar sind, und/oder (c) wenigstens zwei benachbarte Infrarotheizmodule (9; 101, 101'; 333; 401; 501; 603), vorzugsweise alle paarweise benachbarten Infrarotheizmodule (9; 101, 101'; 333; 401; 501; 603), thermisch voneinander entkoppelt sind, vorzugsweise durch einen Luftspalt (17), ein thermisches Isoliermaterial (113'), und/oder ein Aerogel, das beispielsweise zwischen den Infrarotheizmodulen (9; 101, 101'; 333; 401; 501; 603) zumindest bereichsweise angeordnet ist, und/oder wobei zumindest eines der Infrarotheizmodule (9; 101, 101'; 333; 401; 501; 603) der Vorrichtung (1; 201; 301) eine Einheit (401; 501; 603) zum Zuführen von Wärme an eine Hautpartie (5) eines Lebewesens aufweist oder darstellt, wobei die Einheit (401; 501; 603) aufweist: ein Gehäuse (103; 303; 403; 503) und zumindest eine innerhalb des Gehäuses (103; 303; 403; 503) angeordnete Wärmequelle (105, 105'; 407; 507) in Form eines Infrarotstrahlers zur Abgabe von Wärme an die Umgebung.

7. Vorrichtung nach einem der vorangehenden Ansprüche, wobei wenigstens eines, vorzugsweise jedes, der drei oder mehr als drei Infrarotheizmodule (9; 101, 101'; 333; 401; 501; 603) umfasst: ein Gehäuse (103; 303; 403; 503), zumindest ein innerhalb des Gehäuses (103; 303; 403; 503) angeordnetes Infrarotstrahlerelement (105, 105'; 407; 507), zumindest ein Arretierungsmittel (109, 109') und ein mit dem Arretierungsmittel (109, 109') lösbar in und/oder an dem Gehäuse (103; 303; 403; 503) und/oder an dem Infrarotstrahlerelement (105, 105'; 407; 507) anordenbares Abstrahlelement (107, 107'; 409; 509),

wobei, optional:

beim Einsatz des Infrarotheizmoduls (9; 101, 101'; 333; 401; 501; 603) das Abstrahlelement (107, 107'; 409; 509) zumindest bereichsweise zwischen dem Infrarotstrahlerelement (105, 105'; 407; 507) und der Hautpartie (5) angeordnet ist, und/oder

das Arretierungsmittel (109, 109') in Form eines Schnellspannrahmens (123) ausgebildet ist und vorzugsweise der Schnellspannrahmen (123) mit dem Gehäuse (103; 303; 403; 503) verliersicher verbunden ist und/oder von dem Gehäuse (103; 303; 403; 503) bereitgestellt ist, und/oder

beim Einsatz des Infrarotheizmoduls (9; 101, 101'; 333; 401; 501; 603) das Abstrahlelement (107, 107'; 409; 509) und das Infrarotstrahlerelement (105, 105'; 407; 507) derart zueinander angeordnet sind, dass das Abstrahlelement (107, 107'; 409; 509) durch das Infrarotstrahlerelement (105, 105'; 407; 507) aufgewärmt wird und dann seinerseits Wärmestrahlung abgibt, insbesondere zumindest teilweise in Richtung der Hautpartie (5) beim Einsatz des Infrarotheizmoduls (9; 101, 101'; 333; 401; 501; 603), wobei vorzugsweise das Aufwärmen des Abstrahlelements (107, 107'; 409; 509) zumindest teilweise durch zumindest einen Teil der von dem Infrarotstrahlerelement (105, 105'; 407; 507) abgestrahlten Wärmestrahlung und/oder durch Konduktion von dem Infrarotstrahlerelement (105, 105'; 407; 507) auf das Abstrahlelement (107, 107'; 409; 509) erfolgt, und/oder

das Arretierungsmittel (109, 109') (a) von einer Entnahmestellung, in der vorzugsweise das Abstrahlelement (107, 107'; 409; 509) von dem Gehäuse (103; 303; 403; 503) lösbar ist, in eine Arretierstellung, in der vorzugsweise das Abstrahlelement (107, 107'; 409; 509) form-, reibund/oder kraftschlüssig an dem Gehäuse (103; 303; 403; 503) und/oder an dem Infrarotstrahlerelement (105, 105'; 407; 507) angeordnet ist und/oder mit dem Infrarotstrahlerelement (105, 105'; 407; 507) zumindest bereichsweise in Kontakt steht, und umgekehrt überführbar ist, und/oder (b) eine Anlagefläche aufweist, die mit einem Oberflächenbereich des Abstrahlelements (107, 107'; 409; 509) in Kontakt bringbar ist und wobei vorzugsweise das Infrarotheizmodul (9; 101, 101'; 333; 401; 501; 603) dazu ausgebildet ist, dass, wenn das Arretierungsmittel (109, 109') von der Entnahmestellung in die Arretierstellung überführt wird, das Abstrahlelement (107, 107'; 409; 509) durch die mit ihm in Kontakt stehende Anlagefläche gegen das Gehäuse (103; 303; 403; 503) und/oder das Infrarotstrahlerelement (105, 105'; 407; 507) gedrückt und dadurch das Abstrahlelement (107, 107'; 409; 509), während sich das Arretierungsmittel (109, 109') in der Arretierstellung befindet, form-, reib-und/oder kraftschlüssig an dem Gehäuse (103; 303; 403; 503) und/oder dem Infrarotstrahlerelement (105, 105'; 407; 507) gehalten und/oder in direkten oder indirekten Kontakt mit dem Infrarotstrahlerelement (105, 105'; 407; 507) gebracht wird.

8. Vorrichtung nach Anspruch 7, wobei (a) das Gehäuse (103; 303; 403; 503) eine Öffnung (405) mit einem, vorzugsweise ringförmigen, Randbereich aufweist, und vorzugsweise das Abstrahlelement (107, 107'; 409; 509) an dem Randbereich angeordnet ist, insbesondere wenn das Arretierungsmittel (109, 109') in der Arretierstellung ist, und/oder (b) das Infrarotheizmodul (9; 101, 101'; 333; 401; 501; 603) einen Berührschutz (117'; 331; 411) des Abstrahlelements (107, 107'; 409; 509) aufweist, wodurch ein unbeabsichtigtes Berühren des Abstrahlelements (107, 107'; 409; 509) verhinderbar ist, insbesondere der Berührschutz (117'; 331; 411) einstückig mit zumindest einem Teil des Arretierungsmittels (109, 109') und/oder des Gehäuses (103; 303; 403; 503) ausgebildet ist, und/oder wobei das Abstrahlelement (107, 107'; 409; 509) zumindest teilweise vor, innerhalb und/oder hinter der Öffnung (405) des Gehäuses (103; 303; 403; 503) angeordnet ist und/oder das Abstrahlelement (107, 107'; 409; 509) die Öffnung (405) zumindest teilweise verschließt, und/oder wobei das Abstrahlelement (107, 107'; 409; 509) zumindest bereichsweise einstückig mit dem Gehäuse (103; 303; 403; 503) ausgebildet ist, insbesondere das Abstrahlelement (107, 107'; 409; 509) einen Bereich eines ersten Gehäuseabschnitts aufweist oder darstellt, und/oder

wobei das Abstrahlelement (107, 107'; 409; 509) zumindest bereichsweise membranförmig und/oder flächig ausgebildet ist, und/oder
wobei das Abstrahlelement (107, 107'; 409; 509) als Material Keramik und/oder Carbon aufweist oder daraus besteht.

9. Vorrichtung nach einem der Ansprüche 1 bis 8, wobei die Vorrichtung (1; 201; 301) umfasst

einen Temperatursensor (119'; 417; 517), der dazu angepasst ist, die Temperatur von zumindest Teilen der beim Gebrauch der Vorrichtung (1; 201; 301) vor der Vorrichtung (1; 201; 301) befindlichen Hautpartie (5) zu messen, und

ein Steuermodul (421; 521), das dazu eingerichtet ist, die während des Gebrauchs der Vorrichtung (1; 201; 301) von dem Infrarotstrahlerelement (105, 105'; 407; 507) oder der Wärmequelle pro Zeiteinheit abgegebene Wärme nach Maßgabe der gemessenen Temperatur zu steuern,

wobei, optional, das Steuermodul (421; 521) dazu eingerichtet ist, das Infrarotstrahlerelement (105, 105'; 407; 507) oder die Wärmequelle (105, 105'; 407; 507) derart zu steuern, dass eine definierte oder definierbare maximal zulässige Hauttemperatur, zumindest lokal, nicht überschritten wird und/oder das Steuermodul (421; 521) dazu eingerichtet ist, das Infrarotstrahlerelement (105, 105'; 407; 507) oder die Wärmequelle (105, 105'; 407; 507) derart zu steuern, dass die von dem Infrarotstrahlerelement (105, 105'; 407; 507) oder der Wärmequelle (105, 105'; 407; 507) abgegebene Wärme pro Zeiteinheit so lange zunimmt, insbesondere mit einer definierten oder definierbaren Änderungsrate, bis, zumindest lokal, die maximal zulässige Hauttemperatur erreicht wird.

10. Vorrichtung nach einem der vorangehenden Ansprüche 7 bis 9, wobei die Vorrichtung (1; 201; 301) eine zumindest teilweise innerhalb des Gehäuses (103; 303; 403; 503) angeordnete Wärmeisolierung (413) aufweist, wobei die Wärmeisolierung (413) vorzugsweise ein Hochtemperaturaerogel aufweist oder daraus besteht, und/oder wobei das Gehäuse (103; 303; 403; 503) Abschnitte mit unterschiedlicher Wärmeleitfähigkeit aufweist und ein erster Gehäuseabschnitt eine, vorzugsweise mittlere, Wärmeleitfähigkeit aufweist, die höher ist, insbesondere 10 % oder mehr, vorzugsweise 30 % oder mehr, vorzugsweise 50 % oder mehr, vorzugsweise 100 % oder mehr, vorzugsweise 200 % oder mehr, als die Wärmeleitfähigkeit von wenigstens des zweiten Gehäuseabschnitts, vorzugsweise als die Wärmeleitfähigkeit aller übrigen Abschnitte.

11. Vorrichtung nach einem der Ansprüche 7 oder 8 in Kombination mit Anspruch 9, wobei der Temperatursensor (119'; 417; 517) ein Infrarottemperatursensor ist, innerhalb des Gehäuses (103; 303; 403; 503) angeordnet ist, und/oder zumindest teilweise, insbesondere aus Sicht der Hautpartie (5), hinter dem Abstrahlelement (107, 107'; 409; 509) angeordnet ist, und vorzugsweise das Abstrahlelement (107, 107'; 409; 509) zumindest bereichsweise durchdringt.

12. Vorrichtung nach Anspruch 9 oder 11, wobei der Temperatursensor (119'; 417; 517) ein Sichtbereich von 10° oder mehr, vorzugsweise von 30° oder mehr, vorzugsweise von 40° oder mehr, vorzugsweise von 50° oder mehr, vorzugsweise von 60° oder mehr, vorzugsweise von 60° oder mehr, vorzugsweise von 70° oder mehr, vorzugsweise von 80° oder mehr, vorzugsweise von 90° oder mehr, vorzugsweise von 100° oder mehr, vorzugsweise von 120° oder mehr, vorzugsweise von 150° oder mehr, aufweist und/oder der Temperatursensor (119'; 417; 517) beim Gebrauch der Einheit (401; 501; 603) einen Abstand von der Hautpartie (5) von 30 cm oder weniger, vorzugsweise von 20 cm oder weniger, vorzugsweise von 10 cm oder weniger, vorzugsweise von 5 cm oder weniger, vorzugsweise von 3 cm oder weniger, vorzugsweise von 1 cm oder weniger, aufweist, und/oder

wobei die Vorrichtung (1; 201; 301) zwei, drei, vier oder mehr als vier Temperatursensoren (119'; 417; 517) aufweist, und die Temperatursensoren (119'; 417; 517) derart angeordnet und/oder ausgewählt sind, dass jeweils zwei der Temperatursensoren (119'; 417; 517) die Temperatur von zumindest teilweise verschiedenen Bereichen der Hautpartie (5) eines Benutzers messen, und wobei vorzugsweise das Steuermodul (421; 521) dazu eingerichtet ist, die Wärmequelle (105, 105'; 407; 507) nach Maßgabe der höchsten von den Temperatursensoren (119'; 417; 517) gemessenen Temperatur zu steuern, und/oder wobei das Steuermodul (421; 521) dazu eingerichtet ist, das Infrarotstrahlerelement (105, 105'; 407; 507) oder die Wärmequelle (105, 105'; 407; 507) derart zu steuern, dass bei Benutzung der Einheit (401; 501; 603) die am Ort der im Abstand von 10 cm von dem Temperatursensor (119'; 417; 517) befindliche Hautpartie (5) einer Wärme-Intensität von 150 mW/cm2 oder weniger ausgesetzt wird, wobei vorzugsweise die Wärme-Intensität anhand der gemessenen Temperatur ermittelt wird oder werden kann, und/oder wobei das Steuermodul (421; 521) dazu eingerichtet ist, mittels einer externen Bedienvorrichtung, wie einem Smartphone, zu kommunizieren, insbesondere Befehle von dieser zu empfangen und Daten der Einheit (401; 501; 603) an die Bedienvorrichtung zu senden.

13. Vorrichtung nach einem der vorangehenden Ansprüche, wobei die Vorrichtung (1; 201; 301) zumindest einen Berührungsschutz (117'; 331; 411), insbesondere des Abstrahlelements (107, 107'; 409; 509), aufweist, der vorzugsweise (a) zumindest bereichsweise beabstandet zu einer beim Gebrauch der Vorrichtung (1; 201; 301) der Hautpartie (5) zugewandten Seite des Abstrahlelements (107, 107';

409; 509) vorgesehen ist und/oder (b) gleichzeitig eine Auflagefläche für die Hautpartie (5) bereitstellt oder ausbildet, und

wobei vorzugsweise der Berührungsschutz (117'; 331; 411) mit dem Gehäuse (103; 303; 403; 503) verbunden und/oder zumindest bereichsweise einstückig mit diesem ausgebildet ist und/oder der Berührungsschutz (117'; 331; 411) zumindest bereichsweise eine Wärmeleitfähigkeit von 1 W/(mK) oder weniger, vorzugsweise von 0,5 W/(mK) oder weniger, aufweist, und/oder

wobei die Vorrichtung (1; 201; 301) zumindest eine Auffang- und/oder Abführvorrichtung zum Auffangen und Abführen von von der Hautpartie (5) tropfenden Schweißes aufweist, und/oder wobei die Vorrichtung (1; 201; 301) einen Abstandshalter aufweist, der während der Benutzung die Hautpartie (5) beabstandet zu Teilen der übrigen Vorrichtung (1; 201; 301), insbesondere dem Berührungsschutz (117'; 331; 411) und/oder dem Abstrahlelement (107, 107'; 409; 509), hält, aufweist und/oder der gleichzeitig eine Auflagefläche für die Hautpartie (5) bereitstellt oder ausbildet, und wobei vorzugsweise der Abstandshalter mit dem Gehäuse (103; 303; 403; 503) verbunden und/oder zumindest bereichsweise einstückig mit diesem ausgebildet ist.

14. Vorrichtung nach einem der vorangehenden Ansprüche, wobei die Vorrichtung (1; 201; 301) zumindest ein Abstützelement (307) zum zumindest teilweisen Abstützen der Vorrichtung (1; 201; 301) an dem Lebewesen aufweist,

wobei, optional, das Abstützelement (307) eine spezifische Oberfläche (315) bereitstellt, die vorzugsweise zum Abstützen der Vorrichtung (1; 201; 301) an dem Lebewesen mit einer Kontaktoberfläche, insbesondere eines Rückenbereichs, des Lebewesens in Kontakt bringbar ist, wobei der Verlauf und/oder die Gestalt der spezifischen Oberfläche (315) zumindest abschnittsweise und/oder bereichsweise an den Verlauf und/oder die Gestalt der Kontaktoberfläche anpassbar ist, wobei, ferner optional, das Abstützelement (307) mit spezifischer Oberfläche (315) mehrere, vorzugsweise gegeneinander bewegbare, insbesondere in ihrer Position und/oder Lage veränderbare, und/oder, insbesondere durch Komprimierung, in ihrer Form und/oder Gestalt reversibel veränderbare, Einzelelemente (311) aufweist und jedes Einzelelement (311) mit einem Oberflächenbereich einen Teil der spezifischen Oberfläche (315) des Abstützelements (307) bereitstellt,
wobei, ferner optional, das Abstützelement (307) mit spezifischer Oberfläche (315) ein elastisches Grundelement (313), insbesondere bestehend aus oder aufweisend Silikon, aufweist, auf dem die Einzelelemente (311) angeordnet sind, und insbesondere stoffschlüssig mit dem elastischen Grundelement (313) verbunden sind, wobei vorzugsweise (i) die Einzelelemente (311), vorzugsweise nebeneinander und/oder flächig, auf dem elastischen Grundelement (313) angeordnet sind, (ii) das elastische Grundelement (313) schichtförmig ausgebildet ist, (iii) das elastische Grundelement (313), insbesondere unmittelbar, zwischen einerseits den Einzelelementen (311) und andererseits dem Gehäuse (103; 303; 403; 503) angeordnet ist, vorzugsweise das elastische Grundelement (313) direkt auf dem Gehäuse (103; 303; 403; 503) angeordnet ist, (iv) das elastische Grundelement (313) quaderförmig ist und/oder (v) das elastische Grundelement (313) eine Dicke von wenigstens 0,5 cm und/oder von maximal 10 cm aufweist.

15. Vorrichtung nach einem der vorangehenden Ansprüche, wobei die Vorrichtung (1; 201; 301), insbesondere an der Seitenpartie und/oder an der Frontpartie, zumindest ein, vorzugsweise pyramidenstumpfförmige oder parallelepipedförmige, Podestelement (305, 305') aufweist, vorzugsweise mehrere solcher Podestelemente (305, 305') aufweist, und wobei vorzugsweise die Podestelemente (305, 305') von dem Gehäuse (103; 303; 403; 503) ausgebildet werden, und/oder

wobei die Infrarotheizmodule (9; 101, 101'; 333; 401; 501; 603) jeweils ein lasergeschnittenes Heizelement oder ein Flächenheizelement aus Kunstglimmer aufweisen und/oder eine Abdeckung aus, insbesondere, eloxiertem, Aluminium aufweisen; und/oder wobei die Infrarotheizmodule (9; 101, 101'; 333; 401; 501; 603) jeweils zumindest ein Infrarotstrahlerelement in Form eines Heizdrahtes, einer Folienheizung, eines Heizgewebes, von Mikanit, einer Dickschichtheizung und/oder einer Silikonheizmatte, aufweist, vorzugsweise in Form eines Carbongewebes, und/oder wobei sich die Infrarotheizmodule (9; 101, 101'; 333; 401; 501; 603) entlang einer zur spezifischen Transversalebene (7) senkrecht verlaufenden Richtung streifenförmig erstrecken.

**Claims**

1. Device (1; 201; 301) for supplying heat to a skin area (5) of a living being, wherein the device (1; 201; 301) comprises three or more than three infrared heating modules (9; 101, 101'; 333; 401; 501; 603) which, when the device (1; 201; 301) is in use, are arranged relative to and/or at the living being in such a way that, for a defined or definable specific transverse plane (7) of the living being, each of the infrared heating modules (9; 101, 101'; 333; 401; 501; 603) has an intersection area with the specific transverse plane (7),

**characterized in that**

each infrared heating module (9; 101, 101'; 333; 401; 501; 603) is operable at an individual operating temperature, and
wherein, when the device (1; 201; 301) is in use, the operating temperatures of the infrared heating modules (9; 101, 101'; 333; 401; 501; 603) are set or can be set to at least partially differ in such a way that the intensity of the thermal radiation provided by the device (1; 201; 301) for delivery to the skin area (5) is homogeneous at least along a first and a second direction (A, U) respectively extending parallel to the specific transverse plane (7) within at least one region of interest (13) outside the device (1; 201; 301).

2. Device according to claim 1, wherein, when the device (1; 201; 301) is in use, (a) the first direction is parallel to a defined or definable sagittal plane of the living being and/or the first direction is a direction of distance (A) from the device (1; 201; 301) to the skin area, and preferably the region of interest (13) extends 1 cm or more and/or 30 cm or less along the first direction, and/or (b) the second direction is a circumferential direction (U) of the body part or body region (3) of the living being comprising the skin area (5), and/or the region of interest (13) extends 10 cm or more and/or 100 cm or less along the second direction.

3. Device according to any one of the preceding claims, wherein (a) the region of interest (13) is a volumetric area, preferably of at least 50 cm$^3$ and/or at most 30,000 cm$^3$, and/or the region of interest (13), when the device (1; 201; 301) is in use, is disposed at least partially between the device (1; 201; 301) and the skin area (5), and/or (b) the region of interest (13) extends at least partially laterally along at least two adjacent infrared heating modules (9; 101, 101'; 333; 401; 501; 603).

4. Device according to any one of the preceding claims, wherein, when the device (1; 201; 301) is in use, the operating temperature of the individual infrared heating modules (9; 101, 101'; 333; 401; 501; 603) is set or can be set to between 100°C and 400°C, preferably between 100°C and 300°C, preferably between 150°C and 300°C, preferably between 150°C and 250°C, or between 200°C and 300°C, and/or wherein, when the device (1; 201; 301) is in use, the operating temperature of the two lateral infrared heating modules (9; 101, 101'; 333; 401; 501; 603) preferably in the specific transversal plane, is respectively set or can be set to be (a) up to 50%, preferably up to 40%, preferably up to 30%, preferably up to 20%, preferably up to 10%, higher than the highest operating temperature of the remaining infrared heating modules (9; 101, 101'; 333; 401; 501; 603) and/or (b) is set or can be set to be 1% or more, preferably 5% or more, preferably 10% or more, higher than the highest operating temperature of the remaining infrared heating modules (9; 101, 101'; 333; 401; 501; 603).

5. Device according to any one of the preceding claims, wherein the device (1; 201; 301) comprises a control module (11) configured to monitor, in particular to regulate or control, the operating temperatures of the individual infrared heating modules (9; 101, 101'; 333; 401; 501; 603), preferably based on at least one operating mode selected or selectable by a user, wherein optionally:

(a) the control module (11) is configured to operate at least the two infrared heating modules (9; 101, 101'; 333; 401; 501; 603) disposed laterally, when the device (1; 201; 301) is in use, at the same operating temperature and/or to operate at least two of the infrared heating modules (9; 101, 101'; 333; 401; 501; 603) at different operating temperatures, and/or (b) the control module (11) is configured to operate the two infrared heating modules (9; 101, 101'; 333; 401; 501; 603) disposed laterally, when the device (1; 201; 301) is in use, at the same operating temperature, which is higher than any of the operating temperatures at which the remaining infrared heating modules (9; 101, 101'; 333; 401; 501; 603) are operated, and/or
the device (1; 201; 301) comprises one or more sensors (119'; 417; 517) that are configured to measure, respectively, a local temperature of the skin area (5), and wherein the control module (11) is configured to monitor, in particular to regulate or control, the operating temperatures of the individual infrared heating modules (9; 101; 101'; 333; 401; 501; 603) at least based on the measured temperature values, and wherein the sensors (119'; 417; 517) are preferably arranged in a matrix configuration.

6. Device according to any one of the preceding claims, wherein (a) at least two of the infrared heating modules (9; 101, 101'; 333; 401; 501; 603) are movable relative to one another, in particular in a direction that, when the device (1; 201; 301) is in use, extends parallel to the specific transverse plane (7), (b) at least two, preferably three or more than three, of the infrared heating modules (9; 101, 101'; 333; 401; 501; 603) can be tilted relative to one another and/or wherein at least two, preferably three or more than three, of the infrared heating modules (9; 101, 101'; 333; 401; 501; 603) can be angled relative to one another, and/or (c) at least two adjacent infrared heating modules (9; 101, 101'; 333; 401; 501; 603), preferably all pairs of adjacent infrared heating modules (9; 101, 101'; 333; 401; 501; 603), are thermally decoupled from one another, preferably by an air gap (17), a thermal insulation material (113') and/or an aerogel, which is arranged, for example, between the infrared heating modules (9; 101, 101'; 333; 401; 501; 603) at least in some areas, and/or wherein at least one of the infrared heating modules (9; 101, 101'; 333; 401; 501; 603) of the device (1; 201; 301) comprises or represents a unit (401; 501; 603) for supplying heat to a skin area (5) of a living being, wherein the unit (401; 501; 603) comprises: a housing (103; 303; 403; 503) and at least one heat source (105, 105'; 407; 507) arranged within the housing (103; 303; 403; 503) in the form of an infrared emitter for emitting heat into the environment.

7. Device according to any one of the preceding claims, wherein at least one, preferably each, of the three or more than three infrared heating modules (9; 101, 101'; 333; 401; 501; 603) comprises: a housing (103; 303; 403; 503), at least one infrared emitter element (105, 105'; 407; 507) disposed within the housing (103; 303; 403; 503), at least one locking means (109, 109'), and a radiation element (107, 107'; 409; 509) that can be detachably arranged with the locking means (109, 109') in and/or at the housing (103; 303; 403; 503) and/or at the infrared emitter element (105, 105'; 407; 507), wherein, optionally:

   when the infrared heating module (9; 101, 101'; 333; 401; 501; 603) is in use, the radiation element (107, 107'; 409; 509) is arranged at least in some areas between the infrared emitter element (105, 105'; 407; 507) and the skin area (5), and/or
   the locking means (109, 109') is designed as a quick-release frame (123) and, preferably, the quick-release frame (123) is connected loss-proof to the housing (103; 303; 403; 503) and/or is provided by the housing (103; 303; 403; 503), and/or
   when the infrared heating module (9; 101, 101'; 333; 401; 501; 603) is in use, the radiation element (107, 107'; 409; 509) and the infrared emitter element (105, 105'; 407; 507) are arranged relative to one another in such a way that the radiation element (107, 107'; 409; 509) is heated by the infrared emitter element (105, 105'; 407; 507) and then emits thermal radiation itself, in particular at least partially toward the skin area (5) when the infrared heating module (9; 101, 101'; 333; 401; 501; 603) is in use, wherein the heating of the radiation element (107, 107'; 409; 509) is preferably at least partially effected by at least a portion of the thermal radiation emitted by the infrared emitter element (105, 105'; 407; 507) and/or by conduction from the infrared emitter element (105, 105'; 407; 507) to the radiation element (107, 107'; 409; 509), and/or
   the locking means (109, 109') can be transferred (a) from a removal position, in which preferably the radiation element (107, 107'; 409; 509) is detachable from the housing (103; 303; 403; 503), to a locking position in which the radiation element (107, 107'; 409; 509) is preferably arranged at the housing (103; 303; 403; 503) and/or the infrared emitter element (105, 105'; 407; 507) in a form-fitting or frictionally engaged or force-fitting manner and/or is in contact with the infrared emitter element (105, 105'; 407; 507) at least in some areas, and vice versa, and/or (b) comprises an abutment surface that can be brought into contact with a surface area of the radiation element (107, 107'; 409; 509), and wherein preferably the infrared heating module (9; 101, 101'; 333; 401; 501; 603) is configured such that, when the locking means (109, 109') is transferred from the removal position to the locking position, the radiation element (107, 107'; 409; 509) is pressed against the housing (103; 303; 403; 503) and/or the infrared emitter element (105, 105'; 407; 507) by the abutment surface brought into contact with the radiation element, thereby retaining the radiation element (107, 107'; 409; 509) in a form-fitting, frictionally engaged and/or force-fitting manner against the housing (103; 303; 403; 503) and/or the infrared emitter element (105, 105'; 407; 507) and/or brought into direct or indirect contact with the infrared emitter element (105, 105'; 407; 507), while the locking means (109, 109') is in the locking position.

8. Device according to claim 7, wherein (a) the housing (103; 303; 403; 503) comprises an opening (405) with a, preferably annular, edge region, and preferably the radiation element (107, 107'; 409; 509) is arranged at the edge region, in particular when the locking means (109, 109') is in the locking position, and/or (b) the infrared heating module (9; 101, 101'; 333; 401; 501; 603) comprises a touch guard (117'; 331; 411) for the radiation element (107, 107'; 409;

509), whereby accidental contact with the radiation element (107, 107'; 409; 509) can be prevented, in particular the touch guard (1 17'; 331; 411) is formed integrally with at least a portion of the locking means (109, 109') and/or the housing (103; 303; 403; 503), and/or

wherein the radiation element (107, 107'; 409; 509) is arranged at least partially in front of, within, and/or behind the opening (405) of the housing (103; 303; 403; 503) and/or the radiation element (107, 107'; 409; 509) at least partially closes the opening (405), and/or wherein the radiation element (107, 107'; 409; 509) is formed integrally with the housing (103; 303; 403; 503) at least in some areas, in particular the radiation element (107, 107'; 409; 509) comprises or represents a region of a first housing section, and/or
wherein the radiation element (107, 107'; 409; 509) is formed at least in some areas as a membrane and/or as a flat surface, and/or
wherein the radiation element (107, 107'; 409; 509) comprises or consists of ceramic and/or carbon as a material.

9. Device according to any one of claims 1 to 8, wherein the device (1; 201; 301) comprises:

a temperature sensor (119'; 417; 517) adapted to measure the temperature of at least portions of the skin area (5) located in front of the device (1; 201; 301) during use of the device (1; 201; 301), and
a control module (421; 521) configured to control the heat emitted by the infrared emitter element (105, 105'; 407; 507) or the heat source per unit of time during use of the device (1; 201; 301) in accordance with the measured temperature,
wherein, optionally, the control module (421; 521) is configured to control the infrared emitter element (105, 105'; 407; 507) or the heat source (105, 105'; 407; 507) in such a way that a defined or definable maximum permissible skin temperature is not exceeded, at least locally, and/or the control module (421; 521) is configured to control the infrared emitter element (105, 105'; 407; 507) or the heat source (105, 105'; 407; 507) in such a way that the heat emitted by the infrared emitter element (105, 105'; 407; 507) or the heat source (105, 105'; 407; 507) per unit of time increases in particular at a defined or definable rate of change, until, at least locally, the maximum permissible skin temperature is reached.

10. Device according to any one of the preceding claims 7 to 9, wherein the device (1; 201; 301) comprises a thermal insulation (413) arranged at least partially within the housing (103; 303; 403; 503), wherein the thermal insulation (413) preferably comprises or consists of a high-temperature aerogel, and/or wherein the housing (103; 303; 403; 503) comprises portions with different thermal conductivities, and a first housing portion has a, preferably average, thermal conductivity that is higher, in particular by 10% or more, preferably by 30% or more, preferably 50% or more, preferably 100% or more, preferably 200% or more, than the thermal conductivity of at least the second housing portion, preferably than the thermal conductivity of all remaining portions.

11. Device according to any one of claims 7 or 8 in combination with claim 9, wherein the temperature sensor (119'; 417; 517) is an infrared temperature sensor, is arranged within the housing (103; 303; 403; 503), and/or is arranged at least partially, in particular as viewed from the skin area (5), behind the radiation element (107, 107'; 409; 509), and preferably penetrates the radiation element (107, 107'; 409; 509) at least in some areas.

12. Device according to claim 9 or 11, wherein the temperature sensor (119'; 417; 517) has a viewing angle of 10° or more, preferably 30° or more, preferably 40° or more, preferably 50° or more, preferably 60° or more, preferably 60° or more, preferably 70° or more, preferably of 80° or more, preferably of 90° or more, preferably of 100° or more, preferably of 120° or more, preferably of 150° or more, and/or the temperature sensor (1 19'; 417; 517) has, when the unit (401; 501; 603) is in use, a distance from the skin area (5) of 30 cm or less, preferably 20 cm or less, preferably 10 cm or less, preferably 5 cm or less, preferably 3 cm or less, preferably 1 cm or less, and/or

wherein the device (1; 201; 301) comprises two, three, four, or more than four temperature sensors (119'; 417; 517), and the temperature sensors (119'; 417; 517) are arranged and/or selected such that in two of the temperature sensors (119'; 417; 517) respectively measure the temperature of at least partially different areas of the skin (5) of a user, and wherein preferably the control module (421; 521) is configured to control the heat source (105, 105'; 407; 507) in accordance with the highest temperature measured by the temperature sensors (119'; 417; 517), and/or
wherein the control module (421; 521) is configured to control the infrared emitter element (105, 105'; 407; 507) or the heat source (105, 105'; 407; 507) in such a way that, when the unit (401; 501; 603) is in use, the skin area (5) located at a distance of 10 cm from the temperature sensor (119'; 417; 517) is exposed to a heat intensity of 150 mW/cm² or less, wherein the heat intensity is preferably determined or can be determined based on the measured temperature, and/or

wherein the control module (421; 521) is configured to communicate via an external operating device, such as a smartphone, in particular to receive commands from it and to send data of the unit (401; 501; 603) to the operating device.

13. Device according to any one of the preceding claims, wherein the device (1; 201; 301) comprises at least one touch guard (117'; 331; 411), in particular for the radiation element (107, 107'; 409; 509), which is preferably (a) provided at least in some areas at a distance from a side of the radiating element (107, 107'; 409; 509) facing the skin area (5) during use of the device (1; 201; 301) and/or (b) simultaneously provides or forms a support surface for the skin area (5), and

wherein the contact guard (117'; 331; 411) is preferably connected to the housing (103; 303; 403; 503) and/or is formed integrally with it at least in some areas, and/or the touch guard (117'; 331; 411) has a thermal conductivity of 1 W/(mK) or less, preferably 0.5 W/(mK) or less, at least in some areas, and/or

wherein the device (1; 201; 301) comprises at least one collection and/or drainage device for collecting and draining sweat dripping from the skin area (5), and/or

wherein the device (1; 201; 301) comprises a spacer which, during use, keeps the skin area (5) spaced apart from parts of the rest of the device (1; 201; 301), in particular the touch guard (117'; 331; 411) and/or the radiation element (107, 107'; 409; 509), and/or which simultaneously provides or forms a support surface for the skin area (5), and wherein the spacer is preferably connected to the housing (103; 303; 403; 503) and/or is formed integrally with it at least in some regions.

14. Device according to any one of the preceding claims, wherein the device (1; 201; 301) comprises at least one support element (307) for at least partially supporting the device (1; 201; 301) at the living being,

wherein, optionally, the support element (307) provides a specific surface (315) which can be preferably brought into contact with a contact surface, in particular a back region, of the living being for supporting the device (1; 201; 301) at the living being, wherein the contour and/or shape of the specific surface (315) is adaptable, at least in sections and/or regions, to the contour and/or shape of the contact surface,

wherein, further optionally, the support element (307) comprising a specific surface (315) comprises a plurality of individual elements (311) that are preferably movable relative to one another and are in particular variable in their position and/or orientation, and/or, are reversibly variable in their shape and/or configuration in particular by compression, and wherein each individual element (311) provides, with a surface area, a part of the specific surface (315) of the support element (307),

wherein, further optionally, the support element (307) comprising a specific surface (315) comprises an elastic base element (313), in particular consisting of or comprising silicone, on which the individual elements (311) are arranged, and are in particular connected to the elastic base element (313) by material bonding, wherein preferably (i) the individual elements (311) are arranged, preferably side by side and/or in a planar manner, on the elastic base element (313), (ii) the elastic base element (313) is formed in a layered configuration, (iii) the elastic base element (313) is arranged, in particular directly, between the individual elements (311) on the one hand and the housing (103; 303; 403; 503) on the other hand, preferably the elastic base element (313) is arranged directly on the housing (103; 303; 403; 503), (iv) the elastic base element (313) is cuboid in shape, and/or (v) the elastic base element (313) has a thickness of at least 0.5 cm and/or at most 10 cm.

15. Device according to any one of the preceding claims, wherein the device (1; 201; 301), in particular at the side portion and/or at the front portion, comprises at least one, preferably truncated pyramid-shaped or parallelepiped-shaped, platform element (305, 305'), preferably several such platform elements (305, 305'), and wherein preferably the platform elements (305, 305') are formed from the housing (103; 303; 403; 503), and/or

wherein the infrared heating modules (9; 101, 101'; 333; 401; 501; 603) each comprise a laser-cut heating element or a surface heating element made of synthetic mica and/or a cover made of, in particular, anodized aluminum, and/or

wherein the infrared heating modules (9; 101, 101'; 333; 401; 501; 603) each comprise at least one infrared emitter element in the form of a heating wire, a foil heater, a heating fabric, micanite, a thick-film heater, and/or a silicone heating mat, preferably in the form of a carbon fabric, and/or

wherein the infrared heating modules (9; 101,

101', 333; 401; 501; 603) extend in a strip-like manner along a direction perpendicular to the specific transverse plane (7).

**Revendications**

1. Dispositif (1; 201; 301) pour l'apport de chaleur à une partie de la peau (5) d'un être vivant, le dispositif (1; 201; 301) présentant trois ou plus de trois modules de chauffage à infrarouge (9; 101, 101'; 333; 401; 501; 603) qui, lors de l'utilisation du dispositif (1; 201; 301), sont disposés par rapport à et/ou sur l'être vivant de telle sorte que, pour un plan transversal spécifique (7) défini ou définissable de l'être vivant, chacun des modules de chauffage à infrarouge (9; 101, 101'; 333; 401; 501; 603) présente une surface d'intersection avec le plan transversal spécifique (7),

   caractérisé en ce que chaque module de chauffage à infrarouge (9; 101, 101'; 333; 401; 501; 603) peut fonctionner à une température de fonctionnement individuelle, et
   en ce que, lors de l'utilisation du dispositif (1; 201; 301), les températures de fonctionnement des modules de chauffage à infrarouge (9; 101, 101'; 333; 401; 501; 603) sont réglées ou réglables de manière au moins partiellement différente de sorte que l'intensité du rayonnement thermique fourni par le dispositif (1; 201; 301) pour l'apport à la partie de la peau (5) soit homogène au moins le long d'une première et d'une deuxième direction (A, U) s'étendant chacune parallèlement au plan transversal spécifique (7) à l'intérieur d'au moins une zone considérée (13) à l'extérieur du dispositif (1; 201; 301).

2. Dispositif selon la revendication 1, dans lequel, lors de l'utilisation du dispositif (1; 201; 301) : (a) la première direction est parallèle à un plan sagittal défini ou définissable de l'être vivant et/ou la première direction est une direction de distance (A) allant du dispositif (1; 201; 301) vers la partie de la peau, et de préférence la zone considérée (13) s'étend sur 1 cm ou plus et/ou 30 cm ou moins le long de la première direction, et/ou (b) la deuxième direction est une direction circonférentielle (U) de la partie du corps ou de la région du corps (3) de l'être vivant présentant la partie de la peau (5) et/ou la zone considérée (13) s'étend sur 10 cm ou plus et/ou 100 cm ou moins le long de la deuxième direction.

3. Dispositif selon l'une des revendications précédentes, dans lequel : (a) la zone considérée (13) est une zone volumique, de préférence d'au moins 50 cm$^3$ et/ou d'au plus 30 000 cm$^3$, et/ou la zone considérée (13) se situe au moins partiellement entre le dispositif (1; 201; 301) et la partie de la peau (5) lors de l'utilisation du dispositif (1; 201; 301), et/ou (b) la zone considérée (13) s'étend latéralement, au moins partiellement, le long d'au moins deux modules de chauffage à infrarouge (9; 101, 101'; 333; 401; 501; 603) adjacents.

4. Dispositif selon l'une des revendications précédentes, dans lequel, lors de l'utilisation du dispositif (1; 201; 301), la température de fonctionnement des différents modules de chauffage à infrarouge (9; 101, 101'; 333; 401; 501; 603) est respectivement réglée ou réglable entre 100 °C et 400 °C, de préférence entre 100 °C et 300 °C, de préférence entre 150 °C et 300 °C, de préférence entre 150 °C et 250 °C ou entre 200 °C et 300 °C, et/ou dans lequel, lors de l'utilisation du dispositif (1; 201; 301), la température de fonctionnement des deux modules de chauffage à infrarouge latéraux (9; 101, 101'; 333; 401; 501; 603), de préférence dans le plan transversal spécifique (7), est réglée ou réglable pour être respectivement : (a) jusqu'à 50 %, de préférence jusqu'à 40 %, de préférence jusqu'à 30 %, de préférence jusqu'à 20 %, de préférence jusqu'à 10 %, supérieure à la température de fonctionnement la plus élevée des autres modules de chauffage à infrarouge (9; 101, 101'; 333; 401; 501; 603) et/ou (b) 1 % ou plus, de préférence 5 % ou plus, de préférence 10 % ou plus, supérieure à la température de fonctionnement la plus élevée des autres modules de chauffage à infrarouge (9; 101, 101'; 333; 401; 501; 603).

5. Dispositif selon l'une des revendications précédentes, dans lequel le dispositif (1; 201; 301) présente un module de contrôle (11) qui est configuré pour contrôler, en particulier pour réguler ou commander, les températures de fonctionnement des différents modules de chauffage à infrarouge (9; 101, 101'; 333; 401; 501; 603), de préférence sur la base d'au moins un mode de fonctionnement sélectionné ou sélectionnable par un utilisateur, dans lequel, optionnellement :

   (a) le module de contrôle (11) est configuré pour faire fonctionner au moins les deux modules de chauffage à infrarouge latéraux (9; 101, 101'; 333; 401; 501; 603) lors de l'utilisation du dispositif (1; 201; 301) à une même température de fonctionnement et/ou pour faire fonctionner au moins deux des modules de chauffage à infrarouge (9; 101, 101'; 333; 401; 501; 603) à une température de fonctionnement différente, et/ou (b) le module de contrôle (11) est configuré pour faire fonctionner les deux modules de chauffage à infrarouge latéraux (9; 101, 101'; 333; 401; 501; 603) lors de l'utilisation du dispositif (1; 201; 301) à une même température de fonctionnement qui est supérieure à chacune des températures de fonctionnement auxquelles les autres modules de chauffage à infrarouge (9; 101, 101'; 333; 401; 501; 603) sont exploités, et/ou le dispositif (1; 201; 301) présente un ou plusieurs capteurs (119' ; 417 ; 517) qui sont configurés pour mesurer respectivement une température locale de la partie de la peau (5) et le module de contrôle (11) est configuré pour contrôler, en particulier pour réguler ou commander, les températures de fonctionnement des différents modules de chauffage à infrarouge (9; 101, 101'; 333; 401; 501; 603) au moins sur la base des valeurs de température mesurées, et dans lequel de préférence les capteurs (119'; 417; 517) sont disposés sous forme de matrice.

6. Dispositif selon l'une des revendications précédentes, dans lequel : (a) au moins deux des modules de chauffage à infrarouge (9; 101, 101'; 333; 401; 501; 603) sont mobiles l'un par rapport à l'autre par coulissement, en particulier dans une direction qui s'étend parallèlement au plan transversal spécifique (7) lors de l'utilisation du dispositif (1; 201; 301), (b) au moins deux, de préférence trois ou plus de trois, des modules de chauffage à infrarouge (9; 101, 101'; 333; 401; 501; 603) sont inclinables l'un par rapport à l'autre et/ou dans lequel au moins deux, de préférence trois ou plus de trois, des modules de chauffage à infrarouge (9; 101, 101'; 333; 401; 501; 603) sont articulables l'un par rapport à l'autre, et/ou (c) au moins deux modules de chauffage à infrarouge (9; 101, 101'; 333; 401; 501; 603) adjacents, de préférence toutes les paires de modules de chauffage à infrarouge (9; 101, 101'; 333; 401; 501; 603) adjacents, sont découplés thermiquement l'un de l'autre, de préférence par un entrefer (17), un matériau d'isolation thermique (113'), et/ou un aérogel, qui est disposé par exemple au moins par zones entre les modules de chauffage à infrarouge (9; 101, 101'; 333; 401; 501; 603), et/ou dans lequel au moins l'un des modules de chauffage à infrarouge (9; 101, 101'; 333; 401; 501; 603) du dispositif (1; 201; 301) présente ou constitue une unité (401; 501; 603) pour l'apport de chaleur à une partie de la peau (5) d'un être vivant, l'unité (401; 501; 603) comprenant : un boîtier (103; 303; 403; 503) et au moins une source de chaleur (105, 105'; 407; 507) disposée à l'intérieur du boîtier (103; 303; 403; 503) sous la forme d'un radiateur à infrarouge pour l'émission de chaleur vers l'environnement.

7. Dispositif selon l'une des revendications précédentes, dans lequel au moins l'un, de préférence chacun, des trois ou plus de trois modules de chauffage à infrarouge (9; 101, 101'; 333; 401; 501; 603) comprend : un boîtier (103; 303; 403; 503), au moins un élément radiateur à infrarouge (105, 105'; 407; 507) disposé à l'intérieur du boîtier (103; 303; 403; 503), au moins un moyen d'arrêt (109, 109') et un élément de rayonnement (107, 107'; 409; 509) pouvant être disposé de manière amovible avec le moyen d'arrêt (109, 109') dans et/ou sur le boîtier (103; 303; 403; 503) et/ou sur l'élément radiateur à infrarouge (105, 105'; 407; 507), dans lequel, optionnellement :

lors de l'utilisation du module de chauffage à infrarouge (9; 101, 101'; 333; 401; 501; 603), l'élément de rayonnement (107, 107'; 409; 509) est disposé au moins par zones entre l'élément radiateur à infrarouge (105, 105'; 407; 507) et la partie de la peau (5), et/ou
le moyen d'arrêt (109, 109') est réalisé sous la forme d'un cadre de serrage rapide (123) et de préférence le cadre de serrage rapide (123) est relié de manière imperdable au boîtier (103; 303; 403; 503) et/ou est fourni par le boîtier (103; 303; 403; 503), et/ou
lors de l'utilisation du module de chauffage à infrarouge (9; 101, 101'; 333; 401; 501; 603), l'élément de rayonnement (107, 107'; 409; 509) et l'élément radiateur à infrarouge (105, 105'; 407; 507) sont disposés l'un par rapport à l'autre de telle sorte que l'élément de rayonnement (107, 107'; 409; 509) est chauffé par l'élément radiateur à infrarouge (105, 105'; 407; 507) et émet ensuite lui-même un rayonnement thermique, en particulier au moins partiellement en direction de la partie de la peau (5) lors de l'utilisation du module de chauffage à infrarouge (9; 101, 101'; 333; 401; 501; 603), le chauffage de l'élément de rayonnement (107, 107'; 409; 509) s'effectuant de préférence au moins partiellement par au moins une partie du rayonnement thermique émis par l'élément radiateur à infrarouge (105, 105'; 407; 507) et/ou par conduction de l'élément radiateur à infrarouge (105, 105'; 407; 507) vers l'élément de rayonnement (107, 107'; 409; 509), et/ou
le moyen d'arrêt (109, 109') : (a) peut passer d'une position de retrait, dans laquelle l'élément de rayonnement (107, 107'; 409; 509) peut de préférence être détaché du boîtier (103; 303; 403; 503), à une position d'arrêt, dans laquelle l'élément de rayonnement (107, 107'; 409; 509) est de préférence disposé par complémentarité de forme, par friction et/ou par force sur le boîtier (103; 303; 403; 503) et/ou sur l'élément radiateur à infrarouge (105, 105'; 407; 507) et/ou est en contact au moins par zones avec l'élément radiateur à infrarouge (105, 105'; 407; 507), et inversement, et/ou (b) présente une surface d'appui qui peut être mise en contact avec une zone de surface de l'élément de rayonnement (107, 107'; 409; 509) et dans lequel de préférence le module de chauffage à infrarouge (9; 101, 101'; 333; 401; 501; 603) est configuré de telle sorte que, lorsque le moyen d'arrêt (109, 109') passe de la position de retrait à la position d'arrêt, l'élément de rayonnement (107, 107'; 409; 509) est pressé contre le boîtier (103; 303; 403; 503) et/ou l'élément radiateur à infrarouge (105, 105' ; 407 ; 507) par la surface d'appui en contact avec lui et ainsi l'élément de rayonnement (107, 107'; 409; 509) est maintenu par complémentarité de forme, par friction et/ou par force sur le boîtier (103; 303; 403; 503) et/ou sur l'élément radiateur à infrarouge (105, 105' ; 407; 507) et/ou est mis en contact direct ou indirect avec l'élément radiateur à infrarouge (105, 105'; 407; 507) pendant que le moyen d'arrêt (109, 109') se trouve en position d'arrêt.

8. Dispositif selon la revendication 7, dans lequel :

> (a) le boîtier (103; 303; 403; 503) présente une ouverture (405) avec une zone de bord, de préférence annulaire, et de préférence l'élément de rayonnement (107, 107'; 409; 509) est disposé sur ladite zone de bord, en particulier lorsque le moyen d'arrêt (109, 109') est en position d'arrêt, et/ou (b) le module de chauffage à infrarouge (9; 101, 101'; 333; 401; 501; 603) présente une protection contre le contact (117'; 331; 411) avec l'élément de rayonnement (107, 107'; 409; 509), permettant d'empêcher tout contact involontaire avec ledit élément de rayonnement (107, 107' ; 409 ; 509), ladite protection contre le contact (117; 331; 411) étant en particulier réalisée d'un seul tenant avec au moins une partie du moyen d'arrêt (109, 109') et/ou du boîtier (103; 303; 403; 503), et/ou dans lequel l'élément de rayonnement (107, 107'; 409; 509) est disposé au moins partiellement devant, à l'intérieur et/ou derrière l'ouverture (405) du boîtier (103; 303; 403; 503) et/ou l'élément de rayonnement (107, 107'; 409; 509) ferme au moins partiellement l'ouverture (405), et/ou

>> dans lequel l'élément de rayonnement (107, 107'; 409; 509) est réalisé au moins par zones d'un seul tenant avec le boîtier (103; 303; 403; 503), en particulier l'élément de rayonnement (107, 107'; 409; 509) présente ou constitue une zone d'une première section de boîtier, et/ou
>> dans lequel l'élément de rayonnement (107, 107'; 409; 509) est réalisé au moins par zones sous forme membranaire et/ou plane, et/ou
>> dans lequel l'élément de rayonnement (107, 107'; 409; 509) comporte de la céramique et/ou du carbone comme matériau constitutif ou en est constitué.

9. Dispositif selon l'une des revendications 1 à 8, le dispositif (1 ; 201 ; 301) comprenant :

> un capteur de température (119'; 417; 517) qui est adapté pour mesurer la température d'au moins des parties de la partie de la peau (5) située devant le dispositif (1; 201; 301) lors de l'utilisation du dispositif (1; 201; 301), et
> un module de commande (421; 521) qui est configuré pour commander la chaleur émise par unité de temps par l'élément radiateur à infrarouge (105, 105'; 407; 507) ou par la source de chaleur lors de l'utilisation du dispositif (1; 201; 301) en fonction de la température mesurée, dans lequel, optionnellement, le module de commande (421; 521) est configuré pour commander l'élément radiateur à infrarouge (105, 105'; 407; 507) ou la source de chaleur (105, 105'; 407; 507) de telle sorte qu'une température cutanée maximale admissible définie ou définissable ne soit pas dépassée, au moins localement, et/ou le module de commande (421; 521) est configuré pour commander l'élément radiateur à infrarouge (105, 105'; 407; 507) ou la source de chaleur (105, 105'; 407; 507) de telle sorte que la chaleur émise par unité de temps par l'élément radiateur à infrarouge (105, 105'; 407; 507) ou par la source de chaleur (105, 105'; 407; 507) augmente, en particulier avec un taux de variation défini ou
> définissable, jusqu'à ce que, au moins localement, la température cutanée maximale admissible soit atteinte.

10. Dispositif selon l'une des revendications précédentes 7 à 9, le dispositif (1 ; 201 ; 301) présentant une isolation thermique (413) disposée au moins partiellement à l'intérieur du boîtier (103 ; 303 ; 403 ; 503), l'isolation thermique (413) comportant de préférence un aérogel à haute température ou en étant constituée, et/ou dans lequel le boîtier (103 ; 303 ; 403 ; 503) présente des sections ayant une conductivité thermique différente et une première section de boîtier présente une conductivité thermique, de préférence moyenne, qui est supérieure, en particulier de 10 % ou plus, de préférence de 30 % ou plus, de préférence de 50 % ou plus, de préférence de 100 % ou plus, de préférence de 200 % ou plus, à la conductivité thermique d'au moins la deuxième section de boîtier, de préférence à la conductivité thermique de toutes les autres sections.

11. Dispositif selon l'une des revendications 7 ou 8 en combinaison avec la revendication 9, dans lequel le capteur de température (119'; 417; 517) est un capteur de température à infrarouge, est disposé à l'intérieur du boîtier (103; 303; 403; 503), et/ou est disposé au moins partiellement, en particulier du point de vue de la partie de la peau (5), derrière l'élément de rayonnement (107, 107'; 409; 509), et traverse de préférence l'élément de rayonnement (107, 107'; 409; 509) au moins par zones.

12. Dispositif selon la revendication 9 ou 11, dans lequel le capteur de température (119'; 417; 517) présente un champ de vision de 10° ou plus, de préférence de 30° ou plus, de préférence de 40° ou plus, de préférence de 50° ou plus, de préférence de 60° ou plus, de préférence de 60° ou plus, de préférence de 70° ou plus, de préférence de 80° ou plus, de préférence de 90° ou plus, de préférence de 100° ou plus, de préférence de 120° ou plus, de préférence de 150° ou plus, et/ou le capteur de température (119'; 417; 517) présente, lors de l'utilisation de l'unité (401; 501; 603), une distance par rapport à la partie de la peau (5) de 30 cm ou moins, de préférence de 20 cm ou moins, de préférence de 10 cm ou moins, de préférence de 5 cm ou moins, de préférence de 3 cm ou moins, de préférence de 1 cm ou moins, et/ou dans lequel le dispositif (1; 201; 301) présente deux, trois, quatre ou plus de quatre capteurs de température (119'; 417; 517), et les capteurs de température (119'; 417; 517) sont disposés et/ou sélectionnés de telle sorte que deux capteurs de température (119'; 417; 517) mesurent respectivement la température de zones au moins partiellement différentes de la partie de la peau (5) d'un utilisateur, et dans lequel de préférence le module de commande (421; 521) est configuré pour commander la source de chaleur (105, 105'; 407; 507) en fonction de la température la plus élevée mesurée par les capteurs de température (119'; 417; 517), et/ou dans lequel le module de commande (421; 521) est configuré pour commander l'élément radiateur à infrarouge (105, 105'; 407; 507) ou la source de chaleur (105, 105'; 407; 507) de telle sorte que, lors de l'utilisation de l'unité (401; 501; 603), la partie de la peau (5) située à une distance de 10 cm du capteur de température (119'; 417; 517) soit exposée à une intensité thermique de 150 mW/cm$^2$ ou moins, l'intensité thermique étant ou pouvant être de préférence déterminée sur la base de la température mesurée, et/ou dans lequel le module de commande (421; 521) est configuré pour communiquer au moyen d'un dispositif de commande externe, tel qu'un smartphone, en particulier pour recevoir des commandes de celui-ci et envoyer des données de l'unité (401; 501; 603) vers le dispositif de commande.

13. Dispositif selon l'une des revendications précédentes, le dispositif (1; 201; 301) présentant au moins une protection contre le contact involontaire (117'; 331; 411), en particulier de l'élément de rayonnement (107, 107'; 409; 509), qui de préférence :

(a) est prévue au moins par zones à distance d'un côté de l'élément de rayonnement (107, 107'; 409; 509) tourné vers la partie de la peau (5) lors de l'utilisation du dispositif (1; 201; 301) et/ou (b) fournit ou constitue simultanément une surface d'appui pour la partie de la peau (5), et

dans lequel de préférence la protection contre le contact (117'; 331; 411) est reliée au boîtier (103; 303; 403; 503) et/ou est réalisée au moins par zones d'un seul tenant avec celui-ci et/ou la protection contre le contact (117'; 331; 411) présente au moins par zones une conductivité thermique de 1 W/(mK) ou moins, de préférence de 0,5 W/(mK) ou moins, et/ou le dispositif (1; 201; 301) présentant au moins un dispositif de collecte et/ou d'évacuation destiné à collecter et évacuer la sueur s'égouttant de la partie de la peau (5), et/ou dans lequel le dispositif (1; 201; 301) présente un écarteur qui, pendant l'utilisation, maintient la partie de la peau (5) à distance de parties du reste du dispositif (1; 201; 301), en particulier de la protection contre le contact (117'; 331; 411) et/ou de l'élément de rayonnement (107, 107'; 409; 509), et/ou qui fournit ou constitue simultanément une surface d'appui pour la partie de la peau (5), et dans lequel de préférence l'écarteur est relié au boîtier (103; 303; 403; 503) et/ou est réalisé au moins par zones d'un seul tenant avec celui-ci.

14. Dispositif selon l'une des revendications précédentes, le dispositif (1; 201; 301) présentant au moins un élément d'appui (307) destiné à appuyer au moins partiellement le dispositif (1; 201; 301) sur l'être vivant,

dans lequel, optionnellement, l'élément d'appui (307) fournit une surface spécifique (315) qui peut de préférence être mise en contact avec une surface de contact, en particulier d'une région dorsale de l'être vivant, pour appuyer le dispositif (1; 201; 301) sur l'être vivant, le tracé et/ou la forme de la surface spécifique (315) pouvant être adaptés, au moins par sections et/ou par zones, au tracé et/ou à la forme de ladite surface de contact,
dans lequel, en outre optionnellement, l'élément d'appui (307) à surface spécifique (315) présente plusieurs éléments individuels (311), de préférence mobiles les uns par rapport aux autres, en particulier variables dans leur position et/ou leur orientation, et/ou modifiables de manière réversible dans leur forme et/ou leur configuration, en particulier par compression, et chaque élément individuel (311) fournit, avec une zone de surface, une partie de la surface spécifique (315) de l'élément d'appui (307), dans lequel, en outre optionnellement, l'élément d'appui (307) à surface spécifique (315) présente un élément de base élastique (313), constitué notamment de silicone ou en comportant, sur lequel les éléments individuels (311) sont disposés et notamment reliés par liaison de matière audit élément de base élastique (313), dans lequel de préférence : (i) les éléments individuels (311) sont disposés, de préférence côte à côte et/ou à plat, sur l'élément de base élastique (313), (ii) l'élément de base élastique (313) est réalisé sous forme de couche, (iii) l'élément de base élastique (313) est disposé, notamment directement, entre d'une part les éléments individuels (311) et d'autre part le boîtier (103 ; 303 ; 403 ; 503), de préférence l'élément de base élastique (313) est disposé directement sur le boîtier (103 ; 303 ; 403 ; 503), (iv) l'élément de base élastique (313) est parallélépipédique et/ou (v) l'élément de base élastique (313) présente une épaisseur d'au moins 0,5 cm et/ou d'au plus 10 cm.

15. Dispositif selon l'une des revendications précédentes, le dispositif (1 ; 201 ; 301) présentant, en particulier sur la partie latérale et/ou sur la partie frontale, au moins un élément de socle (305, 305'), de préférence en forme de tronc de pyramide ou de parallélépipède, présentant de préférence plusieurs desdits éléments de socle (305, 305'), et lesdits éléments de socle (305, 305') étant de préférence formés par le boîtier (103 ; 303 ; 403 ; 503), et/ou dans lequel les modules de chauffage à infrarouge (9 ; 101, 101' ; 333 ; 401 ; 501 ; 603) présentent chacun un élément chauffant découpé au laser ou un élément chauffant de surface en mica synthétique et/ou présentent un couvercle en aluminium notamment anodisé ; et/ou

    dans lequel les modules de chauffage à infrarouge (9 ; 101, 101' ; 333 ; 401 ; 501 ; 603) présentent chacun au moins un élément radiateur à infrarouge sous la forme d'un fil chauffant, d'un film chauffant, d'un tissu chauffant, de micanite, d'un chauffage à couche épaisse et/ou d'un tapis chauffant en silicone, de préférence sous la forme d'un tissu de carbone, et/ou dans lequel les modules de chauffage à infrarouge (9 ; 101, 101' ; 333 ; 401 ; 501 ; 603) s'étendent sous forme de bandes le long d'une direction perpendiculaire au plan transversal spécifique (7).

Fig. 1a

Fig. 1b

**Fig. 2**

**Fig. 3**

**Fig. 4**

**Fig. 5**

**Fig. 6a**

103′

109′

117′

115b′

107′

105′

113′

115a′

**Fig. 6b**

101′

103′

119′

119′

105′

107′

105′

109′, 115b′

**Fig. 7**

**Fig. 8**

**Fig. 9**

**Fig. 10a**

**Fig. 10b**

**Fig. 10c**

Fig. 10d

Fig. 11

Fig. 12

**Fig. 13**

**Fig. 14**

601

603

605

603

603

**Fig. 15a**

601

603

603

603

**Fig. 15b**

601

603

605

603

603

607

**Fig. 15c**

601

603

605

603

603

607

**Fig. 15d**

701 601

603

605

603

603

703

Fig. 16

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- CA 3057840 A1
- US 2015105844 A1
- US 2007179571 A1
- US 2012122636 A1